# EUROPÄISCHE PATENTSCHRIFT

(11) **EP 0 647 644 B1**
(45) Veröffentlichungstag und Bekanntmachung des Hinweises auf die Patenterteilung: **05.11.1997**
(21) Anmeldenummer: 94113044.5
(22) Anmeldetag: 22.08.1994
(51) Int. Cl.: C07D 498/06, A61K 31/535, C07D 215/58, C07D 401/04, C07D 413/04, A61K 31/47, A61K 31/435

(54) **Aktibakteriell wirksame Pyrido 1,2,3-d,e 1,3,4 Benzoxadiazinderivate**
Antibacterial pyrido 1,2,3-d,e 1,3,4 benzoxacin derivatives
Dérivés de pyrido 1,2,3-d,e 1,3,4 benzoxzine avec une activité antibactérienne

(30) Priorität: 02.09.1993 DE 4329600
(43) Veröffentlichungstag der Anmeldung: 12.04.1995
(73) Patentinhaber: BAYER AG, 51368 Leverkusen (DE)
(72) Erfinder: Jaetsch, Thomas, Dr., D-50668 Köln (DE); Mielke, Burkhard, Dr., D-51375 Leverkusen (DE); Petersen, Uwe, Dr., D-51375 Leverkusen (DE); Philipps, Thomas, Dr., D-51065 Köln (DE); Schenke, Thomas, Dr., D-51469 Bergisch Gladbach (DE); Bremm, Klaus Dieter, Dr., D-42109 Wuppertal (DE); Endermann, Rainer, Dr., D-42113 Wuppertal (DE); Metzger, Karl Georg, Dr., D-42115 Wuppertal (DE); Scheer, Martin, Dr., D-42113 Wuppertal (DE); Stegemann, Michael, Dr., D-51381 Leverkusen (DE); Wetzstein, Heinz-Georg, Dr., D-51377 Leverkusen (DE)

(56) Entgegenhaltungen:
- EP-A- 0 259 804
- EP-A- 0 287 951
- EP-A- 0 343 524
- EP-A- 0 391 132
- EP-A- 0 520 240
- EP-A- 0 550 903
- EP-A- 0 572 259
- WO-A-94/17074
- EUR.J.MED.CHEM., Bd.26, 1991, WASHINGTON Seiten 889 - 906 OGATA,M. ET AL. 'Synthesis and antibacterial activity of new 7-(aminoaza bicycloalkanyl)quinolonecarboxylic acids'
- J.Org.Chem.57,744(1992)
- Res.Dislc.291,548(1991)

## Beschreibung

Die Erfindung betrifft neue Pyrido[1,2,3-d,e][1,3,4]benzoxadiazinderivate, Verfahren zu ihrer Herstellung sowie diese enthaltende antibakterielle Mittel und Futterzusatzstoffe.

Es ist bereits bekannt geworden, daß derartige Pyridobenzoxadiazincarbonsäuren antibakteriell wirksam sind. Beispiele hierfür finden sich in EP-O 259 804, EP-O 343 524 und im European Journal of Medicinal Chemistry 26, 889 (1991).

Weitere antibakteriell wirkende Chinolonsäurederivate sind bekannt aus EP-A 287 951, 550 903, 520 240, 391 132, WO-A 94/17 074, J. Org. Chem 57, 744 (1992), Res. Discl. 291, 548 (1991). Die Wirkung der bekannten Verbindungen ist jedoch nicht immer zufriedenstellend.

Es wurden nun Verbindungen der allgemeinen Formel (I) gefunden 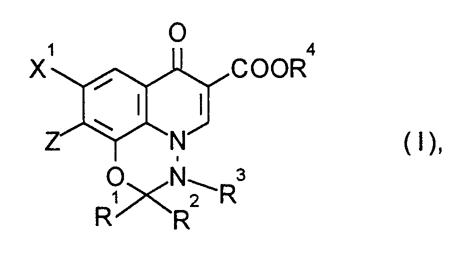 in welcher
- R¹: für Wasserstoff oder gegebenenfalls durch Hydroxy oder Halogen substituiertes C₁-C₄-Alkyl steht,
- R²: unabhängig von R¹ für Wasserstoff oder Methyl steht,
- R³: für Wasserstoff oder C₁-C₄-Alkyl steht,
- R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- X¹: für Wasserstoff oder Halogen steht,
- Z: für Reste der Strukturen 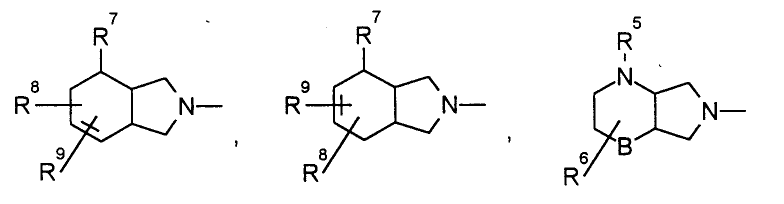 steht,
worin
- R⁷: für Wasserstoff, Hydroxy, -NR¹⁰R¹¹, Hydroxymethyl, -CH₂-NR¹⁰R¹¹, Carboxyl, Methoxycarbonyl oder Ethoxycarbonyl steht,
wobei
- R¹⁰: für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
- R¹¹: für Wasserstoff oder Methyl steht,
- R⁸: für Wasserstoff, geradkettiges oder verzweigtes C₁-C₃-Alkyl oder Cyclopropyl steht,
- R⁹: für Wasserstoff oder Methyl steht,
- R⁶: für Wasserstoff oder Methyl steht,
- R⁵: für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
- R^{5'}: für Methyl oder Ethyl steht,
- B: für -CH₂-, O oder eine direkte Bindung steht.

Die Verbindungen der Formel (I) können in Form von Racematen oder als enantiomerenreine Verbindungen sowie in Form ihrer pharmazeutisch verwendbaren Hydrate und Säureadditionssalze sowie in Form ihrer Alkali-, Erdalkali-, Silber- und Guanidiniumsalze vorliegen.

Man erhält die Verbindungen der Formel (I), wenn man Verbindungen der Formel (II) 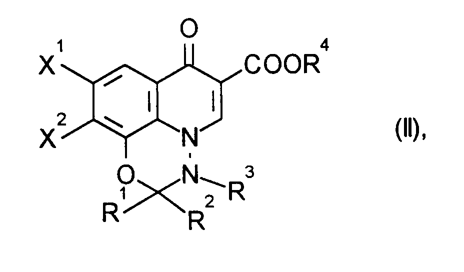 in welcher
- R¹, R², R³, R⁴ und X¹: die oben angegebene Bedeutung haben und
- X²: für Halogen, insbesondere Fluor oder Chlor, steht,
mit Verbindungen der Formel (III)

Z-H (III),

in welcher
- Z: die oben angegebene Bedeutung hat,
gegebenenfalls in Gegenwart von Säurefängern umsetzt.

Die erfindungsgemäßen Verbindungen weisen im Vergleich zu bekannten Vertretern dieses Strukturtyps eine höhere antibakterielle Wirkung insbesondere im grampositiven Bereich auf. Sie eignen sich daher als Wirkstoffe für die Human-und Veterinärmedizin, wobei zur Veterinärmedizin auch die Behandlung von Fischen zur Therapie oder zur Vorbeugung bakterieller Infektionen zu zählen ist.

Bevorzugt sind Verbindungen der Formel (I), in welcher
- R¹: für Wasserstoff oder gegebenenfalls durch Hydroxy substituiertes C₁-C₃-Alkyl steht,
- R²: unabhängig von R¹ für Wasserstoff oder Methyl steht,
- R³: für Wasserstoff, Methyl oder Ethyl steht,
- R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)methyl steht,
- X¹: für Wasserstoff, Fluor oder Chlor steht,
- Z: für Reste der Strukturen 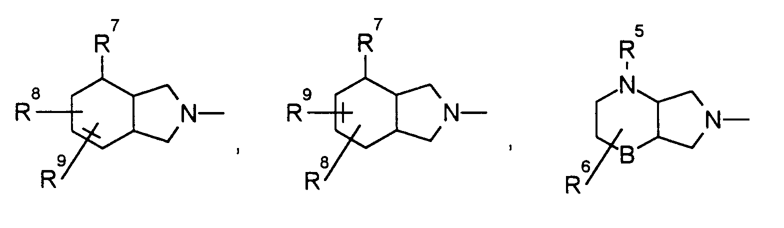 steht,
worin
- R⁷: für Wasserstoff, Hydroxy, -NR¹⁰R¹¹, Hydroxymethyl oder -CH₂-NR¹⁰R¹¹ steht,
wobei
- R¹⁰: für Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl steht,
- R¹¹: für Wasserstoff oder Methyl steht,
- R⁸: für Wasserstoff, geradkettiges oder verzweigtes C₁-C₃-Alkyl oder Cyclopropyl steht,
- R⁹: für Wasserstoff oder Methyl steht,
- R⁵: für Wasserstoff oder Methyl steht,
- R⁶: für Wasserstoff steht,
- B: für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

Besonders bevorzugt sind die Verbindungen der Formel (I), in welcher
- R¹: für Wasserstoff oder Methyl steht,
- R²: für Wasserstoff,
- R³: für Methyl oder Ethyl steht,
- R⁴: für Wasserstoff, Methyl oder Ethyl steht,
- X¹: für Fluor steht,
- Z: für Reste der Strukturen 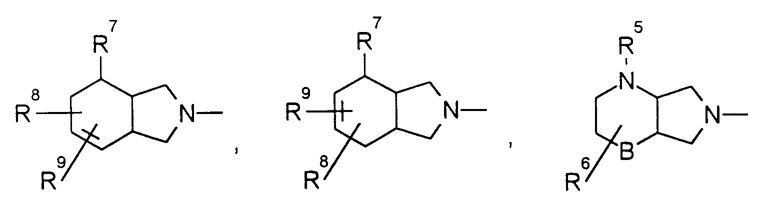 steht,
worin
- R⁷: für Wasserstoff, Hydroxy, -NR¹⁰R¹¹, Hydroxymethyl oder -CH₂-NR¹⁰R¹¹ steht,
wobei
- R¹⁰: für Wasserstoff, Methyl, Alkoxycarbonyl mit 1 bis 4 C-Atomen im Alkoxyteil oder C₁-C₃-Acyl steht,
- R¹¹: für Wasserstoff oder Methyl steht,
- R⁸: für Wasserstoff, geradkettiges oder verzweigtes C₁-C₃-Alkyl oder Cyclopropyl steht,
- R⁶: für Wasserstoff steht,
- R⁹: für Wasserstoff oder Methyl steht,
- R⁵: für Wasserstoff oder Methyl steht,
- B: für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

Im einzelnen seien folgende Verbindungen der Formel (I) genannt:

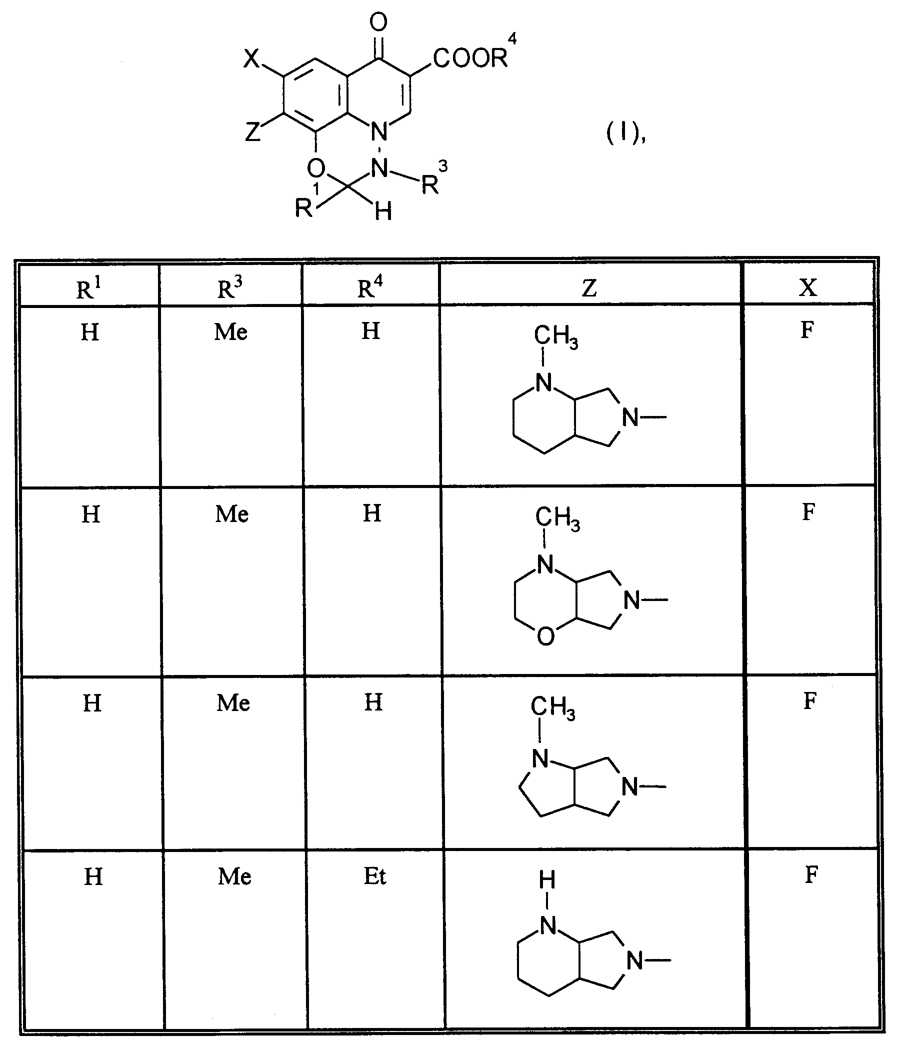

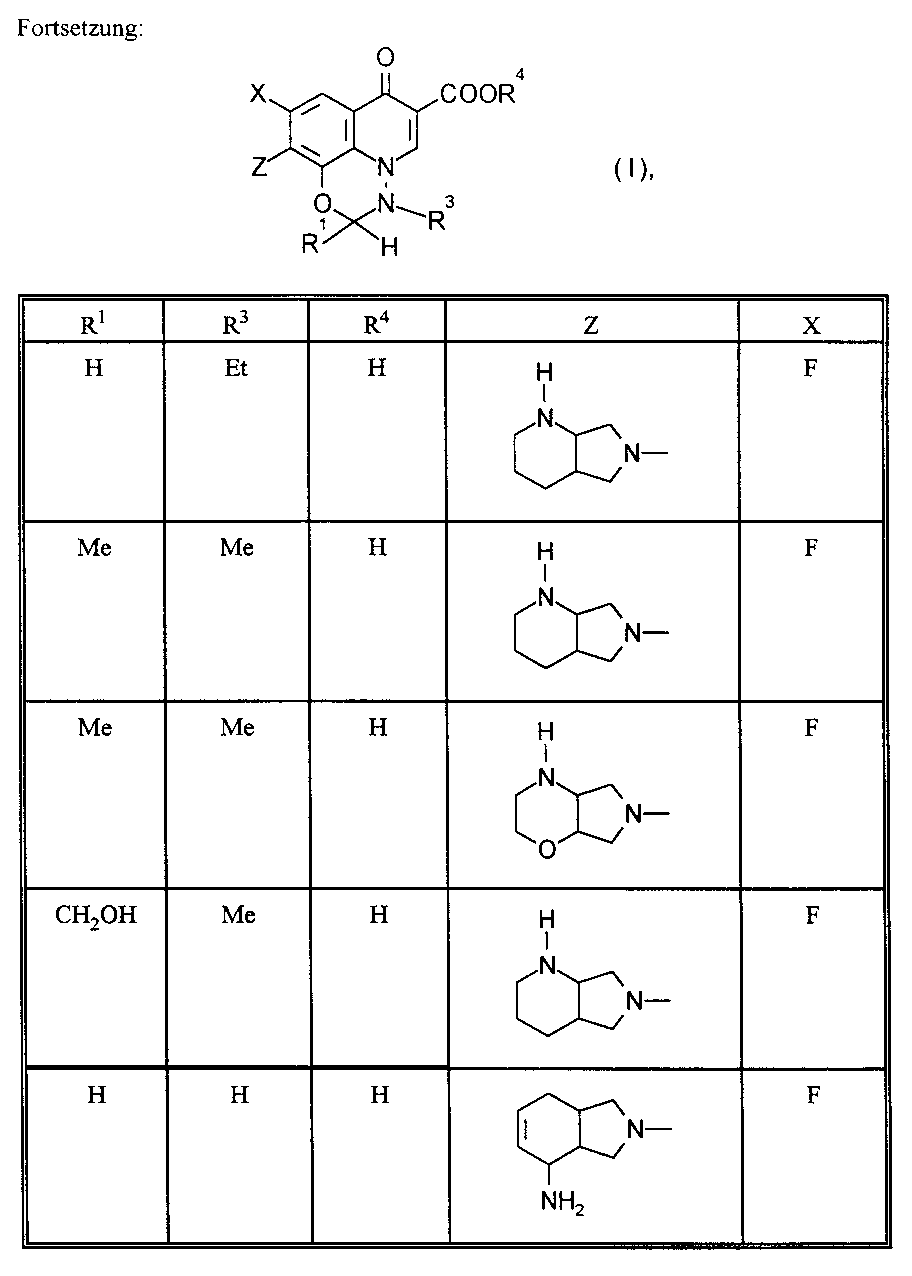

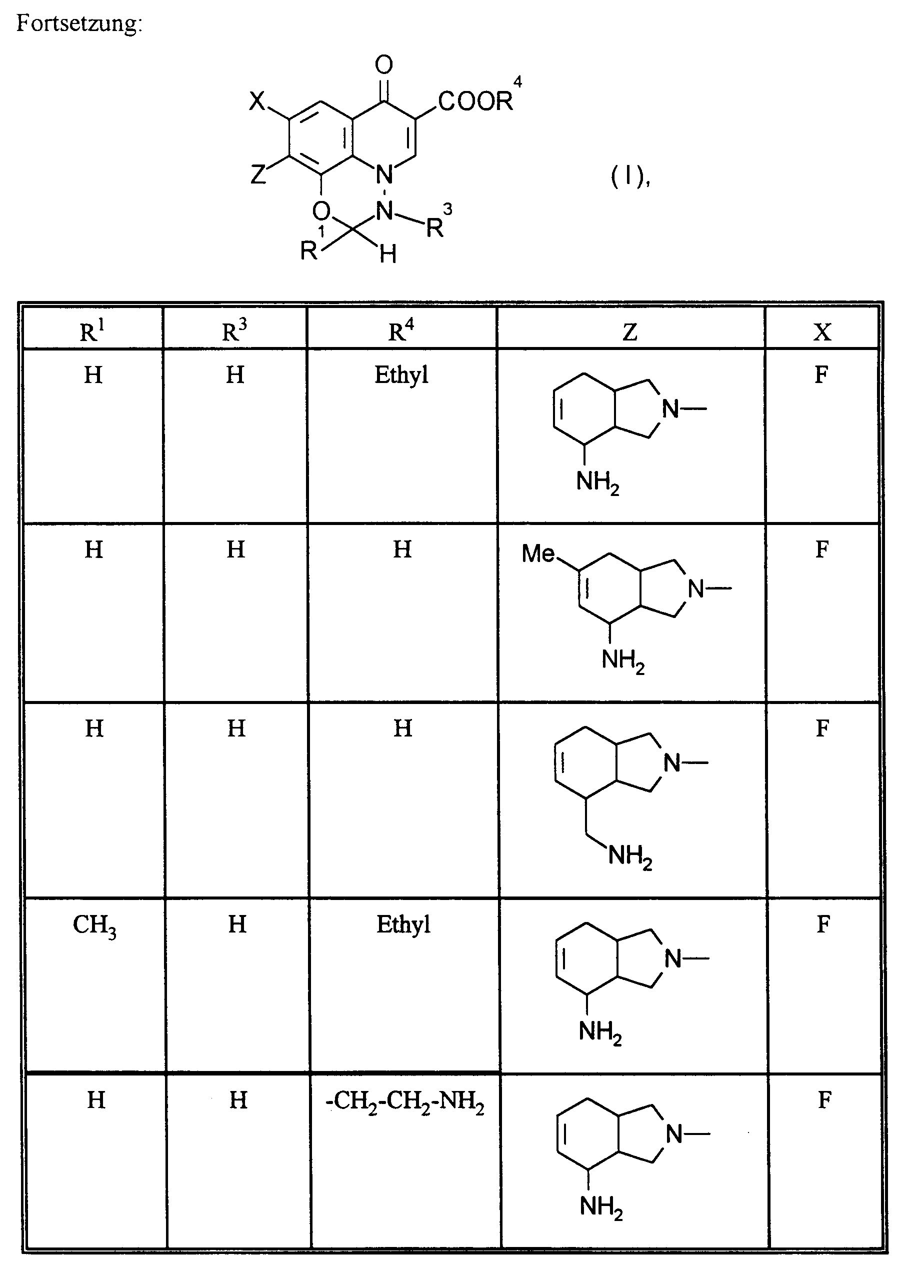

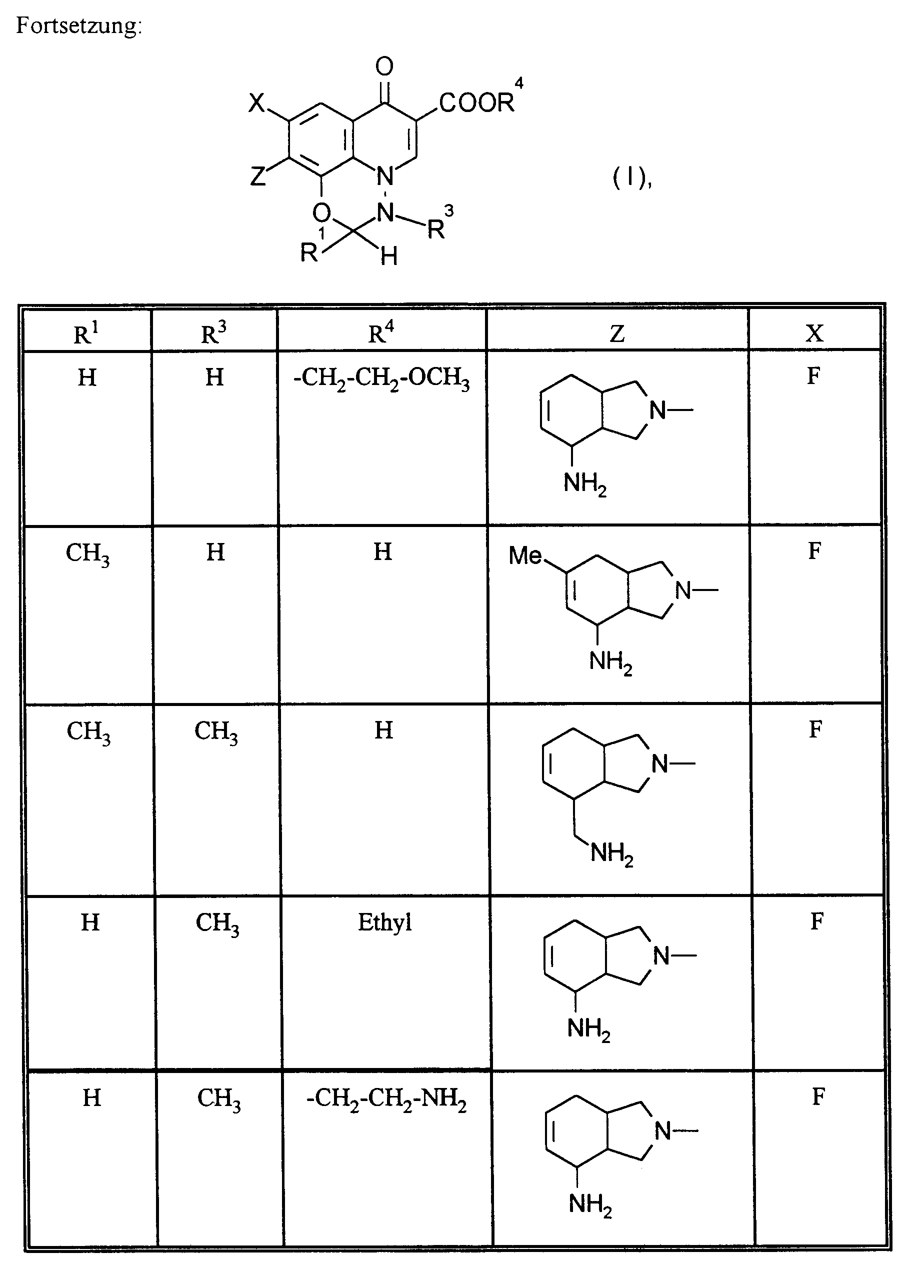

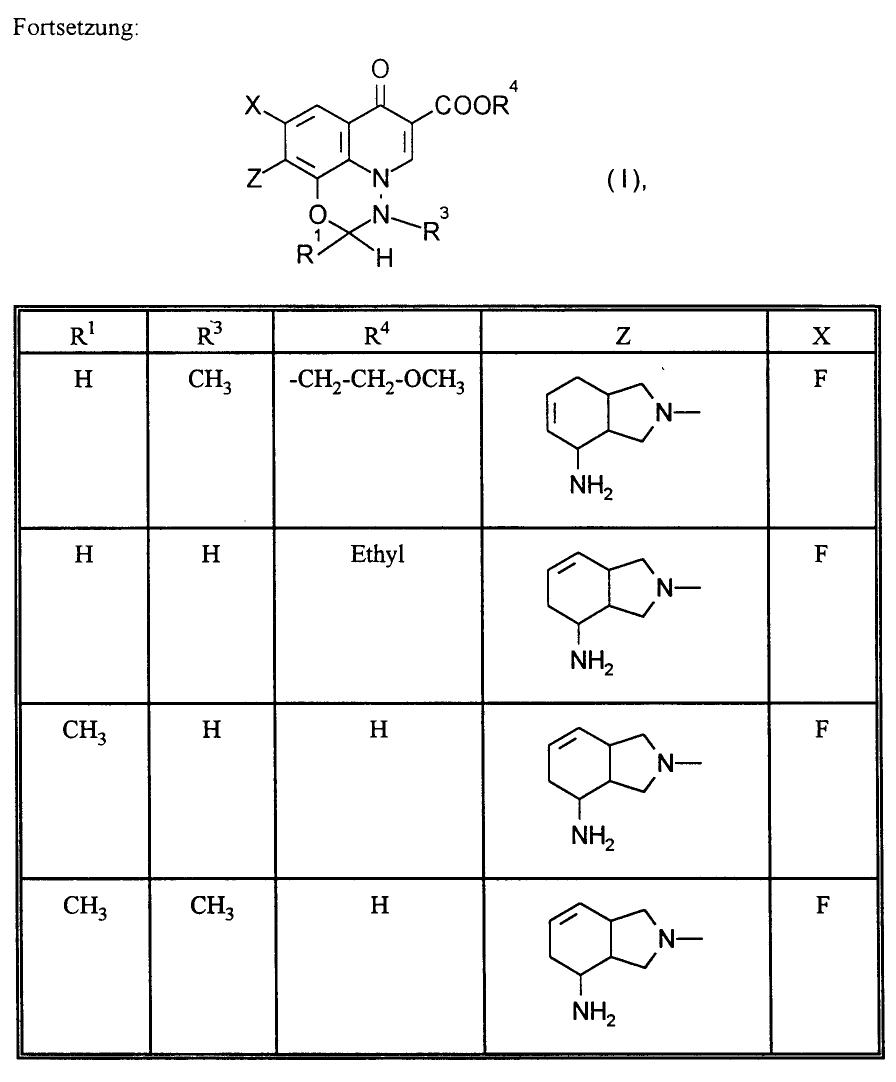

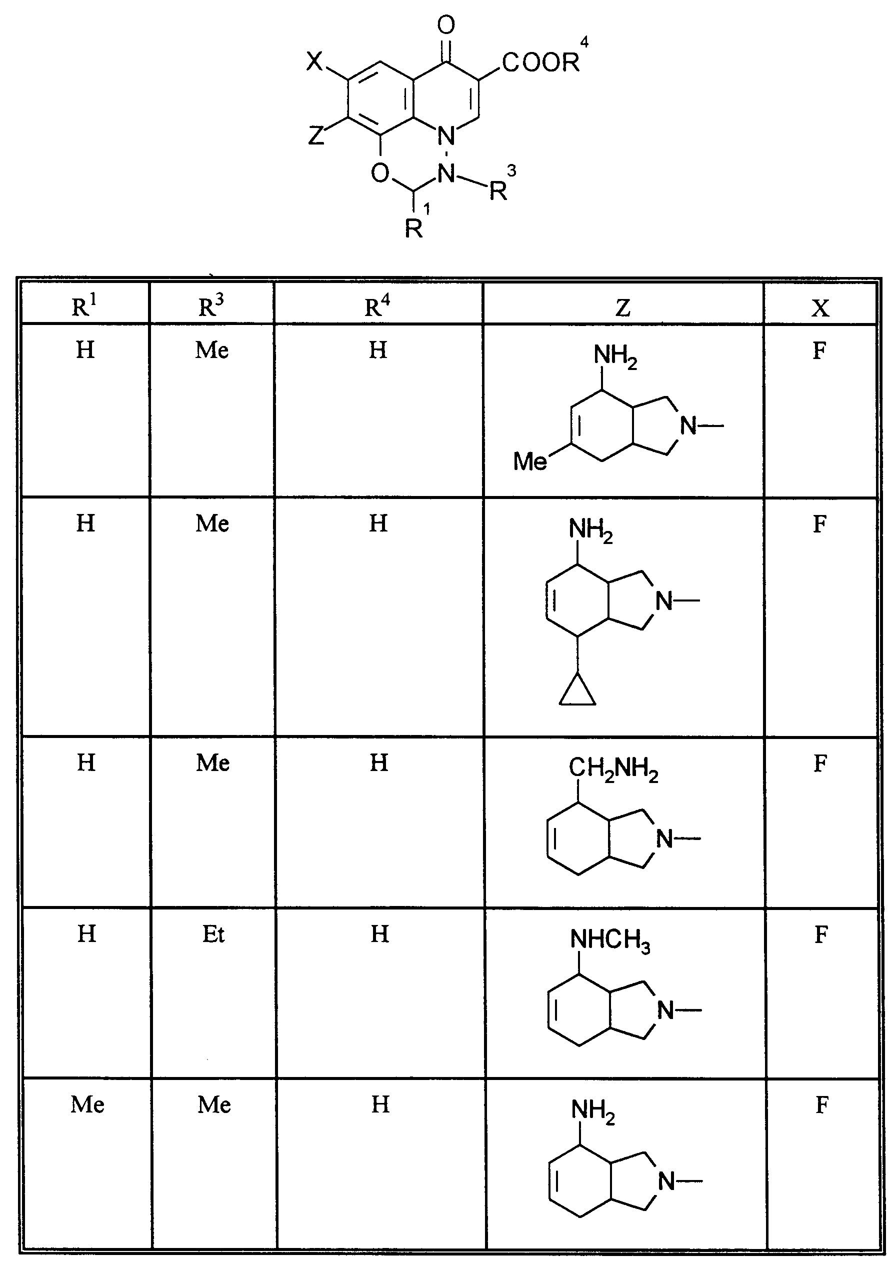

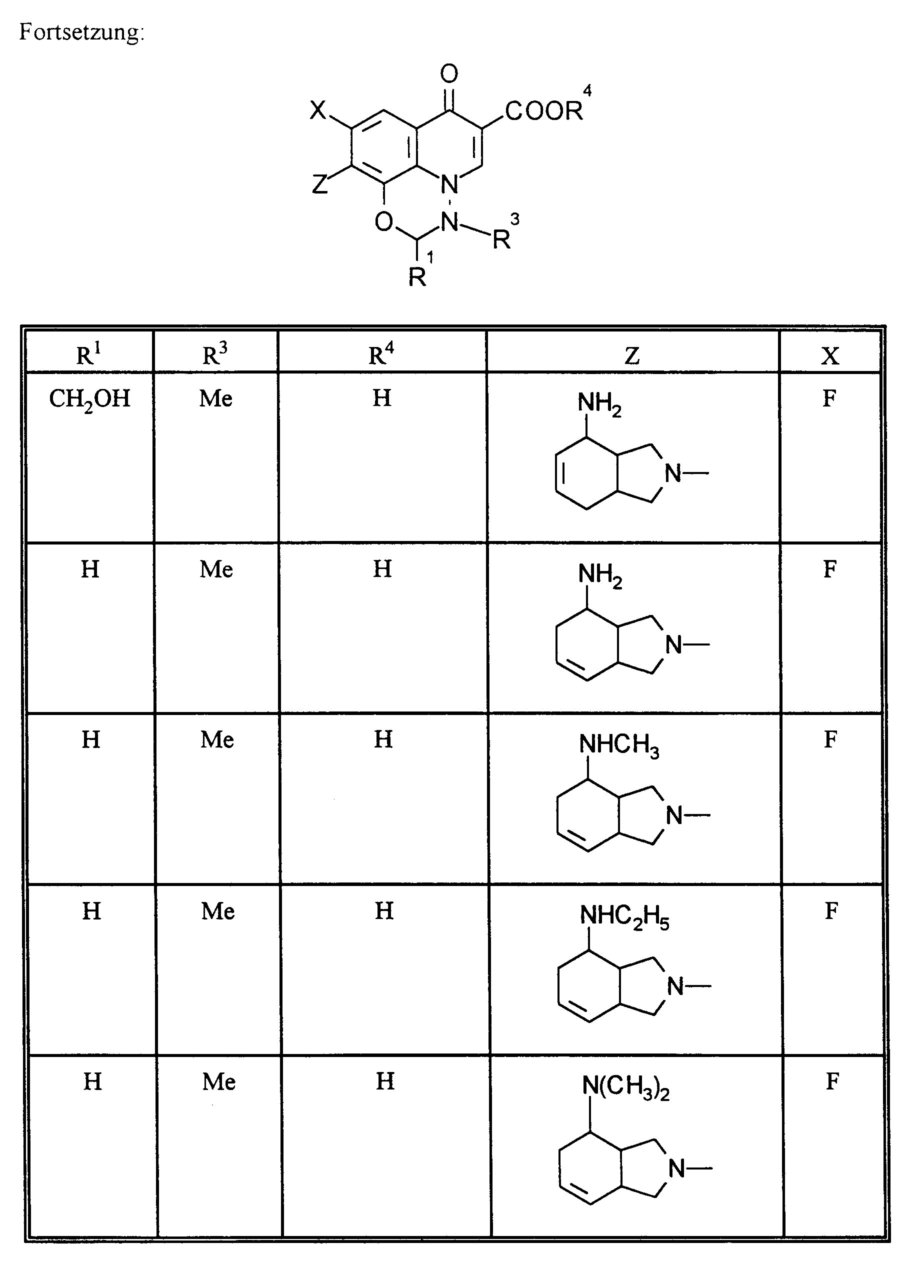

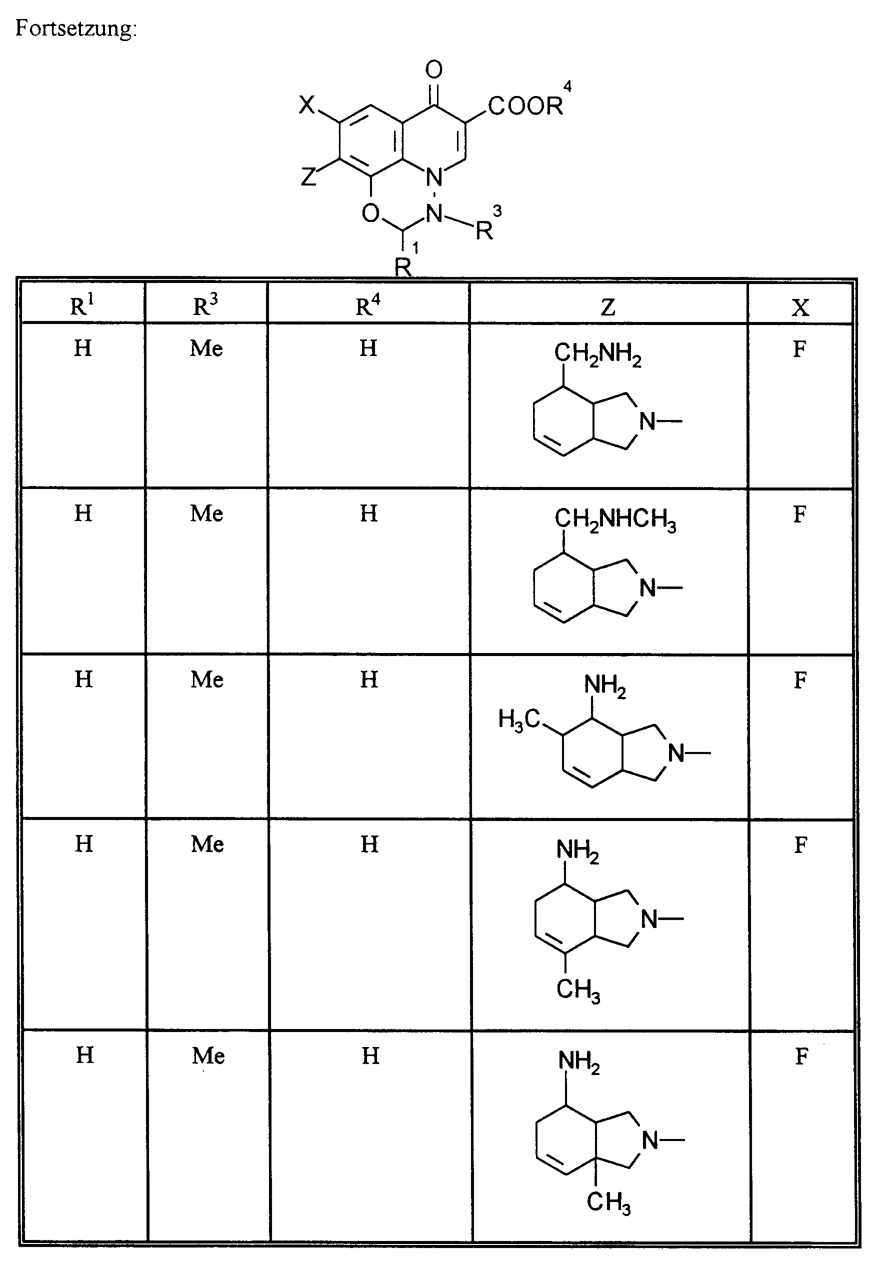

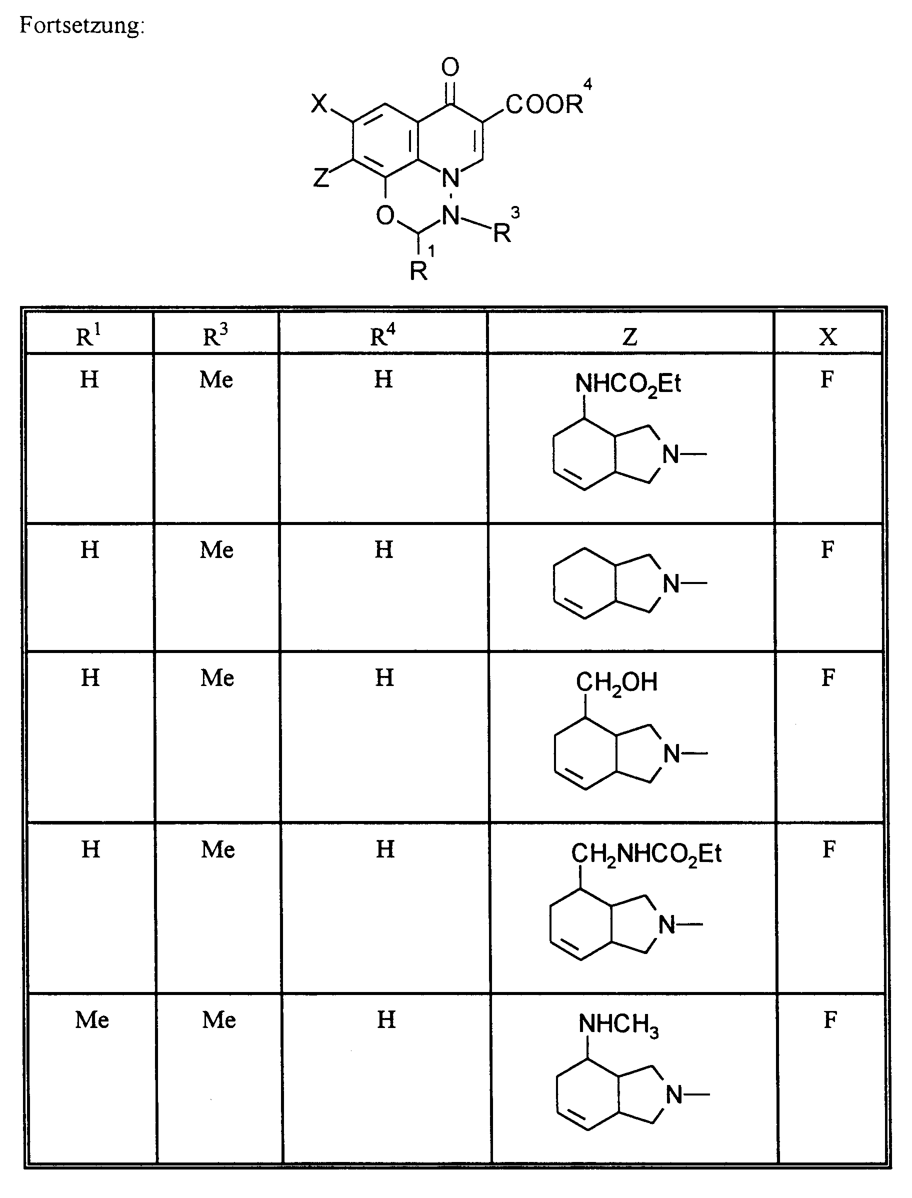

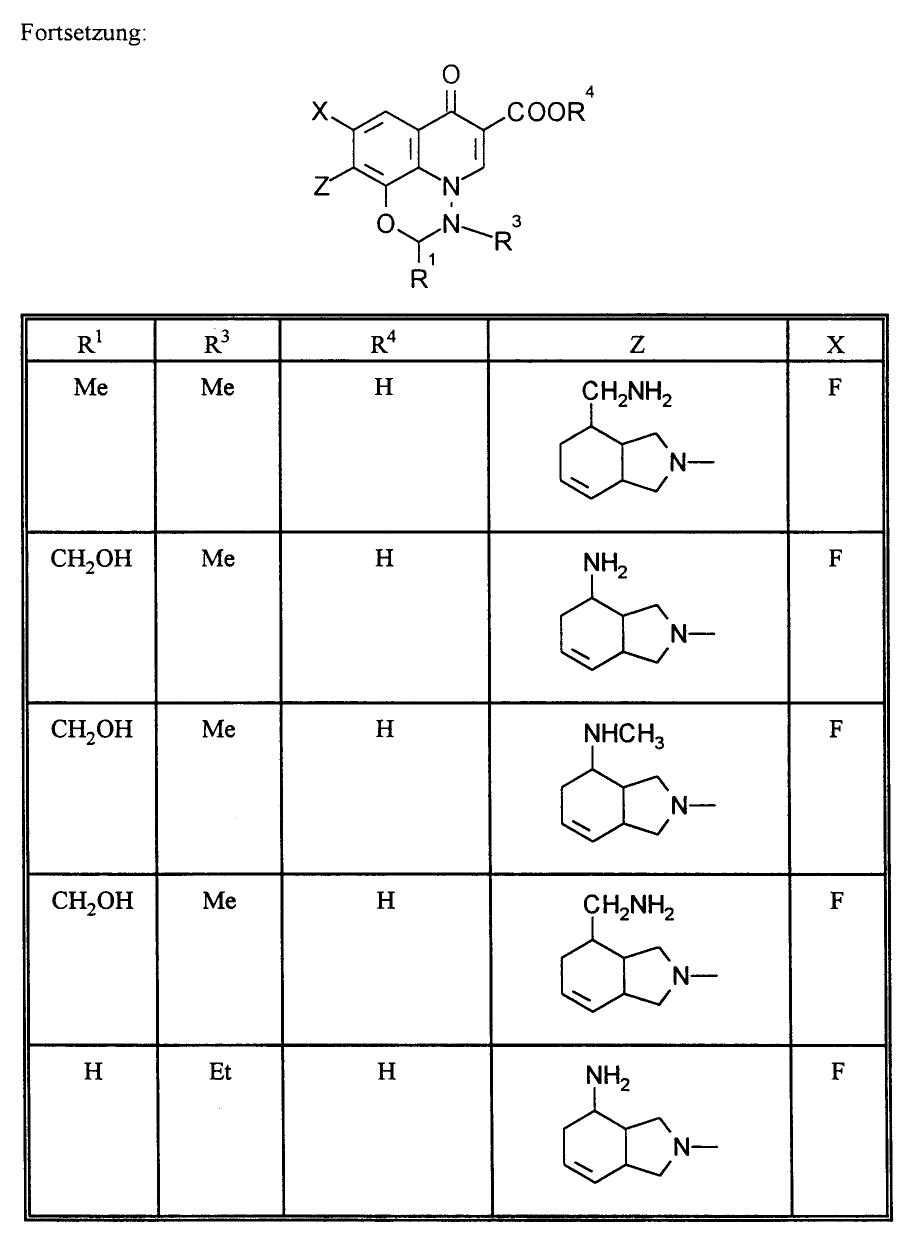

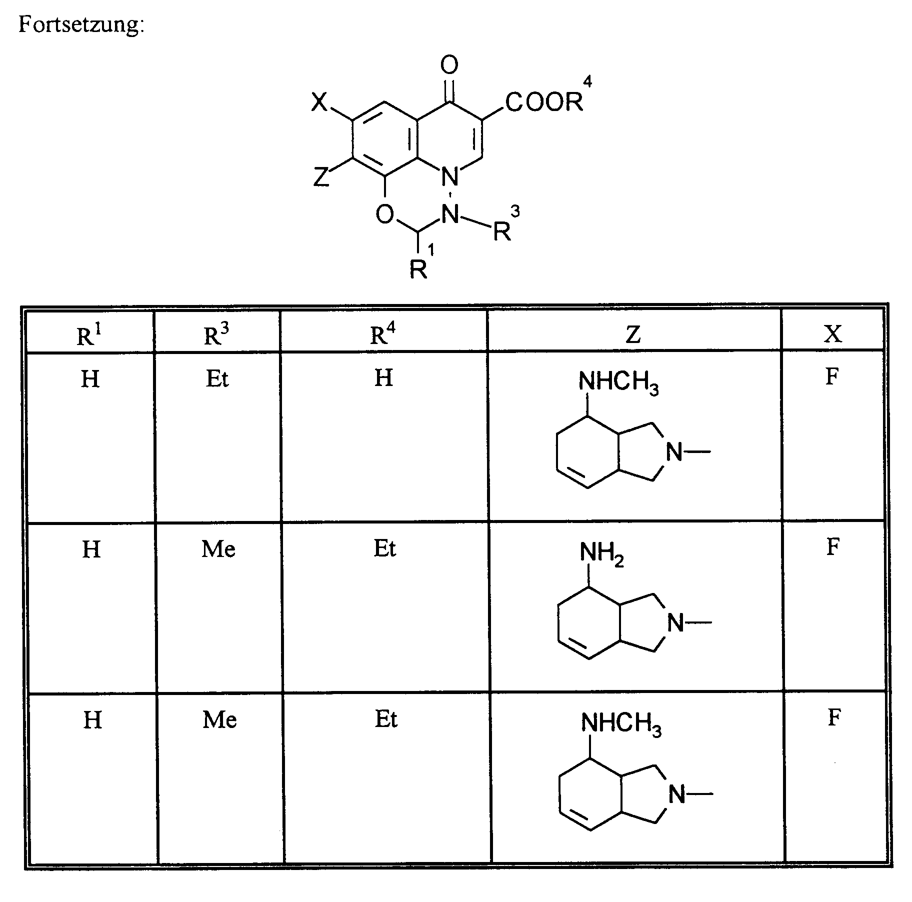

Verwendet man zur Herstellung von Verbindungen der Formel (I) beispielsweise 9,10-Difluor-3-methyl-7-oxo-2,3-dihydro-7H-pyrido[1,2,3-d,e][1,3,4]benzoxadiazin-6-carbonsäure und 2,8-Diazabicyclo[4.3.0]nonan, so kann der Reaktionsverlauf durch das folgende Formelschema wiedergegeben werden: 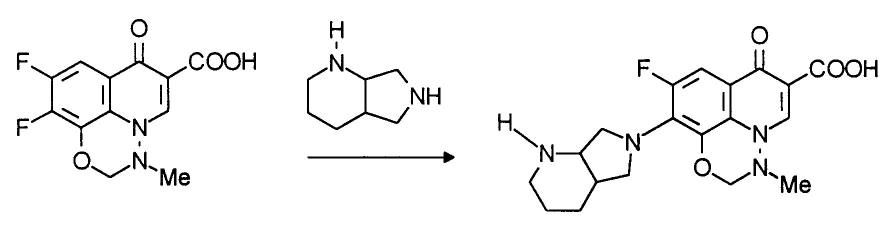 Die als Ausgangsverbindungen verwendeten Verbindungen der Formel (II) sind bekannt, bzw. können nach bekannten Verfahren hergestellt werden. Sie können gegebenenfalls als Racemate, Enantiomere oder reine Diastereomere eingesetzt werden.

Als Beispiele seien genannt:
9,10-Difluor-3-methyl-7-oxo-2,3-dihydro-7H-pyrido[1,2,3-d,e][1,3,4]benzoxadiazin-6-carbonsäure
9,10-Difluor-2,3-dimethyl-7-oxo-2,3-dihydro-7H-pyrido[1,2,3-d,e][1,3,4]benzoxadiazin-6-carbonsäure
9,10-Difluor-2-(hydroxymethyl)-3-methyl-7-oxo-2,3-dihydro-7H-pyrido[1,2,3-d,e][1,3,4]benzoxadiazin-6-carbonsäure
9,10-Difluor-3-ethyl-7-oxo-2,3-dihydro-7H-pyrido[1,2,3-d,e][1,3,4]benzoxadiazin-6-carbonsäure
9,10-Difluor-3-methyl-7-oxo-2,3-dihydro-7H-pyrido[1,2,3-d,e][1,3,4]benzoxadiazin-6-carbonsäureethylester
Die als Ausgangsverbindungen verwendeten Amine der Formel (III) sind bekannt. Chirale Amine können sowohl als Racemate, als auch als enantiomeren- oder diastereomerenreine Verbindungen eingesetzt werden.

Als Beispiele seien genannt:
2,7-Diazabicyclo[3.3.0]octan
2-Methyl-2,7-diazabicyclo[3.3.0]octan
2,8-Diazabicyclo[4.3.0]nonan
2-Methyl-2,8-diazabicyclo[4.3.0]nonan
2-Oxa-5,8-diazabicyclo[4.3.0]nonan
5-Methyl-2-oxa-5,8-diazabicyclo[4.3.0]nonan.
2-Amino-8-azabicyclo[4.3.0]non-3-en
2-Methylamino-8-azabicyclo[4.3.0]non-3-en
4-Methyl-2-methylamino-8-azabicyclo[4.3.0]non-3-en
5-Methyl-2-methylamino-8-azabicyclo[4.3.0]non-3-en
2-Dimethylamino-8-azabicyclo[4.3.0]non-3-en
2-Ethylamino-8-azabicyclo[4.3.0]non-3-en
2-Methylaminomethyl-8-azabicyclo[4.3.0]non-3-en
2-Hydroxy-8-azabicyclo[4.3.0]non-3-en
5-Isopropyl-2-methylamino-8-azabicyclo[4.3.0]non-3-en
2-Amino-5-isopropyl-8-azabicyclo[4.3.0]non-3-en
2-Amino-5-methyl-8-azabicyclo[4.3.0]non-3-en
2-Hydroxymethyl-8-azabicyclo[4.3.0]non-3-en
2-Amino-5-cyclopropyl-8-azabicyclo[4.3.0]non-3-en
8-Azabicyclo[4.3.0]non-2-en
8-Azabicyclo[4.3.0]non-4-en-2-carbonsäureethylester
2-Hydroxymethyl-8-azabicyclo[4.3.0]non-4-en
2-Amino-8-azabicyclo[4.3.0]non-4-en
2-Ethyloxycarbonylamino-8-azabicyclo[4.3.0]non-4-en
2-tert.-Butyloxycarbonylamino-8-azabicyclo[4.3.0]non-4-en
2-Benzyloxycarbonylamino-8-azabicyclo[4.3.0]non-4-en
2-Allyloxycarbonylaminomethyl-8-azabicyclo[4.3.0]non-4-en
2-Aminomethyl-8-azabicyclo[4.3.0]non-4-en
2-Ethyloxycarbonylaminomethyl-8-azabicyclo[4.3.0]non-4-en
2-tert.-Butyloxycarbonylaminomethyl-8-azabicyclo[4.3.0]non-4-en
2-Methylamino-8-azabicyclo[4.3.0]non-4-en
2-Ethylamino-8-azabicyclo[4.3.0]non-4-en
2-Cyclopropylamino-8-azabicyclo[4.3.0]non-4-en
2-Dimethylamino-8-azabicyclo[4.3.0]non-4-en
2-[(2-Hydroxyethyl)-amino]-8-azabicyclo[4.3.0]non-4-en
2-Amino-1-methyl-8-azabicyclo[4.3.0]non-4-en
2-Amino-2-methyl-8-azabicyclo[4.3.0]non-4-en
2-Amino-3-methyl-8-azabicyclo[4.3.0]non-4-en
2-Ethyloxycarbonylamino-3-methyl-8-azabicyclo[4.3.0]non-4-en
2-tert.-Butyloxycarbonylamino-3-methyl-8-azabicyclo[4.3.0]non-4-en
2-Benzyloxycarbonylamino-3-methyl-8-azabicyclo[4.3.0]non-4-en
2-Allyloxycarbonylaminomethyl-3-methyl-8-azabicyclo[4.3.0]non-4-en
2-Amino-4-methyl-8-azabicyclo[4.3.0]non-4-en
2-Amino-5-methyl-8-azabicyclo[4.3.0]non-4-en
2-Amino-6-methyl-8-azabicyclo[4.3.0]non-4-en
2-Amino-7-methyl-8-azabicyclo[4.3.0]non-4-en
2-Amino-9-methyl-8-azabicyclo[4.3.0]non-4-en

Die substituierten 8-Azabicyclo[4.3.0]non-4-ene und 8-Azabicyclo[4.3.0]non-2-en sind Gegenstand einer noch nicht zum Stand der Technik gehörenden Anmeldung der Anmelderin (Le A 29 200).

Verbindungen der allgemeinen Formel (IV) 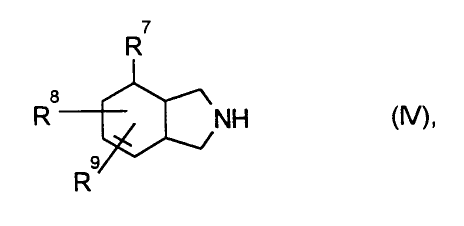 in welcher
- R⁷, R⁸ und R⁹: die oben angegebenen Bedeutungen haben,
werden erhalten, indem man geeignete Diene mit geeigneten Dienophilen in einer Diels-Alder Reaktion umsetzt, die intermolekular oder intramolekular durchgeführt werden kann, und gegebenenfalls anschließend weitere chemische Reaktionen durchführt, um gegebenenfalls den Pyrrolidinring aufzubauen und um für die biologische Wirkung gewünschte Substituenten einzuführen und als letzten Schritt die Schutzgruppe am Pyrrolidinstickstoff abspaltet.

Bei intramolekularer Durchführung der Diels-Alder-Reaktion werden Verbindungen der Formel (1) oder (2) 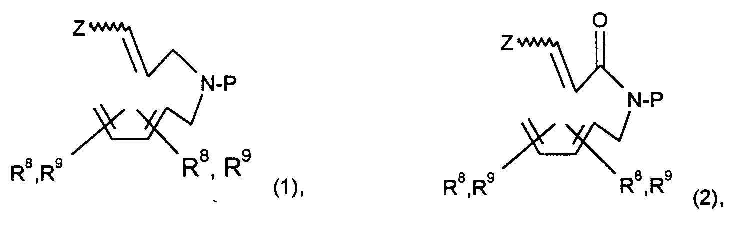 in denen
- R⁸ und R⁹: die oben angegebene Bedeutung haben und
- P: für eine Schutzgruppe (beispielsweise Allyl, Acyl, Carbamoyl oder Trityl),
- Z: für Wasserstoff, eine Carboxyl-, Carbonester- oder Carbonamidgruppe, CN oder NO₂ steht,
zu Verbindungen der Formel (3) [ausgehend von (1)] oder (4) [ausgehend von (2)] 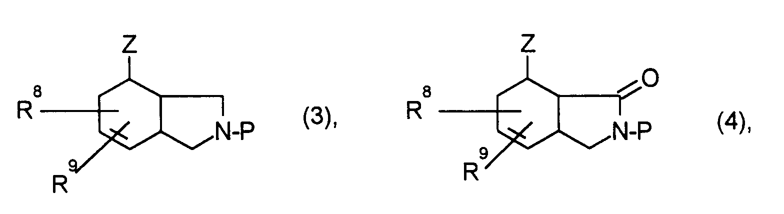 in denen
- R⁸, R⁹, P und Z: die oben angegebenen Bedeutungen haben,
umgesetzt.

Intramolekulare Diels-Alder-Reaktionen ähnlicher Art sind teilweise bekannt: J.M. Mellor, A.M. Wagland; J. Chem. Soc. Perkin I, 997-1005 (1989); W.R. Roush, S.E. Hall; J. Am. Chem. Soc. 103, 5200 (1980); E. Ciganek; Organic Reactions 32, 1-374 (1984). In diesen Arbeiten fehlen jedoch Hinweise auf Schutzgruppen, die gleichzeitig für die Reaktion geeignet und anschließend problemlos abspaltbar sind.

Bei intermolekularer Durchführung der Diels-Alder-Reaktion werden Diene der Formel (5) mit Dienophilen der Formel (6) zu Verbindungen der Formel (7) umgesetzt, und gegebenenfalls nach Modifizierung der Gruppen Z¹ und Z², beispielsweise Überführung eines cyclischen Carbonsäureanhydrids in einen Diester unter Abspaltung der Schutzgruppen P¹ oder P¹ und P², unter Cyclisierung zu den Lactamen der Formel (8) umgesetzt. 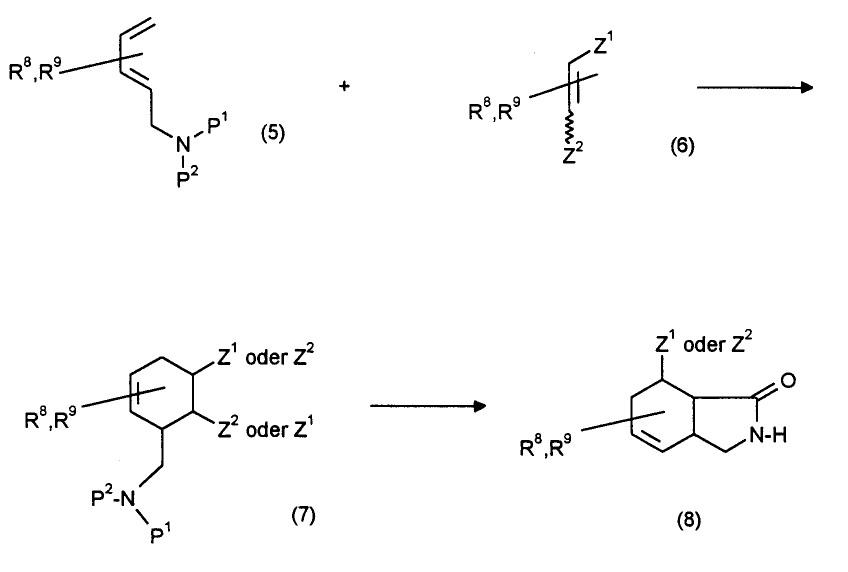 In der Formel (5), (6), (7) und (8) haben R⁸, R⁹ die oben angegebene Bedeutung,
- P¹: steht für eine Acyl- oder Carbamoylschutzgruppe, wenn
- P²: für Wasserstoff steht, oder
- P¹: bildet gemeinsam mit P² ein Imid,
- Z¹ und Z²: stehen für Wasserstoff, Carboxyl, Carbonester- oder Carbonamidgruppen, CN oder NO₂, wobei mindestens eine der beiden Gruppen Z¹ oder Z² eine Carbonestergruppe oder eine Carbonamidgruppe oder CN sein muß oder Z¹ und Z² bilden gemeinsam eine Brücke, so daß ein cyclisches Carbonsäureanhydrid gebildet wird.

Bevorzugte Schutzgruppen P, P¹, P² sind solche Schutzgruppen, bei denen unter den Bedingungen, die zu ihrer Abspaltung verwendet werden, die Cyclisierung zum Lactam und gegebenenfalls eine Veresterung einer zweiten, noch freien Carboxylfunktion mit dem als Lösungsmittel verwendeten Alkohol stattfindet, derart, daß alle Reaktionsschritte in einer Eintopfreaktion ausgeführt werden können, und eine unkontrollierte Umwandlung gegebenenfalls diastereomeren-und enantiomerenreiner Ausgangsstoffe in nicht oder schwer trennbare Isomerengemische nicht stattfindet.

Als Beispiele seien genannt:
1. die tert.-Butyloxycarbonylschutzgruppe
   (Abspaltung mit wäßrigen oder alkoholischen Säuren)
2. die Phthalimidoschutzgruppe
   (Aminolyse mit primären Aminen in wäßrigen oder wasserfreien Alkoholen als Lösungsmittel)

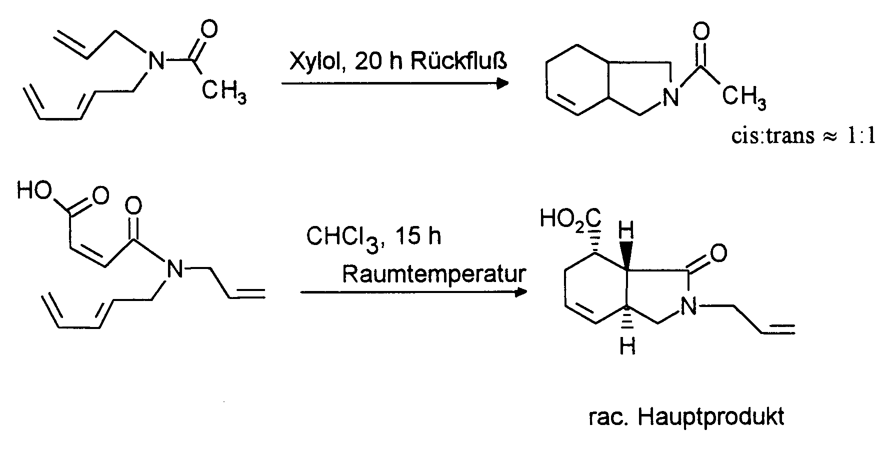

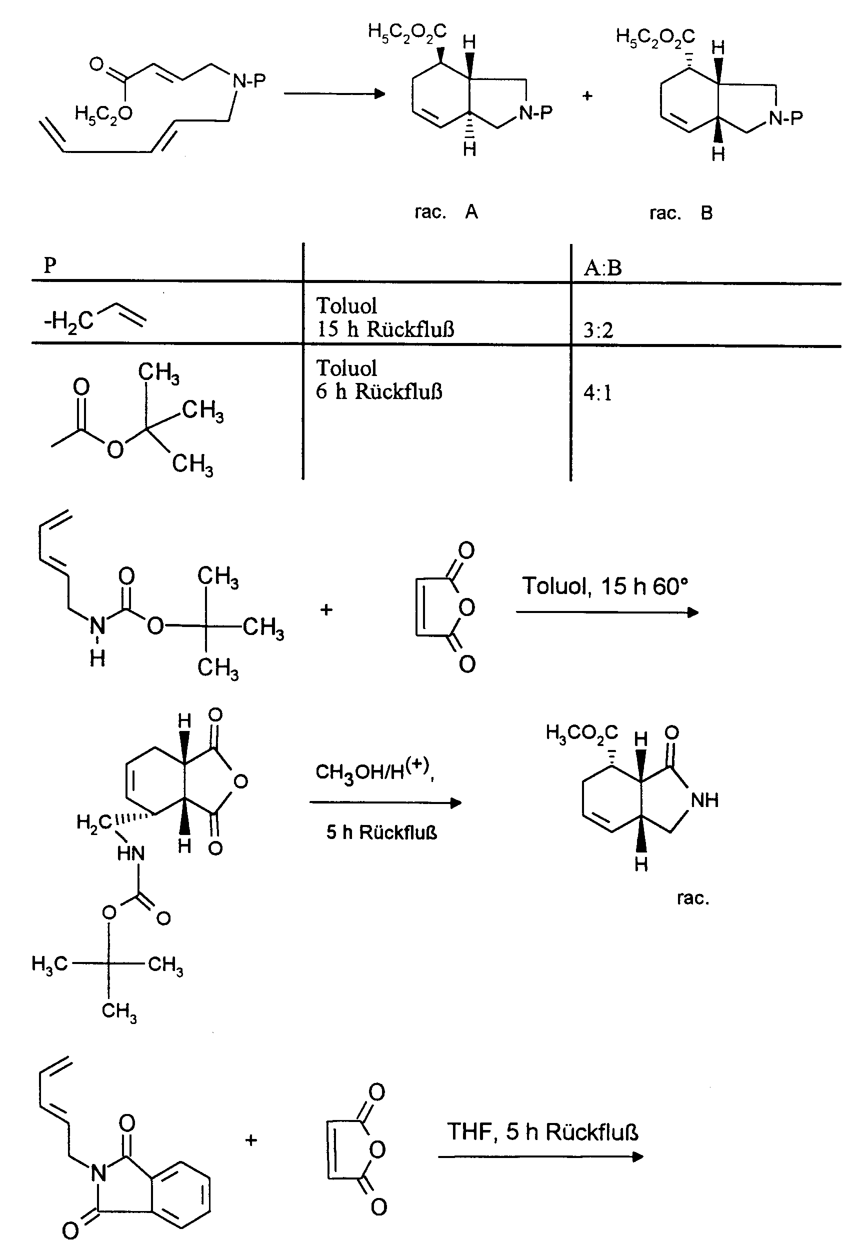

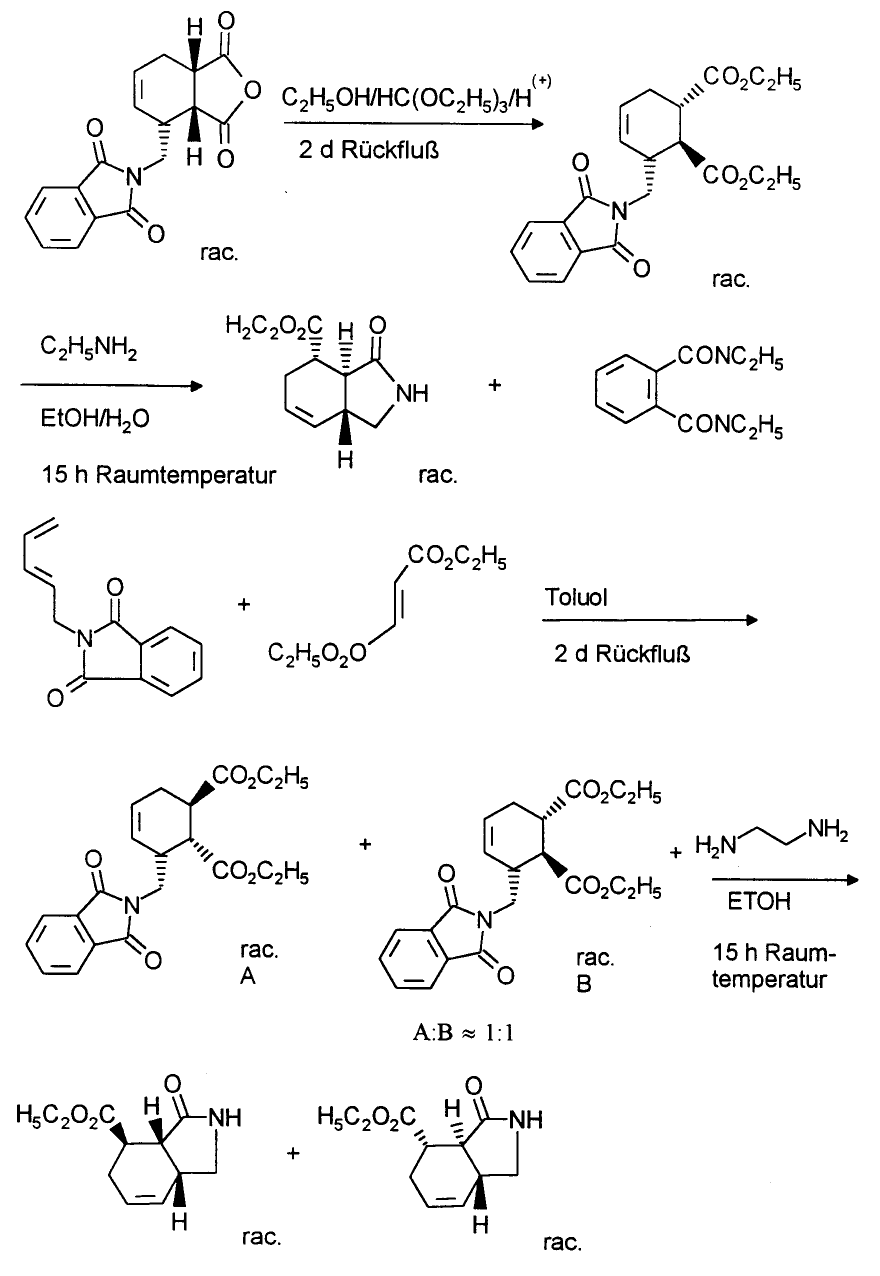

Für die Diels-Alder-Reaktion kommen als Verdünnungsmittel alle inerten organischen Lösungsmittel in Frage. Hierzu gehören vorzugsweise Ether wie Diisopropylether, Di-n-butylether, Dimethoxyethan, Tetrahydrofuran und Anisol, Kohlenwasserstoffe, wie z.B. Hexan, Methylcyclohexan, Toluol, Xylol und Mesitylen und halogenierte Kohlenwasserstoffe, wie z.B. Chloroform, 1,2-Dichlorethan und Chlorbenzol. Die Diels-Alder-Reaktion kann aber auch ohne Lösungsmittel durchgeführt werden.

Die Reaktionstemperaturen können in einem größeren Bereich variiert werden. Im allgemeinen arbeitet man zwischen etwa -20°C und +200°C, vorzugsweise zwischen -20°C und +150°C. Die Diels-Alder-Reaktion wird normalerweise bei Normaldruck durchgeführt. Zur Beschleunigung der Reaktion können aber auch Drucke bis zu 1,5 GPa eingesetzt werden.

Die weitere Umsetzung der Verbindungen der Formel (7) zu den Verbindungen der Formel (8) erfolgt wie in den Beispielen beschrieben oder nach bekannten Methoden der organischen Chemie.

Um ausgehend von den Verbindungen der Formel (3), (4) oder (8) zu den Verbindungen der Formel (III) zu gelangen, sind weitere Reaktionen erforderlich.

Beispielsweise genannt seien die Hydrolyse eines Esters zur Carbonsäure, die Reduktion von Carbonylgruppen, zum Beispiel von Estern, zu Aldehyden oder Alkoholen oder von Lactamgruppen zu den Pyrrolidinen, die Überführung einer Hydroxyfunktion in eine Aminofunktion, die Überführung einer Carboxylfunktion oder eines ihrer Derivate unter Abbau um ein Kohlenstoffatom in eine Aminfunktion, die reduktive Aminierung eines Aldehyds mit einer im Molekül vorhandenen Aminfunktion, die reduktive Aminierung einer im Molekül vorhandenen Aldehydfunktion mit einem Amin, die Einführung von Schutzgruppen, die Abspaltung der Schutzgruppe am Pyrrolidinstickstoff derart, daß im Molekül eventuell vorhandene weitere Schutzgruppen erhalten bleiben.

Diese Umsetzungen erfolgen wie in den Beispielen beschrieben oder nach in der organischen Chemie üblichen Methoden.

Die weitere Umsetzung der Verbindungen der Formel (3), (4) oder (8) zu den Verbindungen der Formel (III) können beispielsweise durch die nachfolgenden Formelschemata erläutert werden:

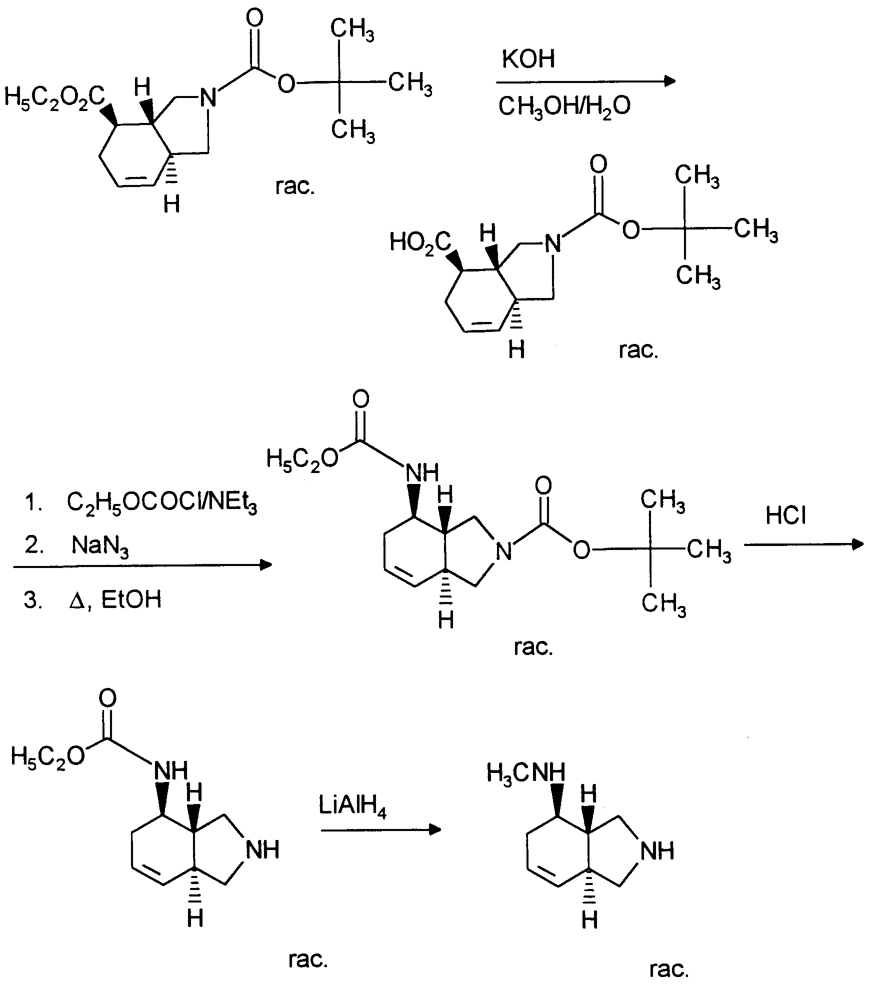

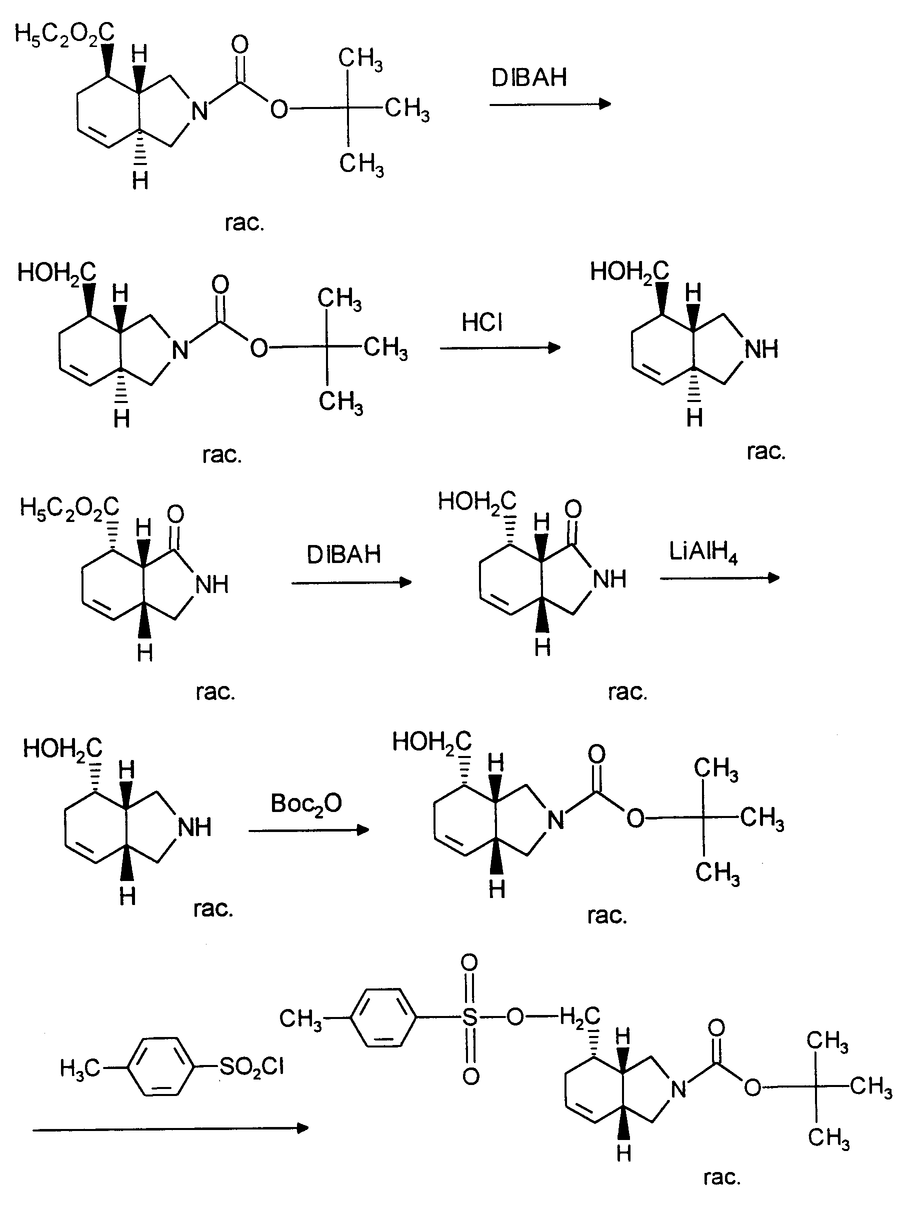

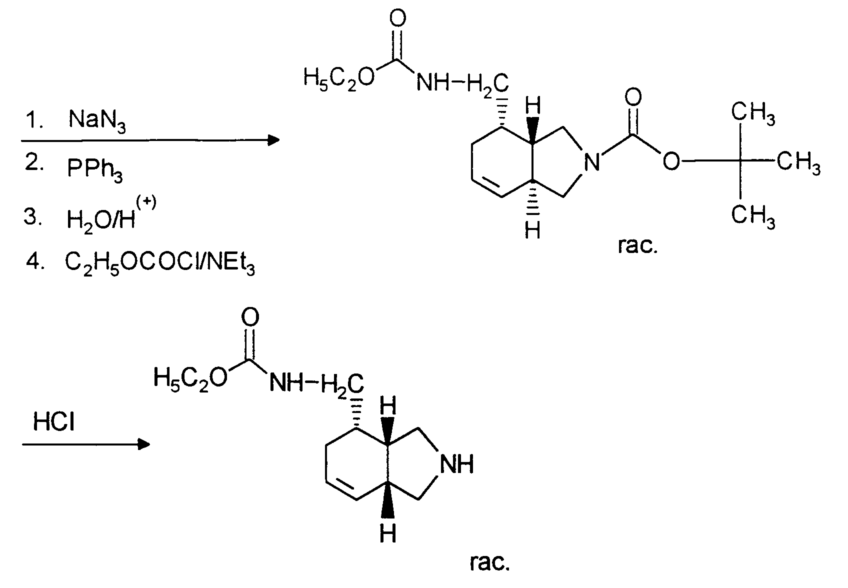

Die meisten Ausgangsstoffe der Formel (1), (2), (5) und (6) sind bekannt oder können nach bekannten Methoden der organischen Chemie hergestellt werden.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäue, Embonsäure, Glutaminsäure oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkung in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen unterhalb von Konzentrationen ähnlicher Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcusaureus,MicrococcusluteusundEnterococcusfaecalisbeobachtetwerden.

Auch gegenüber Bakterien, die gegenüber vergleichbaren Substanzen als weniger empfindlich eingestuft werden, insbesondere resistenten Staphylococcus aureus und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Injektions-und orale verabreichbare Lösungen, Suspensionen und Emulsionen, ferner Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µm/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu 9-Fluor-3-methyl-10-(4-methyl-1-piperazinyl)-7-oxo-2,3-dihydro-7H-pyrido[1,2,3-d,e]-[1,3,4]-benzoxadiazin-6-carbonsäure (EP-O 259 804) als Referenzverbindung aufgeführt.

**Tabelle**

| **MHK-Werte** | | | | | |
|---|---|---|---|---|---|
| Spezies | Stamm | Beispiel Nr. | | | Referenz |
| | | 5 | 6 | 8 | |
| E. coli | Neumann | 0.03 | ≤0.015 | ≤0.015 | ≤0.015 |
| | ATCC 25922 | 0.03 | ≤0.015 | ≤0.015 | ≤0.015 |
| Klebsiella pneumoniae | 8085 | 0.06 | ≤0.015 | 0.031 | 0.062 |
| | 63 | 0.06 | ≤0.015 | 0.031 | 0.062 |
| Providencia sp. | 12012 | 0.06 | ≤0.015 | 0.031 | 0.062 |
| | 12052 | 2 | 1 | 1 | 2 |
| Micrococcus luteus | 9341 | 0.125 | 0.031 | 0.062 | 2 |
| Staphylococcus aureus | ICB 25701 | 0.5 | 0.125 | 0.25 | 16 |
| | ATCC 29213 | 0.03 | ≤0.015 | ≤0.015 | 0.5 |
| | 133 | 0.03 | ≤0.015 | ≤0.015 | 0.5 |
| | ICB 25768 | 1 | 0.5 | 1 | 64 |
| Enterococcus faecalis | 27101 | 0.06 | ≤0.015 | 0.031 | 1 |
| | 9790 | 0.06 | ≤0.015 | 0.031 | 1 |

### Herstellung der Wirkstoffe

### Beispiel 1

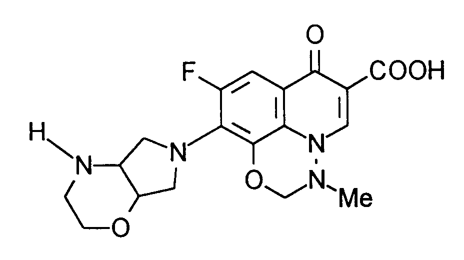

### 9-Fluor-3-methyl-10-(2-oxa-5,8-diazabicyclo[4.3.0]nonan-8-yl)-7-oxo-2,3-dihydro-7H-pyrido[1,2,3-d,e][1,3,4]benzoxadiazin-6-carbonsäure

500 mg (1.77 mmol) 9,10-Difluor-3-methyl-7-oxo-2,3-dihydro-7H-pyrido[1,2,3-d,e][1,3,4]-benzoxadiazin-6-carbonsäure werden mit 450 mg (3.51 mmol) 2-Oxa-5,8-diazabicyclo[4.3.0]nonan in 15 ml Pyridin acht Stunden unter Argon auf 100°C erwärmt. Die Mischung wird im Hochvakuum eingeengt, der Rückstand aus Ethanol umkristallisiert und getrocknet.
Ausbeute: 410 mg (59 % der Theorie)
Schmelzpunkt: 260-262°C (unter Zersetzung)

### Beispiel 2

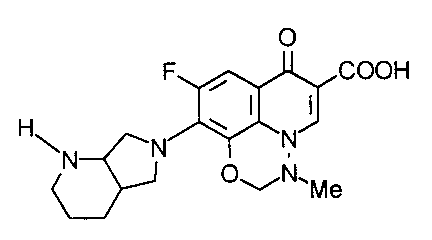

### 10-(2,8-Diazabicyclo[4.3.0]nonan-8-yl)-9-fluor-3-methyl-7-oxo-2,3-dihydro-7H-pyrido[1,2,3-d,e][1,3,4]benzoxadiazin-6-carbonsäure

Analog zum Beispiel 1 wird bei der Umsetzung mit 2,8-Diazabicyclo[4.3.0]nonan die Titelverbindung erhalten.
Schmelzpunkt: 256-258°C (unter Zersetzung)

### Beispiel 3

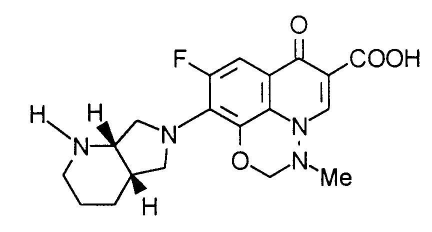

### 10-((1S,6S)-2,8-Diazabicyclo[4,3,0]nonan-8-yl)-9-fluor-3-methyl-7-oxo-2,3-dihydro-7H-pyrido[1,2,3-d,e][1,3,4]benzoxadiazin-6-carbonsäure

Analog zum Beispiel 1 wird bei der Umsetzung mit (1S,6S)-2,8-Diazabicyclo[4.3.0]nonan die Titelverbindung erhalten.
Schmelzpunkt: 255-257°C

### Beispiel 4

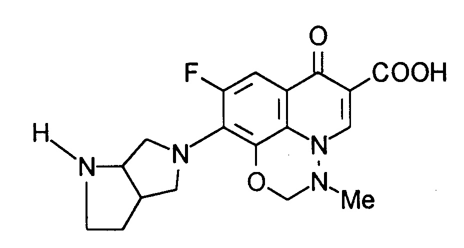

### 10-(2,7-Diazabicyclo[3.3.0]nonan-7-yl)-9-fluor-3-methyl-7-oxo-2,3-dihydro-7H-pyrido[1,2,3-d,e][1,3,4]benzoxadiazin-6-carbonsäure

100 mg (0.35 mmol) 9,10-Difluor-3-methyl-7-oxo-2,3-dihydro-7H-pyrido[1,2,3-d,e][1,3,4]-benzoxadiazin-6-carbonsäure werden mit 80 mg (0.71 mmol) 2,7-Diazabicyclo[3.3.0]nonan in 4 ml Pyridin vier Stunden unter Argon auf 100°C erwärmt. Die Mischung wird im Hochvakuum eingeengt, der Rückstand aus Ethanol umkristallisiert und getrocknet. Die erhaltene Titelverbindung ist mit ca. 15 % eines Regioisomeren kontaminiert.
Ausbeute: 60 mg (46 % der Theorie)
Schmelzpunkt: 220-224°C (unter Zersetzung)

### Beispiel 5

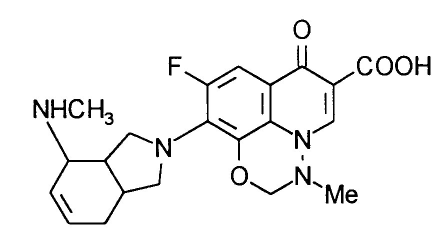

### 9-Fluor-3-methyl-10-(2-methylamino-8-azabicyclo[4.3.0]non-3-en-8-yl)-7-oxo-2,3-dihydro-7H-pyrido[1,2,3-de][1,3,4]benzoxadiazin-6-carbonsäure

150 mg (0,53 mmol) 9,10-Difluor-3-methyl-7-oxo-2,3-dihydro-7H-pyrido[1,2,3-de][1,3,4]-benzoxadiazin-6-carbonsäure werden mit 120 mg (0,79 mmol) 2-Methylamino-8-azabicyclo[4.3.0]non-3-en in 5 ml Pyridin vier Stunden unter Argon auf 100°C erwärmt. Die Mischung wird im Hochvakuum eingeengt, der Rückstand aus Methanol umkristallisiert und getrocknet.
Ausbeute: 118 mg (54 % der Theorie)
Schmelzpunkt: 233-235°C (unter Zersetzung)

### Beispiel 6

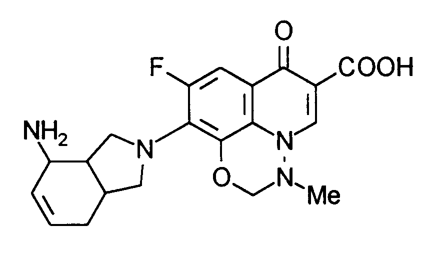

### 10-(2-Amino-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

Analog zum Beispiel 1 wird bei der Umsetzung mit 2-Amino-8-azabicyclo[4.3.0]non-3-en die Titelverbindung erhalten.
Schmelzpunkt: 246-250°C (unter Zersetzung)

### Beispiel 7

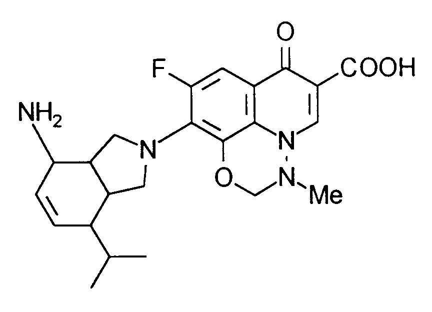

### 10-(2-Amino-5-isopropyl-8-azabicyclo[4.3.0]non-3-en-8-yl)-9-fluor-3-methyl-7-oxo-2,3-dihydro-7H-pyrido[1,2,3-d,e](1,3,4]benzoxadiazin-6-carbonsäure

Analog zum Beispiel 1 wird bei der Umsetzung mit 2-Amino-5-isopropyl-8-azabicyclo[4.3.0]non-3-en die Titelverbindung erhalten.
Schmelzpunkt: 194-199°C

### Beispiel 8

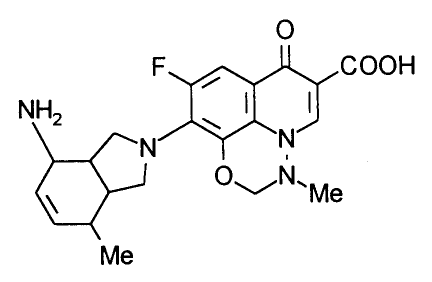

### 10-(2-Amino-5-methyl-8-azabicyclo[4.3.0]non-3-en-8-yl)-9-fluor-3-methyl-7-oxo-2,3-dihydro-7H-pyrido[1,2,3-d,e](1,3,4]benzoxadiazin-6-carbonsäure

150 mg (0,53 mmol) 9,10-Difluor-3-methyl-7-oxo-2,3-dihydro-7H-pyrido[1,2,3-d,e][1,3,4]benzoxadiazin-6-carbonsäure werden mit 120 mg (0,79 mmol) 2-Amino-5-methyl-8-azabicyclo[4.3.0]non-3-en in 5 ml Pyridin 4 Stunden unter Argon auf 100°C erwärmt. Die Mischung wird im Hochvakuum eingeengt, der Rückstand aus Methanol/Chloroform umkristallisiert und getrocknet.
Ausbeute: 90 mg (41 % der Theorie)
Schmelzpunkt: 231-233°C (unter Zersetzung)

### Beispiel 9

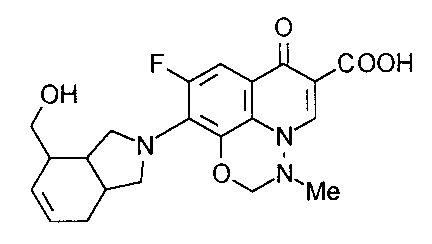

### 10-(2-Hydroxymethyl-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

150 mg (0,53 mmol) 9,10-Difluor-3-methyl-7-oxo-2,3-dihydro-7H-pyrido[1,2,3-d,e][1,3,4]benzoxadiazin-6-carbonsäure werden mit 120 mg (0,78 mmol) 2-Hydroxymethyl-8-azabicyclo[4.3.0]non-3-en in 5 ml Pyridin vier Stunden unter Argon auf 115°C erwärmt. Die Mischung wird im Hochvakuum eingeengt, der Rückstand aus Methanol umkristallisiert und getrocknet.
Ausbeute: 154 mg (70 % der Theorie)
Schmelzpunkt: 270-272°C (unter Zersetzung)

### Beispiel 10

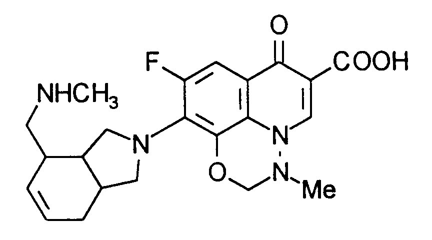

### 10-(2-Methylaminomethyl-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

Analog zum Beispiel 5 wird bei der Umsetzung mit 2-Methylaminomethyl-8-azabicyclo[4.3.0]non-3-en die Titelverbindung erhalten.
Schmelzpunkt: 272-274°C (unter Zersetzung)

### Beispiel 11

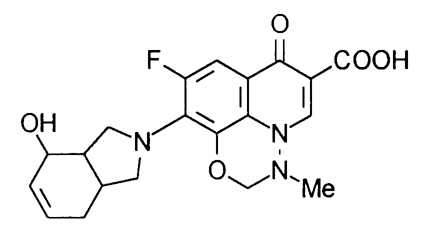

### 10-(2-Hydroxy-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

150 mg (0,53 mmol) 9,10-Difluor-3-methyl-7-oxo-2,3-dihydro-7H-pyrido[1,2,3-d,e][1,3,4]benzoxadiazin-6-carbonsäure werden mit 110 mg (0,79 mmol) 2-Hydroxy-8-azabicyclo[4.3.0]non-3-en in 5 ml Pyridin vierzehn Stunden unter Argon auf 115°C erwärmt. Die Mischung wird im Hochvakuum eingeengt, der Rückstand aus Methanol umkristallisiert und getrocknet.
Ausbeute: 63 mg (30 % der Theorie)
Schmelzpunkt: 248-250°C (unter Zersetzung)

### Beispiel 12

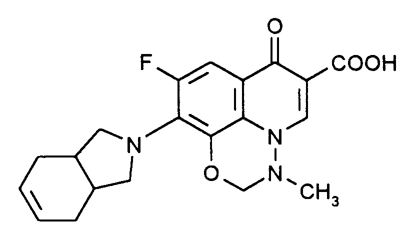

### 10-(8'-Azobicyclo[4,3,0]non-2'-en-8'-yl)-9-fluor-3-methyl-7-oxo-2,3-dihydro-7H-pyrido[1,2,3e][1,3,4]benzoxadiazin-6-carbonsäure

Ein Gemisch aus 846 mg (3,0 mmol) 9,10-Difluor-3-methyl-7-oxo-2,3-dihydro-7H-pyrido[1,2,3-d,e][1,3,4]benzoxadiazin-6-carbonsäure, 553 mg (4,5 mmol) 8-Azabicyclo[4,3,0]non-2-en (Produkt aus Beispiel A) und Pyridin (24 ml) wurde 4 h unter Stickstoff bei 100°C gerührt. Das Gemisch wurde am Hochvakuum eingeengt und das Rohprodukt mit Methanol verrührt, wiederrum abgesaugt und bei 60°C getrocknet.
Ausbeute: 850 mg (66 % der Theorie)
Schmelzpunkt: 309°C

### Beispiel 13

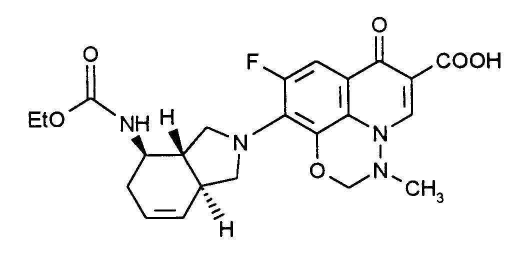

### (1'SR,2'RS,6'SR)-10-(2'-Ethyloxycarbonylamino-8'-azabicyclo[4,3,0]non-4'-en-8'-yl)-9-fluor-3-methyl-7-oxo-2,3-dihydro-7H-pyrido[1,2,3-d,e][1,3,4]benzoxadiazin-6-carbonsäure

Ein Gemisch aus 282 mg (1,0 mmol) 9,10-Difluor-3-methyl-7-oxo-2,3-dihydro-7H-pyrido[1,2,3-d,e][1,3,4]benzoxadiazin-6-carbonsäure, 315 mg (1,5 mmol) (1SR, 2RS, 6SR)-2'-Ethyloxycarbonylamino-8-azabicyclo[4,3,0]non-4-en (Produkt aus Beispiel C) und 8,5 ml Pyridin wird 4 h unter Stickstoff bei 100°C erwärmt. Anschließend wird im Hochvakuum eingeengt, der Rückstand mit Methanol verrührt, abgesaugt und bei 60°C getrocknet.
Ausbeute: 350 mg (74 % der Theorie)
Schmelzpunkt: 195°C

### Beispiel 14

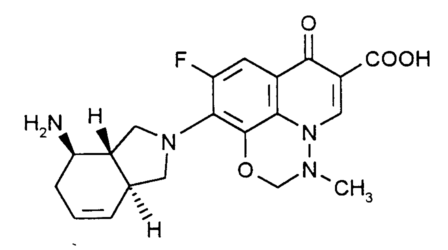

Ein Gemisch aus 480 mg (1,0 mmol) (1SR, 2RS, 6SR)-10-(2-Ethyloxycarbonylamino-8-azabicyclo[4,3]non-4'-en-8'-yl)-fluor-3-methyl-7-oxo-2,3-dihydro-7H-pyrido[1,2,3-d,e][1,3,4]benzoxadiazin-6-carbonsäure (Produkt aus Beispiel II), 640 mg (2,0 mmol) Bariumhydroxid-Octahydrat, Methanol (5 ml) und Wasser (2,5 mmol) wird 4 h auf 80°C erhitzt. Es wird nochmals die gleiche Menge Lösungsmittel ergänzt und weitere 36 h bei 80°C gerührt. Nach dem Abkühlen wird der Niederschlag abgesaugt, mit wenig Methanol und Wasser gewaschen und getrocknet. Der erhaltene Feststoff wird in 5 ml Wasser suspendiert und mit 1 N Salzsäure sauer gestellt. Der verbleibende Feststoff wird wiederum abgesaugt und getrocknet.
Ausbeute: 400 mg (98 % der Theorie)
Schmelzpunkt: > 300°C

### Beispiel 15

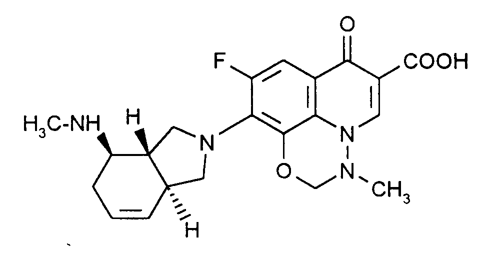

### (1'SR,2'RS,6'RS)-9-Fluor-3-methyl-10-(2'methylamino-8'-azabicyclo[4,3,0]non-4'en-8'-yl)-7-oxo-2,3-dihydro-7H-pyrido[1,2,3-d,e][1,3,4]-benzoxadiazin-6-carbonsäure

282 g (1,0 mmol) 9,10-Difluor-3-methyl-7-oxo-2,3-dihydro-7H-pyrido [1,2,3-d,e][1,3,4]benzoxadiazin-6-carbonsäure und 228 mg (1,5 mmol) (1SR, 2RS, 6RS)-2-Methylamino-8-azabicyclo[4,3,0]non-4-en (Produkt aus Beispiel N) werden in 8,5 ml Pyridin umgesetzt wie in Beispiel 12 beschrieben.
Ausbeute: 250 mg (61 % der Theorie)
Schmelzpunkt: 293°C.

### Beispiel 16

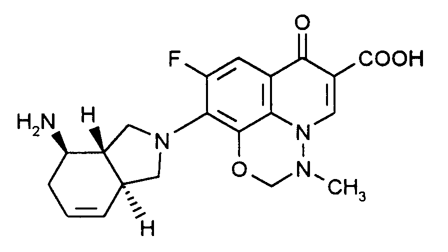

### (1'SR,2'RS,6'RS)-10-(2'-Amino-8'-azabicyclo[4,3,0]non-4'-en-8'-yl)-9-fluor-3-methyl-7-oxo-2,3-dihydro-7H-pyrido[1,2,3-d,e][1,3,4]-benzoxadiazin-6-carbonsäure

455 mg (1,6 mmol) 9,10-Difluor-3-methyl-7-oxo-2,3-dihydro-7H-pyrido [1,2,3-d,e][1,3,4]-benzoxadiazin-6-carbonsäure und 300 mg (2,1 mmol) 2-Amino-8-azabicyclo[4,3,0]non-4-en wurden in 10 ml Pyridin umgesetzt wie in Beispiel 12 beschrieben.
Ausbeute: 500 mg (78 % der Theorie)
Schmelzpunkt: 233°C

### Beispiel 17

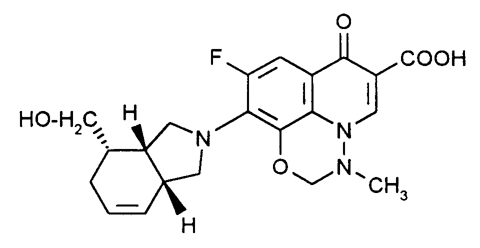

### (1'SR,2'RS,6'RS)-9-Fluor-10-(2'hydroxymethyl-8'-azabicyclo[4,3,0]non-4'-en-8'yl)-3-methyl-7-oxo-2,3-dihydro-7H-pyrido[1,2,3-d,e][1,3,4]-benzoxadiazin-6-carbonsäure

845 mg (3,0 mmol) 9,10-Difluor-3-methyl-7-oxo-2,3-dihydro-7H-pyrido [1,2,3 d,e][1,3,4]-benzoxadiazin-6-carbonsäure und 690 mg (4,5 mmol) 2-Hydroxymethyl-8-azabicyclo[4,3,0]non-4-en wurden in 24 ml Pyridin umgesetzt wie in Beispiel 12 beschrieben.
Ausbeute: 650 mg (52 % der Theorie)
Schmelzpunkt: 240°C

### Beispiel 18

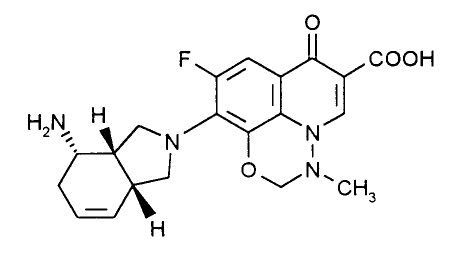

### (1'SR,2'RS,6'RS)-10-(2'-Amino-8'-azabicyclo[4,3,0]non-4'-en-8'-yl)-3-methyl-7-oxo-2,3-dihydro-7H-pyrido[1,2,3-d,e][1,3,4]-benzoxadiazin-6-carbonsäure

425 mg (1,5 mmol) 9,10-Difluor-3-methyl-7-oxo-2,3-dihydro-7H-pyrido [1,2,3-d,e][1,3,4]-benzoxadiazin-6-carbonsäure und 270 mg (20 mmol) 2-Amino-8-azabicyclo[4,3,0]non-4-en wurden in 10 ml Pyridin umgesetzt wie in Beispiel 12 beschrieben.
Ausbeute: 400 mg (67 % der Theorie)
Schmelzpunkt: 242°C

### Beispiel 19

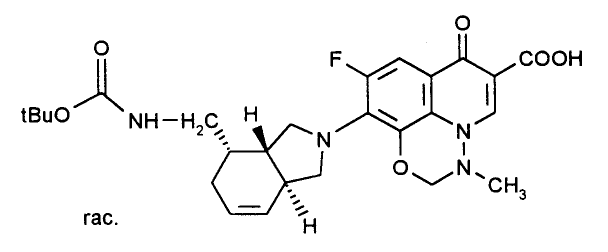

### (1'SR,2'RS,6'RS)-10-(2'-tert.-Butyloxycarbonylamino-8'-azabicyclo[4,3,0]non-4'-en-8'-yl)-9-fluor-3-methyl-7-oxo-2,3-dihydro-7H-pyrido[1,2,3-d,e][1,3,4]-benzoxadiazin-6-carbonsäure

469 mg (1,7 mmol) 9,10-Difluor-3-methyl-7-oxo-2,3-dihydro-7H-pyrido [1,2,3-d,e][1,3,4]-benzoxadiazin-6-carbonsäure und 665 mg (2,5 mmol) 2-tert.-Butyloxycarbonylamino-8-azabicyclo[4,3,0]non-4-en wurden in 15 ml Pyridin umgesetzt wie in Beispiel 12 beschrieben.
Ausbeute: 550 mg (67 % der Theorie)

### Beispiel 20

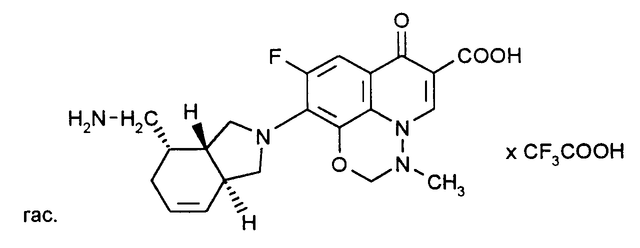

### (1'SR,2'RS,6'RS)-10-(2'-Amino-8'-azabicyclo[4,3,0]non-4'-en-8'-yl)-9-fluor-3-methyl-7-oxo-2,3-dihydro-7H-pyrido[1,2,3-d,e][1,3,4]-benzoxadiazin-6-carbonsäure-trifluoracetat

500 mg (9,0 mmol) der Titelverbindung aus Beispiel 19 werden in 10 ml eisgekühlter Trifluoressigsäure suspendiert, auf Raumtemperatur erwärmen gelassen. Nach 1 h bei Raumtemperatur wird das Produkt mit Methanol ausgefällt, abgesaugt und bei 50°C getrocknet.
Ausbeute: 500 mg (90 % der Theorie)
Schmelzpunkt: 247°C (Zers.)

### Beispiel 21

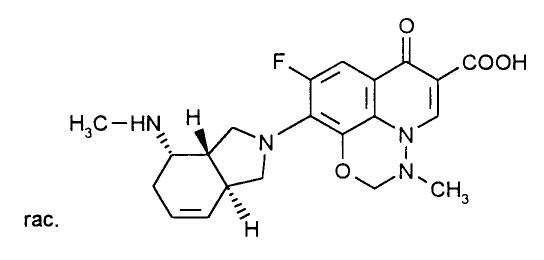

### (1'SR,2'RS,6'RS)-9-Fluor-10-(2'-methylamino-8'-azabicyclo[4,3,0]non-4'-en-8'-yl)-7-oxo-2,3-dihydro-7H-pyrido[1,2,3-d,e][1,3,4]-benzoxadiazin-6-carbonsäure

455 mg (1,6 mmol) 9,10-Difluor-3-methyl-7-oxo-2,3-dihydro-7H-pyrido [1,2,3-d,e][1,3,4]-benzoxadiazin-6-carbonsäure und 319 mg (2,1 mmol) 2-Methylamino-8-azabicyclo[4,3,0]non-4-en wurden in 10 ml Pyridin umgesetzt wie in Beispiel 12 beschrieben.
Ausbeute: 650 mg (98 % der Theorie)
Schmelzpunkt: 247°C

### Beispiel 22

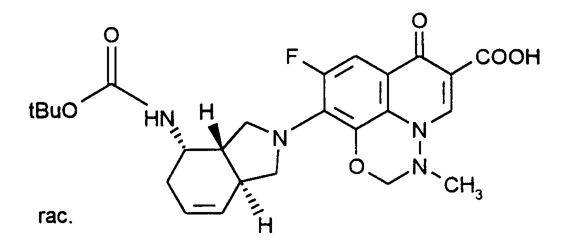

### (1'SR,2'RS,6'RS)-10-(2'-(tert.-Butyloxycarbonylamino)methyl-8'-azabicyclo[4,3,0]non-4'-en-8'-yl)-9-fluor-3-methyl-7-oxo-2,3-dihydro-7H-pyrido[1,2,3-d,e][1,3,4]-benzoxadiazin-6-carbonsäure

469 mg (1,7 mmol) 9,10-Difluor-3-methyl-7-oxo-2,3-dihydro-7H-pyrido [1,2,3-d,e][1,3,4]-benzoxadiazin-6-carbonsäure und 595 mg (2,5 mmol) 2-(tert.-Butyloxycarbonylamino)methyl-8-azabicyclo[4,3,0]non-4-en wurden in 15 ml Pyridin umgesetzt wie in Beispiel 12 beschrieben.
Ausbeute: 580 mg (98 % der Theorie)

### Beispiel 23

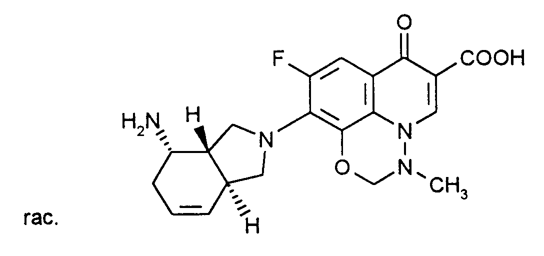

### (1'SR,2'RS,6'RS)-10-(2'-aminomethyl-8'-azabicyclo[4,3,0]non-4'-en-8'-yl)-9-fluor-3-methyl-7-oxo-2,3-dihydro-7H-pyrido[1,2,3-d,e][1,3,4]-benzoxadiazin-6-carbonsäure-trifluoracetat

500 mg (1,0 mmol) der Titelverbindung aus Beispiel 22 werden mit 10 ml Trifluoressigsäure umgesetzt wie in Beispiel 20 beschrieben.
Ausbeute: 480 mg (88 % der Theorie)
Schmelzpunkt: 251°C (Zers.)

### Herstellung der Zwischenprodukte:

### Beispiel A:

### 8-Azabicyclo[4.3.0]non-2-en

### A.1. (E)-1-Brom-2,4-pentadien

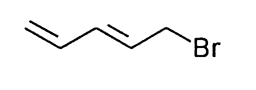

84 g (1,0 mol) 1,4-Pentadien-3-ol bei 0 °C vorlegen. Unter Rühren 150 ml (≈ 1,3 mol) 48 %-ige wäßrige Bromwasserstoffsäure so zutropfen, daß die Innentemperatur 5°C nicht überschreitet. Nach vollständiger Zugabe 1 h bei Raumtemperatur nachrühren. Die organische Phase wird abgetrennt und ohne Reinigung weiter umgesetzt.
Ausbeute: 107-129 g (73-88 % der Theorie)

### A.2. (E)-1-(2-Propenylamino)-2,4-pentadien

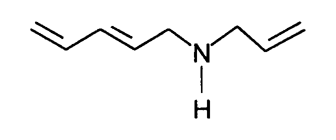

228 g (4,0 mol) 1-Amino-2-propen vorlegen. Unter Rühren 58,8 g (0,4 mol) (E)-1-Brom-2,4-pentadien (Titelverbindung aus Beispiel A.1.) zutropfen. Durch Kühlung die Innentemperatur im Bereich von 20-30°C halten. 5 h bei Raumtemperatur rühren. Ansatz bei 150 mbar einengen. 20 g (0,5 mol) Natriumhydroxid in 200 ml Wasser gelöst zugeben, mit zweimal je 100 ml Methylenchlorid extrahieren, mit Natriumsulfat trocknen, 0,1 g 4-Hydroxyanisol zusetzen, einengen, bei 40 mbar destillieren. Zur Stabilisierung werden dem Destillat 10-20 ppm 4-Hydroxyanisol zugesetzt.
Ausbeute: 33-35 g (67-72 % der Theorie)
Siedepunkt: 77-82 °C bei 40 mbar
¹H-NMR (CDCl₃): δ = 6,07-6,48 (m, 2H); 5,64-6,07 (m, 2H); 5,00-5,27 (m, 4H); 3,19-3,36 ppm (m, 4H).

### A.3. N-[(E)-2,4-Pentadienyl]-N-(2-propenyl)-acetamid

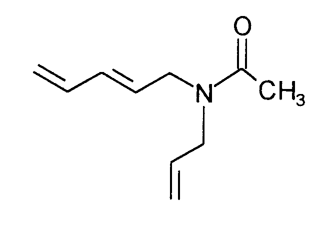

24,6 g (0,2 mol) (E)-1-(2-propenylamino)-2,4-pentadien (Titelverbindung aus Beispiel A.2.) vorlegen, 22,4 g Essigsäureanhydrid zutropfen und über Nacht bei Raumtemperatur rühren. Einengen und als Rohprodukt weiter umsetzen.

### A.4. 8-Acetyl-8-azabicyclo[4.3.0]non-2-en

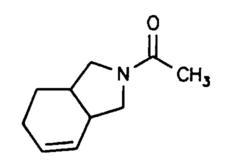

33,1 g (0,2 mol) N-[(E)-2,4-Pentadienyl]-N-(2-propenyl)-acetamid (Titelverbindung aus Beispiel A.3.) in 200 ml Xylol lösen, 15 min einen kräftigen Stickstoffstrom durchleiten, 0,1 g 4-Hydroxyanisol zusetzen, dann über Nacht zum Rückfluß erhitzen. Einengen, im Hochvakuum destillieren.
Ausbeute: 23,1 g (70 % der Theorie bezogen auf die Titelverbindung aus Beispiel A.2.)
Siedepunkt: 88-93 °C bei 0,05 mbar

### A. 5.8-Azabicyclo[4.3.0]non-2-en

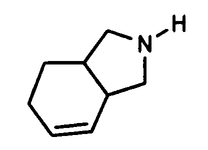

16,5 g (0,1 mol) 8-Acetyl-8-azabicyclo[4.3.0]non-2-en (Titelverbindung aus Beispiel A.4.) in einer Mischung aus 100 ml 45 %-iger Natronlauge, 50 ml Wasser und 100 ml 1,2-Ethandiol 3 h zum Rückfluß erhitzen. Nach Abkühlung viermal mit je 50 ml Diethylether extrahieren. Vereinigte organische Phasen mit Natriumsulfat trocknen, im Hochvakuum destillieren.
Ausbeute: 6,6 g (54 % der Theorie)
Siedepunkt: 36-44 °C bei 0,35 mbar
¹H-NMR (CDCl₃): δ = 5,79 (m, 1H); 5,74 (m, 1H); 3,02-3,17 (m, 2H); 2,47-2,72 (m, 2H); 2,06-2,30 (m, 2H); 1,91-2,06 (m, 2H); 1,68 (m, 1H); 1,45 ppm (m, 1H).

### Beispiel B:

### (1RS,2RS,6SR)-8-Azabicyclo[4.3.0]non-4-en-2-carbonsäureethylester (Diastereomer A) und (1RS,2RS,6RS)-8-Azabicyclo[4.3.0]non-4-en-2-carbonsäureethylester (Diastereomer B)

### B.1. N-[(E)-2,4-Pentadienyl]-phthalimid

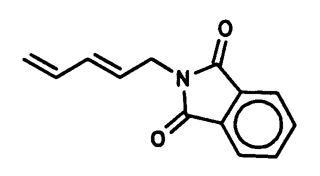

185 g (1,0 mol) Kaliumphthalimid in 800 ml DMF vorlegen. Unter Rühren 147 g (1,0 mol) (E)-1-Brom-2,4-pentadien (Titelverbindung aus Beispiel A.1.) zutropfen, dabei die Innentemperatur durch Kühlen unter 30°C halten. Über Nacht bei Raumtemperatur rühren. Anschließend den Ansatz unter Rühren auf 1,6 l Eiswasser gießen, den Niederschlag absaugen, mit Wasser waschen, bei Raumtemperatur bis zum Erreichen der Gewichtskonstanz trocknen.
Ausbeute: 177-200 g (83-94 % der Theorie)
Schmelzpunkt: 118-121°C (Probe aus Ethanol umkrist.)
¹H-NMR (CDCl₃): δ = 7,85 und 7,72 (m, 4H, Aryl-H); 6,2-6,4 (m, 2H, H an C-3 und C-4); 5,75 (dt, 1H, H an C-2, J = 14 und 6 Hz); 5,20 (d, 1H, Hₐ an C-S, J = 15 Hz); 5,10 (d, 1H, H_{b} an C-5, J = 8 Hz); 4,33 ppm (d, 2H, H an C-1, J = 6 Hz).

### B.2. (E)-1-Amino-2,4-pentadien

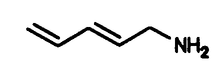

In einer 2l-Destillationsapparatur mit 10 cm Vigreuxkolonne werden 400 g Bis-(2-aminoethyl)-amin und 213 g (1.0 mol) N-[(E)-2,4-Pentadienyl]-phthalimid (Titelverbindung aus Beispiel B.1.) vorgelegt und bei 60 mbar zum Sieden erhitzt. Das Produkt destilliert im Bereich von 45-60 °C bei 60 mbar. Zur Stabilisierung werden dem Destillat 10-20 ppm 4-Hydroxyanisol zugesetzt.
Ausbeute: 71-80 g (86-96 % der Theorie)

### B.3. (E)-4-[(E)-2,4-Pentadienylamino]-2-butensäureethylester

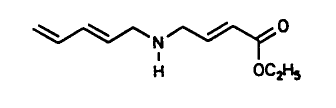

41,6 g (0,5 mol) (E)-1-Amino-2,4-pentadien (Titelverbindung aus Beispiel B.2.) und 50,6 g (0,5 mol) Triethylamin in 250 ml THF bei 0 °C vorlegen und 96,5 g (0,5 mol) (E)-4-Brom-2-butensäureethylester in 250 ml THF gelöst zutropfen.

Innentemperatur durch Eiskühlung unter 5°C halten. 5 h bei 0 °C anschließend über Nacht bei Raumtemperatur rühren. 500 ml MTBE, dann 500 ml 1M Natronlauge zugeben, schütteln, Phasentrennung, wäßrige Phase einmal mit 100 ml MTBE extrahieren, vereinigte organische Phasen mit Natriumsulfat trocknen, 100 ml Toluol und 0,1 g 4-Hydroxyanisol zusetzen, einengen (dabei Temperaturen über 40°C vermeiden). Rückstand an 1 kg Kieselgel (63-200 µm) mit Cyclohexan/Aceton 2:1 säulenchromatographisch reinigen. Vor dem Einengen erneut 0,1 g 4-Hydroxyanisol zusetzen und beim Einengen Temperaturen über 40°C vermeiden.
Ausbeute: 52,7-58,6 g (54-60 % der Theorie) gelbliches Öl
Rf = 0,24
¹H-NMR (CDCl₃): δ = 6,99 (dt, 1H, J = 15 und 5,5 Hz); 6,1-6,45 (m, 2H); 5,98 (d, 1H, J = 15 Hz); 5,75 (dt, 1H, J = 15 und 6,5 Hz), 5,18 (d, 1H, J = 15 Hz); 5,06 (d, 1H, J = 10 Hz); 4,19 (q, 2H); 3,42 (dd, 2H); 3,31 (d, 2H); 1,29 ppm (t, 3H).

### B.4. (1RS,2RS,6SR)-8-tert.-Butyloxycarbonyl-8-azabicyclo[4.3.0]non-4-en-2-carbonsäureethylester (Diastereomer A) und (1RS,2RS,6RS)-8-tert.-Butyloxycarbonyl-8-azabicyclo[4.3.0]non-4-en-2-carbonsäureethylester (Diastereomer B)

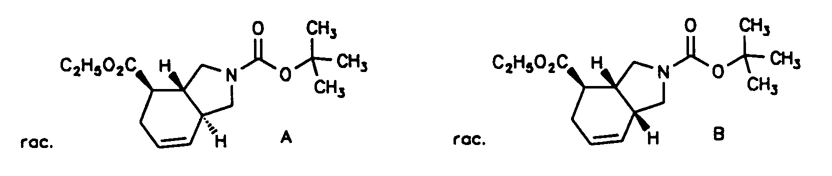

97,5 g (0,5 mol) (E)-4-[(E)-2,4-Pentadienylamino]-2-butensäureethylester (Titelverbindung aus Beispiel B.3.) in 250 ml Toluol gelöst vorlegen. 114,5 g (0,525 mol) Di-tert.-butyl-dicarbonat in 250 ml Toluol gelöst zutropfen, über Nacht bei Raumtemperatur rühren. Anschließend 15 min einen kräftigen Stickstoffstrom durchleiten, 0,1 g 4-Hydroxyanisol zusetzen, dann 6 h zum Rückfluß erhitzen. Einengen, Rückstand an 1 kg Kieselgel (63-200 µm) mit Cyclohexan/Aceton 8:1 säulenchromatographisch reinigen.
Ausbeute: 109-134 g (74-91 % der Theorie) gelbliches Öl; Gemisch aus zwei Diastereomeren A und B im Verhältnis A:B = 4:1
Rf = 0,25
¹H-NMR (Cl₂DC-CDCl₂; 80°C): δ = 5,77 (m, 1H(A) und 1H(B)); 5,68 (m, 1H(A) und 1H(B)); 4,14 (m, 2H(A) und 2H(B)); 3,65 (m, 2H(A) und 1H(B)); 3,48 (dd, 1H(B)); 3,27 (dd, 1H(B)); 3,00 (m, 1H(A) und 1H(B)); 2,85 (dd, 1H(A)); 2,76 (m, 1H(B)); 2,60 (m, 1H(A)); 2,25-2,55 (m, 3H(A) und 4H(B)); 1,93 (m, 1H(A)); 1,51 (s, 9H(B)); 1,44 (s, 9H(A)); 1,25 ppm (t, 3H(A) und 3H(B)).

### B.5. (1RS,2RS,6SR)-8-Azabicyclo[4.3.0]non-4-en-2-carbonsäureethylester (Diastereomer A) und (1RS,2RS,6RS)-8-Azabicyclo[4.3.0]non-4-en-2-carbonsäureethylester (Diastereomer B)

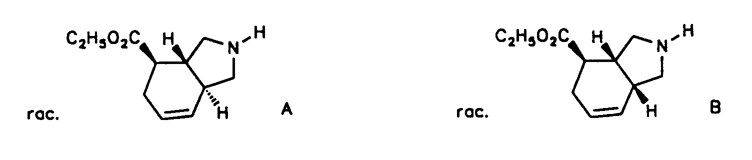

6,0 g (20 mmol) der Titelverbindung aus Beispiel B.4. in 20 ml Dioxan vorlegen. 20 ml konz. Salzsäure unter Kühlung so zutropfen, daß die Innentemperatur 30°C nicht übersteigt. Nach vollständiger Zugabe 10 min nachrühren. 40 ml Methylenchlorid zugeben und 40 ml 20 %-ige eisgekühlte Natronlauge unter Eiskühlung zutropfen. Organische Phase abtrennen, wäßrige Phase einmal mit Methylenchlorid extrahieren, vereinigte organische Phasen mit Natriumsulfat trocknen, einengen. 3,0 g Rohprodukt an 100 g Kieselgel (63-200 µm) mit Cyclohexan/Ethanol/17%-iges wäßriges Ammoniak (1:2:0,1) säulenchromatographisch reinigen.
- Ausbeute:: 0,8 g Diastereomer A und
0,8 g Diastereomer B
- Rf =: 0,79 Titelverbindung aus Beispiel B.4.
0,21 Diastereomer B
0,11 Diastereomer A
¹H-NMR (CDCl₃):
Diastereomer A: δ = 5,83 (d, 1H); 5,69 (m, 1H); 4,15 (q, 2H); 3,21-3,38 (m, 2H); 2,52-2,89 (m, 3H); 2,21-2,52 (m, 3H); 1,95 (m, 1H); 1,28 ppm (t, 3H).
Diastereomer B: δ = 5,64-5,87 (m, 2H); 4,16 (q, 2H); 3,14-3,33 (m, 2H); 2,82 (dd, 1H); 2,15-2,74 (m, 6H); 1,28 ppm (t, 3H).

### Beispiel C:

### (1SR,2RS,6SR)-2-Ethyloxycarbonylamino-8-azabicyclo[4.3.0]non-4-en

### C.1. (1RS,2RS,6SR)-8-tert.-Butyloxycarbonyl-8-azabicyclo-[4.3.0]non-4-en-2-carbonsäure

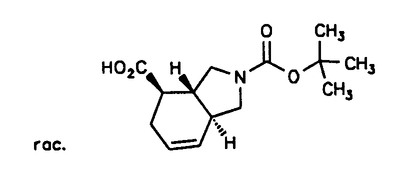

30,8 g (0,55 mol) Kaliumhydroxid in 500 ml Wasser gelöst vorlegen. 147,7 g (0,5 mol) der Titelverbindung aus Beispiel B.4. in 500 ml Methanol gelöst zugeben und 8 h bei 60°C unter einer Stickstoffatmosphäre rühren. Nach Abkühlung Reaktionslösung mit 500 ml Wasser verdünnen und unter Rühren 125 ml Essigsäure langsam zugießen. Nach vollständiger Zugabe 30 min im Eisbad stehen lassen, Niederschlag absaugen, mit Wasser nachwaschen, bei 50°C bis zur Gewichtskonstanz trocknen.
Ausbeute: 84-98 g (63-73 % der Theorie)
Schmelzpunkt: 174-176 °C (Probe aus Isopropanol/Wasser 1:1 umkristallisiert.)
¹H-NMR (Cl₂DC-CDCl₂; 80°C): δ = 5,83 (m, 1H, H an C-5); 5,74 (m, 1H, H an C-4); 3,65-3,80 (m, 2H, Hₐ an C-7 und Hₐ an C-9); 3,09 (dd, 1H, H_{b} an C-9); 2,92 (dd, 1H, H_{b} an C-7); 2,70 (m, 1H, H an C-2); 2,35-2,60 (m, 3H, Hₐ und H_{b} an C-3 und H an C-6); 2,01 (m, 1H, H an C-1); 1,5 ppm (s, 9H).

### C.2. (1SR,2RS,6SR)-8-tert.-Butyloxycarbonyl-2-ethyloxycarbonylamino-8-azabicyclo[4.3.0]non-4-en

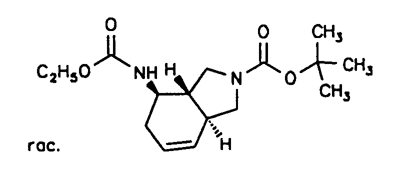

53,3 g (0,2 mol) der Titelverbindung aus Beispiel C.1. und 22,2 g (0,22 mol) Triethylamin in 200 ml wasserfreiem THF gelöst vorlegen. Unter Kühlung mit einer Eis/Kochsalz-Mischung 22,8 g (0,21 mol) Chlorameisensäureethylester in 40 ml THF gelöst so zutropfen, daß die Innentemperatur -10°C nicht überschreitet. Nach vollständiger Zugabe 1 h bei tiefer Temperatur nachrühren. Anschließend eine eisgekühlte Lösung von 15,6 g (0,24 mol) Natriumazid in 50 ml Wasser unter kräftigem Rühren so zutropfen, daß die Innentemperatur -10°C nicht überschreitet. Nach vollständiger Zugabe 30 min bei tiefer Temperatur nachrühren. Anschließend nacheinander 300 ml Wasser und 400 ml Toluol zugeben.

Die organische Phase abtrennen, mit Natriumsulfat trocknen, bei 15 mbar auf die Hälfte des ursprünglichen Volumens einengen (Badtemperatur unter 25°C). Zugabe von 100 ml Ethanol, unter Rühren langsam aufheizen (in dem Maße, wie es die Stickstoffentwicklung zuläßt) und nach beendeter Stickstoffentwicklung 4 h zum Rückfluß kochen. Einengen und Rohprodukt aus Methanol/Wasser 85:15 umkristallisieren, bei 50 °C bis zur Gewichtskonstanz trocknen.
Ausbeute: 24,2-28,5 g (39-46 % der Theorie) der Titelverbindung
Schmelzpunkt: 120-122 °C
¹H-NMR (CDCl₃): δ = 5,78 und 5,73 (2d, 1H, H an C-5); 5,64 (m, 1H, H an C-4); (4,59 br. s, 1H, NH); 4,12 (m, 2H, Ethoxy-CH₂); 3,90 (m, 1H, H an C-2); 3,74 und 3,67 (2m, 1H, Hₐ an C-7); 3,67 und 3,56 (2m, 1H, Hₐ an C-9); 3,12 (m, 1H, H_{b} an C-9); 2,92 (m, 1H, H_{b} an C-7); 2,67 (m, 1H, Hₐ an C-3); 2,49 (m, 1H, H an C-6); 1,95 (m, 1H, H_{b} an C-3); 1,83 (m, 1H, H an C-1); 1,46 (s, 9H); 1,24 ppm (m, 3H, Ethoxy-CH₃).

Die wäßrige Phase durch Zugabe von 10 %-iger Salzsäure auf einen pH von 2-3 einstellen, 30 min im Eisbad stehen lassen, den Niederschlag absaugen, mit Wasser waschen, bei 50 °C bis zur Gewichtskonstanz trocknen.
- Ausbeute:: 16,0-19,2 g (30-36 % der Titelverbindung aus Beispiel C.1.) (zurückgewonnene Ausgangsverbindung)

### C.3. (1SR,2RS,6SR)-2-Ethyloxycarbonylamino-8-azabicyclo[4.3.0]non-4-en

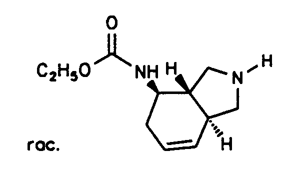

31,0 g (0,1 mol) der Titelverbindung aus Beispiel C.2. in 100 ml einer Mischung aus Methanol/Wasser (1:1) vorlegen (Suspension). 100 ml konz. Salzsäure rasch zulaufen lassen (leicht exotherm bis etwa 40°C, (man erhält eine homogene Lösung) und bis zum Ende der Gasentwicklung (etwa 10 min) nachrühren. 200 ml Eiswasser zugeben und 70 ml 45 %-ige Natronlauge unter Rühren und Eiskühlung zutropfen. Viermal mit je 50 ml Methylenchlorid extrahieren, die vereinigten organischen Phasen mit Natriumsulfat trocknen, einengen, im Hochvakuum Lösungsmittelreste abziehen. Die Substanz wird beim Einengen fest.
- Ausbeute:: 13,7-16,6 g (65-79 % der Theorie) braun-rosafarbener, amorpher Feststoff
- Rf =: 0,81 Titelverbindung aus Beispiel C.2. 0,11 Titelverbindung
Methylenchlorid/Methanol/17 %-iges wäßriges Ammoniak (15:4:0,5)
¹H-NMR (CDCl₃): δ = 5,78 (d, 1H, H an C-5); 5,63 (m, 1H, H an C-4); 4,94 (br.d, 1H, NH); 4,10 (m, 2H, Ethoxy-CH₂); 3,88 (m, 1H, H an C-2); 3,28 (m, 1H, Hₐ an C-7); 3,19 (m, 1H, Hₐ an C-9); 2,84 (m, 1H, H_{b} an C-9); 2,57-2,62 (m, 2H, Hₐ an C-3 und H_{b} an C-7); 2,43 (m, 1H, H an C-6); 1,95 (m, 1H, H_{b} an C-3); 1,79 (m, 1H, H an C-1); 1,23 ppm (m, 3H, Ethoxy-CH₃).

### Beispiel D:

### (1SR,2RS,6SR)-2-Methylamino-8-azabicyclo[4.3.0]non-4-en

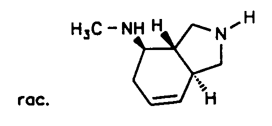

1,9 g (50 mmol) Lithiumaluminiumhydrid in 25 ml wasserfreiem Diethylether in einer Stickstoffatmosphäre vorlegen. 5,25 g (25 mmol) der Titelverbindung aus Beispiel C.3. in 50 ml wasserfreiem Tetrahydrofuran gelöst zutropfen und 3 h zum Rückfluß erhitzen. Weitere 0,95 g (25 mmol) Lithiumaluminiumhydrid zusetzen und nochmals 3 h zum Rückfluß erhitzen. Unter Eiskühlung langsam Wasser zutropfen, bis ein weißer Niederschlag entstanden ist. Niederschlag absaugen, zweimal mit je 100 ml Ethanol auskochen. Ethanolextrakte mit der Mutterlauge der Reaktion vereinigen, 50 ml Toluol zusetzen, einengen, Lösungsmittelreste im Hochvakuum abziehen.
- Ausbeute:: 1,95 g (77 % der Theorie) amorpher Feststoff
- Rf =: 0,11
Methylenchlorid/Methanol/17 %-iges wäßriges Ammoniak (2:4:1)
¹H-NMR (CDCl₃): δ = 5,77 (d, 1H, H an C-5); 5,67 (m, 1H, H an C-4); 3,33 (dd, 1H, Hₐ an C-7); 3,26 (dd, 1H, Hₐ an C-9); 2,73-2,82 und 2,54-2,63 (2m, 4H, H an C-2, Hₐ an C-3, H_{b} an C-7 und H_{b} an C-9); 2,41 (s, 3H, CH₃N); 2,34 (m, 1H, H an C-6); 1,90 (m, 1H, H_{b} an C-3); 1,70 ppm (m, 1H, H an C-1).

### Beispiel E:

### (1RS,2RS,6SR)-2-Hydroxymethyl-8-azabicyclo[4.3.0]non-4-en

### E.1. (1RS,2RS,6SR)-8-tert.-Butyloxycarbonyl-2-hydroxymethyl-8-azabicyclo[4.3.0]non-4-en (Diastereomer A) und (1RS,2RS,6RS)-8-tert.-Butyloxycarbonyl-2-hydroxymethyl-8-azabicyclo[4.3.0]non-4-en (Diastereomer B)

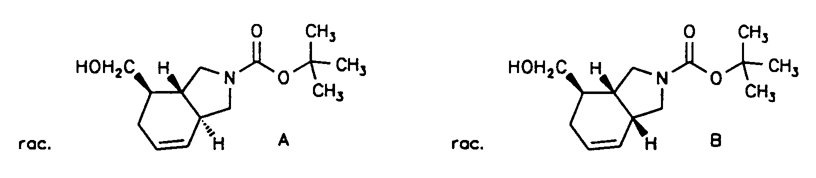

29,5 g (0,1 mol) der Titelverbindung aus Beispiel B.4. in 200 ml wasserfreiem 1,2-Dimethoxyethan in einer Stickstoffatmosphäre vorlegen. Bei einer Innentemperatur <-65°C 150 ml einer 1,5 m DIBAH-Lösung in Toluol (0,225 mol) zutropfen. Nach vollständige Zugabe Kühlbad entfernen und auf Raumtemperatur kommen lassen. 2 h bei Raumtemperatur nachrühren.

Unter kräftigem Rühren 60 ml Methanol zutropfen (exotherme Reaktion); Innentemperatur durch Kühlung mit einem Kaltwasserbad zwischen 35 und 45 °C halten. Anschließend 20 ml 5 %-ige Natronlauge zutropfen. Nach vollständiger Zugabe 10 min nachrühren. Niederschlag absaugen, zweimal unter Rühren mit je 150 ml Ethanol auskochen, Ethanolextrakte und Reaktionslösung vereinigen, einengen, im Hochvakuum Lösungsmittelreste abziehen, Rückstand an 250 g Kieselgel (63-200 µm) mit Cyclohexan/Aceton (4:1) säulenchromatographisch reinigen.
- Ausbeute:: 12,9-17,7 g (51-70 % der Theorie) gelbliches Öl; Gemisch der Diastereomeren A und B im Verhältnis 4:1
- Rf =: 0,36 Titelverbindung aus Beispiel B.4.
0,12 Titelverbindung A und B

Das Rohprodukt wird nach längerem Stehen fest. Durch Umkristallisieren aus Ether/Petrolether kann eine diastereomerenreine Probe des Hauptdiastereomeren A erhalten werden.
¹H-NMR (CDCl₃): (Diastereomer A) δ = 5,67-5,82 (m, 2H, H an C-4 und C-5); 3,50-3,77 (m, 4H, Hₐ an C-7, Hₐ an C-9 und Hydroxymethyl-CH₂); 3,02 (dt, 1H, H_{b} an C-9); 2,85 (m, 1H, H_{b} an C-7); 2,2-2,4 (m, 3H); 1,87-2,00 (m, 3H); 1,62 (m, 1H, H an C-1); 1,46 ppm (s, 9H).

### E.2. (1RS,2RS,6SR)-2-Hydroxymethyl-8-azabicyclo[4.3.0]non-4-en

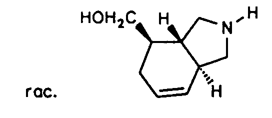

2,5 g (10 mmol) der Titelverbindung A aus Beispiel E.1. in 10 ml Methanol vorlegen. 10 ml konz. Salzsäure rasch zulaufen lassen und 30 min nachrühren. Mit Wasser auf das doppelte Volumen verdünnen dann 45 %-ige Natronlauge unter Rühren und Eiskühlung zutropfen, bis zu einem pH-Wert von ≥12. Einengen, Rückstand zweimal unter Rühren mit Ethanol auskochen, Ethanolextrakte einengen, im Hochvakuum Lösungsmittelreste abziehen.
- Ausbeute:: 2,1 g (Produkt enthält NaCl-Rückstände)
- Rf =: 0,20
Methylenchlorid/Methanol/17 %-iges wäßriges Ammoniak (2:4:1)
¹H-NMR (d₆-DMSO): δ = 5,76 (d, 1H); 5,62 (d, 1H); 3,47-3,56 (m, 2H, Hₐ an C-7 und Hₐ an C-9); 3,32-3,47 (m, 1H, Hₐ von Hydroxymethyl-CH₂); 3,23-3,32 (m, 1H, H_{b} von Hydroxymethyl-CH₂); 2,77 (t, 1H, H_{b} an C-9); 2,64 (t, 1H, H_{b} an C-7); 2,10-2,24 (m, 2H, Hₐ an C-3 und H an C-6); 1,77-1,88 (m, 1H, H_{b} an C-3); 1,69 (m, 1H, H an C-2); 1,40 ppm (m, 1H, H an C-1).

### Beispiel F:

### (1RS,2RS,6SR)-2-Ethyloxycarbonylaminomethyl-8-azabicyclo[4.3.0]non-4-en

### F.1. (1RS,2RS,6SR)-8-tert.-Butyloxycarbonyl-2-(4-toluolsulfonyloxymethyl)-8-azabicyclo[4.3.0]non-4-en (Diastereomer A) und (1RS,2RS,6RS)-8-tert.-Butyloxycarbonyl-2-(4-toluolsulfonyloxymethyl)-8-azabicyclo[4.3.0]non-4-en (Diastereomer B)

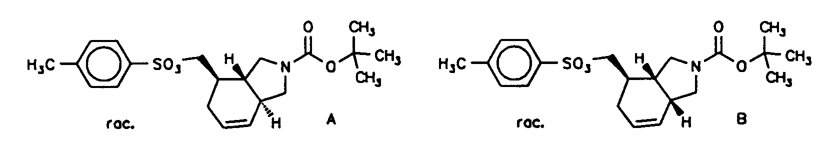

12,7 g (0,05 mol) der Titelverbindung aus Beispiel E.1. (Rohgemisch der Diastereomere A und B) in 25 ml wasserfreiem Pyridin vorlegen und auf -15°C kühlen. 11,0 g (0,0575 mol) 4-Toluolsulfonsäurechlorid portionsweise so zugeben, daß die Innentemperatur -5°C nicht übersteigt. Nach vollständiger Zugabe 2 h bei einer Temperatur von -5 bis -15 °C, dann 3 h bei Raumtemperatur nachrühren. 5 g Eis zugeben, 5 min rühren, auf 50 ml Wasser geben, Niederschlag absaugen, mit Wasser waschen, bei 50 °C bis zur Gewichtskonstanz trocknen.
- Ausbeute:: 14,4-16,3 g (71-80 % der Theorie)
blaßrosafarbener Feststoff
Gemisch der Diastereomeren A und B

Durch Umkristallisieren aus Methanol kann eine diastereomerenreine Probe des Hauptdiastereomeren A erhalten werden.
Schmelzpunkt: 111-113 °C
¹H-NMR (CDCl₃): (Diastereomer A) δ = 7,79 (m, 2H, Aryl-H); 7,36 (d, 2H, Aryl-H); 5,74 und 5,78 (2d, 1H, H an C-5); 5,64 (m, 1H, H an C-4); 3,87-3,97 (m, 2H Tosyl-OCH₂-); 3,59 und 3,67 (2dd, 1H, Hₐ an C-7); 3,48 (dd, 1H, Hₐ an C-9); 2,78-2,96 (m, 2H, H_{b} an C-7 und H_{b} an C-9); 2,47 (s, 3H, Aryl-CH₃); 2,22-2,36 (m, 2H, Hₐ an C-3 und H an C-6); 2,06 (m, 1H, H an C-2); 1,80-1,98 (m, 1H, H_{b} an C-3); 1,59 (m, 1H, H an C-1); 1,45 und 1,47 ppm (2s, 9H).

### F.2. (1RS,2RS,6SR)-8-tert.-Butyloxycarbonyl-2-ethyloxycarbonylaminomethyl-8-azabicyclo[4.3.0]non-4-en (Diastereomer A) und (1RS,2RS,6RS)-8-tert.-Butyloxycarbonyl-2-ethyloxycarbonylaminomethyl-8-azabicyclo[4.3.0]non-4-en (Diastereomer B)

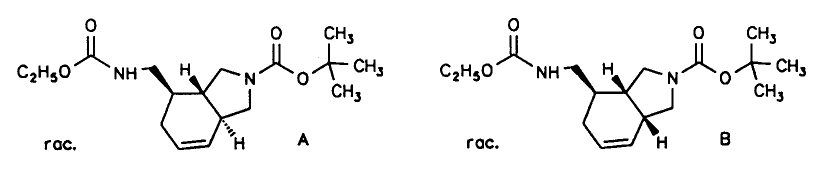

20,5 g (0,05 mol) der Titelverbindung aus Beispiel F.1. (Rohgemisch der Diastereomere A und B) und 6,5 g (0,1 mol) Natriumazid in 100 ml DMF 4 h auf 70 °C erhitzen. Reaktionslösung auf 200 ml Wasser geben, einmal mit 200 ml Petrolether extrahieren, die Petroletherphase einmal mit 50 ml Wasser waschen, mit Natriumsulfat trocknen und bei Raumtemperatur einengen.

Den Rückstand in 80 ml THF aufnehmen und 13,1 g (0,05 mol) Triphenylphosphin in 80 ml THF gelöst zutropfen. Nach vollständiger Zugabe 20 h bei Raumtemperatur rühren, dann 150 ml Wasser langsam zutropfen, nach vollständiger Zugabe 15 min nachrühren. Unter Kühlung Salzsäure zutropfen (konz. HCl/Wasser 1:3), bis ein pH-Wert von 3-4 erreicht ist, THF bei Raumtemperatur im Vakuum abziehen, Reaktionslösung auf 0°C kühlen, ausgefallenes Triphenylphosphinoxid absaugen (oder mit MTBE aufnehmen, falls ölig).

Wäßrige Phase durch Zugabe von 10 %-iger Natronlauge auf einen pH-Wert ≥12 einstellen, zweimal mit je 100 ml Methylenchlorid extrahieren, vereinigte Extrakte mit Natriumsulfat trocknen, anschließend 6,0 g (0,06 mol) Triethylamin zugeben, unter Rühren 6,0 g (0,055 mol) Chlorameisensäureethylester in 20 ml Methylenchlorid gelöst zutropfen, über Nacht bei Raumtemperatur rühren, Reaktionslösung einmal mit 100 ml Wasser waschen, mit Natriumsulfat trocknen und einengen. 23 g Rohprodukt an 100 g Kieselgel (63-200 µm) mit Cyclohexan/ Aceton (4:1) säulenchromatographisch reinigen.
- Ausbeute:: 12,4 g (76 % der Theorie) zähes Öl
Gemisch der Diastereomeren A und B
- Rf-Werte (Cyclohexan/Aceton 2:1):: 0,32 Diastereomer A
0,29 Diastereomer B

Die Diastereomere A und B werden an 250 g Kieselgel (35-70 µm) mit Cyclohexan/Aceton (8:1) säulenchromatographisch getrennt.
- Ausbeute:: 4,3 g (26 % der Theorie) Diastereomer A (zähes Öl)
2,4 g (15 % der Theorie) Mischfraktion
0,6 g (4 % der Theorie) Diastereomer B
¹H-NMR (Cl₂DC-CDCl₂; 80 °C):
Diastereomer A: δ = 5,75 (d, 1H, H an C-5); 5,66 (m, 1H, H an C-4); 4,67 (br, 1H, NH); 4,08 (q, 2H, Ethoxy-CH₂); 3,62 (br, 2H, Hₐ an C-7 und Hₐ an C-9); 3,19 (br, 1H, Hₐ an CH₂-NH); 3,05 (br, H_{b} an CH₂-NH); 2,96 (dd, 1H, H_{b} an C-9); 2,81 (dd, 1H, H_{b} an C-7); 2,24-2,34 (m, 2H, Hₐ an C-3 und H an C-6); 1,78-1,94 (m, 2H, H an C-2 und H_{b} an C-3); 1,54 (m, 1H, H an C-1); 1,43 (s, 9H); 1,22 ppm (t, 3H, Ethoxy-CH₃).

Diastereomer B: δ = 5,69 (m, 1H, H an C-4); 5,57 (m, 1H, H an C-5); 4,65 (br, 1H, NH); 4,08 (q, 2H, Ethoxy-CH₂); 3,52 (dd, 1H, Hₐ an C-7); 3,41 (dd, 1H, Hₐ an C-9); 3,29 (dd, 1H, H_{b} an C-9); 3,24 (dd, 1H, Hₐ an CH₂-NH); 3,03-3,12 (m, 2H, H_{b} an C-7 und H_{b} an CH₂-NH); 2,68 (m, 1H, H an C-6); 2,12-2,22 (m, 2H, H an C-1 und Hₐ an C-3); 1,74-1,87 (m, 2H, H an C-2 und H_{b} an C-3); 1,43 (s, 9H); 1,22 ppm (t, 3H, Ethoxy-CH₃).

### F.3. (1RS,2RS,6SR)-2-Ethyloxycarbonylaminomethyl-8-azabicyclo [4.3.0]non-4-en

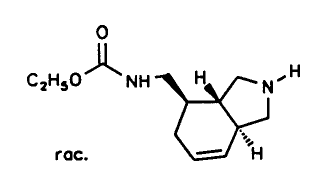

1,6 g (5,7 mmol) der Titelverbindung A aus Beispiel F.2. in 10 ml Methanol vorlegen. 8 ml konz. Salzsäure rasch zulaufen lassen und 30 min nachrühren. Mit Wasser auf das doppelte Volumen verdünnen dann 45 %-ige Natronlauge unter Rühren und Eiskühlung zutropfen, bis zu einem pH-Wert von ≥12. Viermal mit Methylenchlorid extrahieren, die vereinigten organischen Phasen mit Natriumsulfat trocknen, einengen, im Hochvakuum Lösungsmittelreste abziehen.
- Ausbeute:: 0,8 g (63 % der Theorie) zähes Öl
- Rf =: 0,16
Methylenchlorid/Methanol/17 %-iges wäßriges Ammoniak (15:4:0,5)
¹H-NMR (CDCl₃): δ = 5,81 (d, 1H, H an C-5); 5,67 (m, 1H, H an C-4); 5,00 (br, 1H, NH); 4,10 (q, 2H, Ethoxy-CH₂); 3,18-3,28 und 3,08 (m, 3H und m, 1H: Hₐ an C-7, Hₐ an C-9, Hₐ und H_{b} an CH₂-NH-CO); 2,67 (dd, 1H, H_{b} an C-9); 2,53 (dd, 1H, H_{b} an C-7); 2,34 (m, 1H, Hₐ an C-3); 2,25 (m, 1H, H an C-6); 1,79-1,96 (m, 2H, H an C-2 und H_{b} an C-3); 1,50 (m, 1H, H an C-1); 1,24 ppm (t, 3H, Ethoxy-CH₃).

### Beispiel G:

### (1RS,2SR,6RS)-2-Hydroxymethyl-8-azabicyclo[4.3.0]non-4-en

### G.1. (E)-1-tert.-Butyloxycarbonylamino-2,4-pentadien

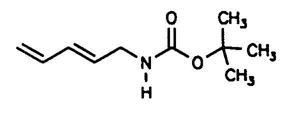

8,3 g (0,1 mol) (E)-1-Amino-2,4-pentadien (Titelverbindung aus Beispiel B.2.) in 50 ml MTBE vorlegen und 20 mg 4-Hydroxyanisol zusetzen. Anschließend bei einer Innentemperatur von 20-30°C 22,9 g (0,105 mol) Di-tert.-butyl-dicarbonat in 50 ml MTBE gelöst zutropfen. Nach vollständiger Zugabe 20 h bei Raumtemperatur rühren. Einengen, Reste von Di-tert.-butyl-dicarbonat im Hochvakuum bei 40 °C abziehen.
Ausbeute: 18,9 g (Rohprodukt) farbloses Öl
Rf = 0,25
Cyclohexan/Aceton (4:1)
¹H-NMR (CDCl₃): δ = 6,05-6,43 (m, 2H, H an C-3 und C-4); 5,68 (dd, 1H, H an C-2, J= 14 und 6 Hz); 5,17 (dd, 1H, Hₐ an C-5, J = 16 Hz); 5,07 (dd, 1H, H_{b} an C-5, J = 10 Hz); 4,75 (br, 1H, NH); 3,77 (t, 2H, H an C-1); 1,45 ppm (s, 9H).

### G.2. (1RS,2RS,6RS)-2-tert.-Butyloxycarbonylaminomethyl-7,9-dioxo-8-oxabicyclo[4.3.0]non-3-en

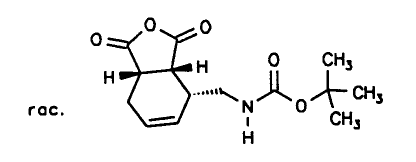

83,2 g (1,0 mol) (E)-1-Amino-2,4-pentadien (Titelverbindung aus Beispiel B.2.) in 250 ml MTBE vorlegen und 0,1 g 4-Hydroxyanisol zusetzen. Anschließend bei einer Innentemperatur von 20-30°C 229,2 g (1,05 mol) Di-tert.-butyl-dicarbonat in 250 ml MTBE gelöst zutropfen. Nach vollständiger Zugabe 20 h bei Raumtemperatur rühren. Reaktionsgemisch einengen und in 1 l Toluol aufnehmen. 103,0 g (1,05 mol) Maleinsäureanhydrid zusetzen und 24 h bei einer Innentemperatur von 60°C rühren. Niederschlag absaugen, mit Toluol waschen und bei 50°C bis zur Gewichtskonstanz trocknen.
- Ausbeute:: 208,2 g (74 % der Theorie)
weißer, kristalliner Feststoff
- Schmelzpunkt:: 157-159 °C
¹H-NMR (d₆-DMSO): δ = 5,81 (m, 1H, H an C-4); 5,59 (d, 1H, H an C-3); 3,77 (dd, 1H Hₐ an CH₂-NH); 3,44 (m, 2H, H an C-1 und H_{b} an CH₂-NH); 2,94 (m, 1H, H an C-2); 2,66 (m, 1H, H an C-6); 2,16 (m, 1H, Hₐ an C-5); 2,06 (m, 1H, H_{b} an C-5); 1,43 ppm (s, 9H).

### G.3. (1RS,2SR,6RS)-9-Oxo-8-azabicyclo[4.3.0]non-4-en-2-carbonsäuremethylester

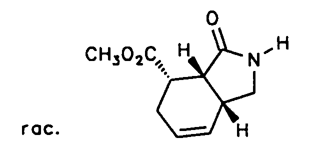

83,2 g (1,0 mol) (E)-1-Amino-2,4-pentadien (Titelverbindung aus Beispiel B.2.) in 250 ml THF vorlegen und 0,1 g 4-Hydroxyanisol zusetzen. Anschließend bei einer Innentemperatur von 20-30°C 229,2 g (1,05 mol) Di-tert.-butyl-dicarbonat in 250 ml THF gelöst zutropfen. Nach vollständiger Zugabe 20 h bei Raumtemperatur rühren. 103,0 g (1,05 mol) Maleinsäureanhydrid zusetzen und 5 h zum Rückfluß erhitzen. Einengen und den Rückstand in 500 ml Methanol aufnehmen, 30 ml p-Toluolsulfonsäure zusetzen, dann erneut 5 h zum Rückfluß erhitzen. Nach Abkühlung unter Eiskühlung und Rühren eine Lösung von 20 g Natriumcarbonat in 500 ml Wasser gelöst rasch zutropfen, Ansatz noch 30 min im Eisbad stehen lassen, Niederschlag absaugen, mit wenig Wasser waschen, bei 50°C bis zur Gewichtskonstanz trocknen.
- Ausbeute:: 125-148 g (64-76 % der Theorie)
weißer, kristalliner Feststoff
- Schmelzpunkt:: 190-193 °C
¹H-NMR (d₆-DMSO): δ = 7,50 (s, 1H, NH); 5,77 (m, 1H, H an C-4); 5,56 (m, 1H, H an C-5); 3,60 (s, 3H, CH₃O); 3,42 (dd, 1H, Hₐ an C-7); 3,16 (dd, 1H, H an C-1); 3,00 (m, 1H, H an C-6); 2,88 (dd, 1H, H_{b} an C-7); 2,67 (m, 1H, H an C-2); 2,02-2,18 ppm (m, 2H, Hₐ und H_{b} an C-3).

### G.4. (1RS,2SR,6RS)-2-Hydroxymethyl-8-azabicyclo[4.3.0]non-4-en

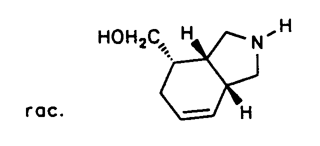

19,6 g (0,1 mol) der Titelverbindung aus Beispiel G.3. in 100 ml THF unter Inertgasatmosphäre vorlegen (Suspension). 100 ml (0,15 mol) 1,5 m DIBAH-Lösung in Toluol bei einer Innentemperatur von 10-20°C zutropfen. Die so erhaltene klare, homogene Lösung zu einer Suspension von 1,9 g Lithiumalanat in 50 ml THF zutropfen. Nach vollständiger Zugabe 15 min bei Raumtemperatur, anschließend 30 min bei Rückflußtemperatur rühren. Nach Abkühlung portionsweise 3,8 g (0,1 mol) Lithiumalanat zugeben, dann 24 h zum Rückfluß erhitzen. Nach Abkühlung nacheinander 50 ml Wasser und 10 ml 1M-Natronlauge zutropfen, den Niederschlag absaugen und dreimal mit je 150 ml Ethanol auskochen. Filtrat und Extrakte vereinigen und einengen.
- Ausbeute:: 16,4 g (Produkt enthält Lithium- und Aluminiumhydroxid)
- Rf =: 0,3
Methylenchlorid/Methanol/17 %-iges wäßriges Ammoniak (2:4:1)

### Beispiel H:

### (1RS,2SR,6RS)-2-Ethyloxycarbonylaminomethyl-8-azabicyclo[4.3.0]non-4-en

### H.1. (1RS,2SR,6RS)-8-tert.-Butyloxycarbonyl-2-hydroxymethyl-8-azabicyclo[4.3.0]non-4-en

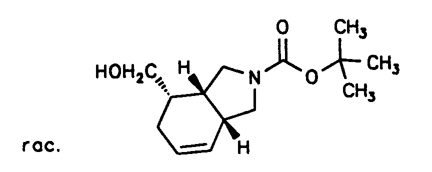

16,4 g Rohprodukt aus Beispiel G.4. (entspricht 0,1 mol der Titelverbindung aus Beispiel G.4.) in 100 ml THF lösen. Bei einer Innentemperatur von 0-5°C 22,9 g (0,105 mol) Di-tert.-butyl-dicarbonat in 100 ml THF gelöst zutropfen, 24 h bei 0 °C, anschließend weitere 24 h bei Raumtemperatur rühren. Einengen, Rohprodukt an 250 g Kieselgel (63-200 µm) mit Cyclohexan/Aceton (2:1) säulenchromatographisch reinigen.
- Ausbeute:: 13,7 g (54 % der Theorie über 2 Stufen); zähes Öl
- Rf =: 0,21 Titelverbindung
0,08 Titelverbindung aus Beispiel G.4.

### H.2. (1RS,2SR,6RS)-8-tert.-Butyloxycarbonyl-2-(4-toluolsulfo-nyloxymethyl)-8-azabicyclo[4.3.0]non-4-en

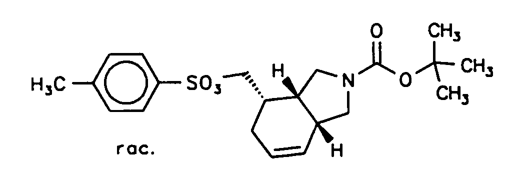

Analog Beispiel F.1. erhält man aus der Titelverbindung aus Beispiel H.1. die Titelverbindung.
Ausbeute: 81-83 % der Theorie
Schmelzpunkt: 160-162 °C
¹H-NMR (CDCl₃): δ = 7,79 (m, 2H, Aryl-H); 7,37 (d, 2H, Aryl-H); 5,67 (m, 1H, H an C-4); 5,47 (m, 1H, H an C-5); 3,78-3,97 (m, 2H, Tosyl-OCH₂-); 3,13-3,42 (m, 3H, CH₂-N); 2,95 (t, 1H, CH₂-N); 2,74 (m, 1H); 2,54 (m, 1H); 2,47 (s, 3H, Aryl-CH₃); 2,32 (m, 1H, H an C-2); 2,06 (m, 1H, Hₐ an C-3); 1,66-1,83 (m, 1H, H_{b} an C-3); 1,44 ppm (s, 9H).

### H.3. (1RS,2SR,6RS)-8-tert.-Butyloxycarbonyl-2-ethyloxycarbonylaminomethyl-8-azabicyclo[4.3.0]non-4-en

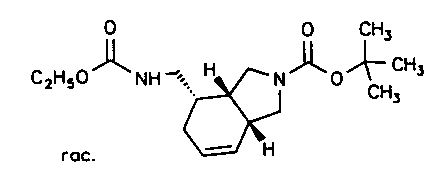

Analog Beispiel F.2. erhält man aus der Titelverbindung aus Beispiel H.2. die Titelverbindung.

Rohprodukt an Kieselgel (63-200 µm) mit Cyclohexan/Aceton (2:1) säulenchromatographisch reinigen.
Ausbeute: 76 % der Theorie; klares, zähes Öl
Rf = 0,35 (Cyclohexan/Aceton 2:1)
¹H-NMR (Cl₂DC-CDCl₂; 80 °C): δ = 5,69 (m, 1H, H an C-4); 5,47 (d, 1H, H an C-5); 4,59 (br, 1H, NH); 4,10 (q, 2H, Ethoxy-CH₂); 3,38 (dd, 1H); 3,32 (m, 1H); 3,24 (m, 1H); 3,01-3,08 (m, 3H); 2,79 (m, 1H); 2,47 (m, 1H); 2,07 (m, 2H); 1,78 (m, 1H); 1,42 (s, 9H); 1,22 ppm (t, 3H, Ethoxy-CH₃).

### H.4. (1RS,2SR,6RS)-2-Ethyloxycarbonylaminomethyl-8-azabicyclo [4.3.0]non-4-en

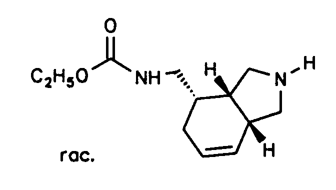

Analog Beispiel C.3. erhält man aus der Titelverbindung aus Beispiel H.3. die Titelverbindung.
- Ausbeute:: 42 % der Theorie
- Rf =: 0,93 Titelverbindung aus Beispiel H.3.
0,23 Titelverbindung
Methylenchlorid/Methanol/17 %-iges wäßriges Ammoniak (15:4:0,5)

### Beispiel I:

### (1SR,2RS,3RS,6SR)-2-Ethyloxycarbonylamino-3-methyl-8-azabicyclo[4.3.0]non-4-en

### I.1. N-[(2E,4E)-2,4-Hexadienyl]-phthalimid

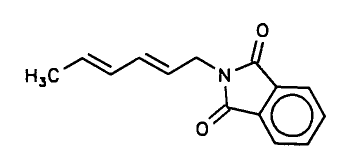

Analog Beispiel B.1. erhält man aus (2E,4E)-1-Brom-2,4-hexadien die Titelverbindung.
Ausbeute: 77-79 % der Theorie
Schmelzpunkt: 114-117 °C (Probe aus Ethanol umkrist.)
¹H-NMR (CDCl₃): δ = 7,85 (m, 2H); 7,72 (m, 2H); 6,25 (dd, 1H); 6,00 (ddd, 1H); 5,5-5,8 (m, 2H); 4,29 (d, 2H); 1,74 ppm (d, 3H).

### I.2. (2E,4E)-1-Amino-2,4-hexadien

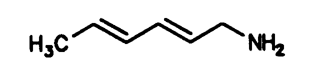

Analog Beispiel B.2. erhält man aus der Titelverbindung aus Beispiel I.1. die Titelverbindung; Siedebereich: 40-70 °C bei 16-18 mbar.
Ausbeute: 67-83 % der Theorie

### I.3. (E)-4-[(2E,4E)-2,4-Hexadienylamino]-2-butensäureethylester

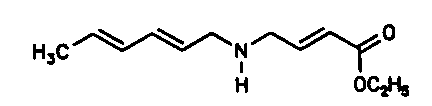

Analog Beispiel B.3. erhält man aus der Titelverbindung aus Beispiel I.2. die Titelverbindung.
Ausbeute: 46 % der Theorie
¹H-NMR (CDCl₃): δ = 6,98 (dt, 1H); 5,9-6,25 (m, 3H); 5,5-5,8 (m, 2H); 4,19 (q, 2H); 3,40 (dd, 2H); 3,27 (d, 2H); 1,76 (d, 3H); 1,29 ppm (t, 3H).

### I.4. (1RS,2RS,3RS,6SR)-8-tert.-Butyloxycarbonyl-3-methyl-8-azabicyclo-[4.3.0]non-4-en-2-carbonsäureethylester (Diastereomer A) und (1RS,2RS,3SR,6RS)-8-tert.-Butyloxycarbonyl-3-methyl-8-azabicyclo-[4.3.0]non-4-en-2-carbonsäureethylester (Diastereomer B)

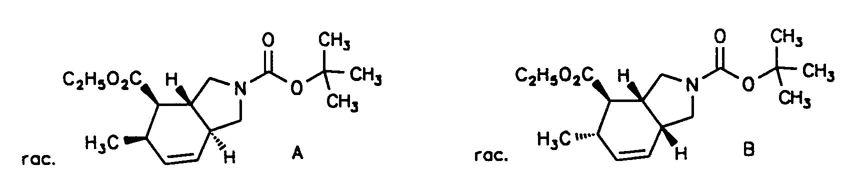

Analog Beispiel B.4. erhält man aus der Titelverbindung aus Beispiel I.3. die Titelverbindungen.
Ausbeute: 70 % der Theorie; Gemisch aus 2 Diastereomeren A und B im Verhältnis A:B = 4:1.
Rf= 0,49 (Cyclohexan/Aceton 2:1)

### I.5. (1RS,2RS,3RS,6SR)-8-tert.-Butyloxycarbonyl-3-methyl-8-azabicy clo[4.3.0]non-4-en-2-carbonsäure

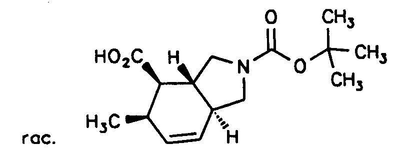

1,17 g (21 mmol) Kaliumhydroxid in 20 ml Wasser gelöst vorlegen. 5,9 g (19 mmol) der Titelverbindung aus Beispiel I.4. in 20 ml Methanol gelöst zugeben und 48 h unter einer Stickstoffatmosphäre zum Rückfluß erhitzen. Einengen, in Wasser aufnehmen, einmal mit Methylenchlorid extrahieren, wässrige Phase mit Essigsäure auf pH 3-4 einstellen, Niederschlag absaugen, mit Wasser waschen, bei Raumtemperatur trocknen, aus Cyclohexan/Aceton 6:1 umkristallisieren
Ausbeute: 2,25 g (42 % der Theorie)
Schmelzpunkt: 189 °C
¹H-NMR (d₆-DMSO): δ = 5,77 (d, 1H); 5,61 (m, 1H); 3,67 (m, 1H); 3,54 (m, 1H); 2,61-2,95 (m, 4H); 2,30 (m, 1H); 1,82 (m, 1H); 1,40 (s, 9H); 0,90 ppm (d, 3H).

### I.6. (1SR,2RS,3RS,6SR)-8-tert.-Butyloxycarbonyl-2-ethyloxycarbonylamino-3-methyl-8-azabicyclo[4.3.0]non-4-en

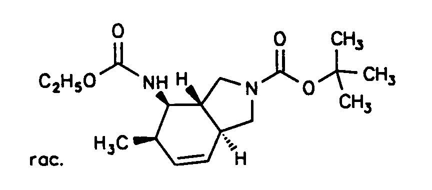

Analog Beispiel C.2. erhält man aus 2,25 g (8 mmol) der Titelverbindung aus Beispiel I.5. die Titelverbindung. Gegenüber Beispiel C.2. geändert: 8 h Rückfluß in Ethanol anstatt 4 h; Reinigung durch Säulenchromatographie an 100 g Kieselgel (63-200 µm) mit Toluol/Essigester (2:1).
Ausbeute: 1,6 g (59 % der Theorie) Klares Öl
¹H-NMR (CDCl₃): δ = 5,68 und 5,72 (2d, 1H); 5,61 (m, 1H); 4,81 (m, 1H); 4,0-4,2 (m, 3H); 3,53 (m), 3,62 (m) und 3,72 (dd) [2H]; 3,08 (t, 1H); 2,92 (t, 1H); 2,75 (m, 1H); 2,47 (m,1H); 1,83 (m, 1H); 1,47 (m, 9H); 1,25 (m, 3H); 0,97 ppm (d, 3H).

### I.7. (1SR,2RS,3RS,6SR)-2-Ethyloxycarbonylamino-3-methyl-8-azabicyclo[4.3.0]non-4-en

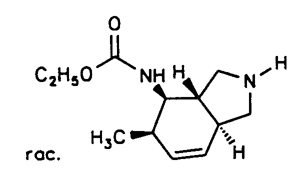

Analog Beispiel C.3. erhält man aus 1,6 g (4,7 mmol) der Titelverbindung aus Beispiel I.6. die Titelverbindung.
Ausbeute: 0,7 g (70 % der Theorie) gelbliches Öl;
Rf = 0,09
Methylenchlorid/Methanol/17 %-iges wäßriges Ammoniak (15:4:0,5)

### Beispiel K:

### (1RS,2RS,6RS)-2-Ethyloxycarbonylaminomethyl-8-azabicyclo[4.3.0]non-4-en

### K.1. 3-Phthalimidomethyl-cyclohex-4-en-1,2-dicarbonsäurediethylester

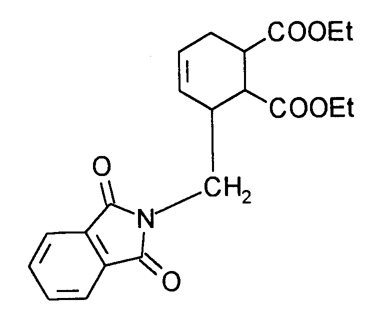

10.67 g (50 mmol) N-[(E)-2,4-Pentadienyl]-phthalimid (Titelverbindung aus Beispiel B.1.) und 8.61 g Fumarsäurediethylester werden in 50 ml Toluol 2 Tage zum Rückfluß erhitz. Der Ansatz wird eingeengt und der Rückstand an Kieselgel chromatographiert (Laufmittel: Cyclohexan/Aceton 8:1).
Ausbeute: 14,8 g (77 % der Theorie)
Schmelzpunkt 80-84°C

### K2. Ethyl-(1RS,2RS,6RS)-9-oxo-8-azabicyclo[4.3.0]non-4-en-2-carboxylat (A) und Ethyl-(1RS,2RS,6SR)-9-oxo-8-azabicyclo[4.3.0]non-4-en-2-carboxylat (B)

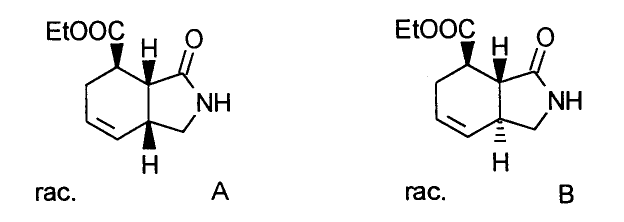

150,3 g (0,39 mol) der Titelverbindung aus Beispiel K.1. werden in 720 ml Ethanol vorgelegt und unter Eiskühlung 173,3 g (2,9 mol) Ethylendiamin zugetropft. Man rührt 20 h bei Raumtemperatur, engt in vacuo ein, verdünnt mit Wasser (ca. 700 ml), stellt mit konz. Salzsäure pH 2-3 ein und extrahiert dreimal mit jeweils 500 ml Dichlormethan. Die organische Phase wird getrocknet (Natriumsulfat) und in vacuo eingeengt. Die Trennung der Diastereomeren gelingt durch Chromatographie (Laufmittel: Cyclohexan/Aceton 1:1).
- Ausbeute:: 36,7 g Produkt A (45 % der Theorie)
RF = 0.47 (Cyclohexan/Aceton 1:1)
27,0 g Produkt B (45 % der Theorie)
RF = 0.22 (Cyclohexan/Aceton 1:1)

### K.3. (1RS,2RS,6RS)-2-Hydroxymethyl-8-azabicyclo[4.3.0]non-4-en

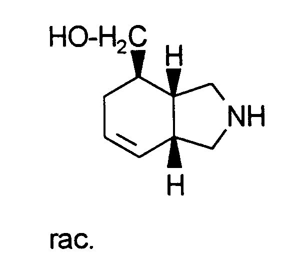

5,2 g (25 mmol) Ethyl-(1RS,2RS,6RS)-9-oxo-8-azabicyclo[4.3.0]non-4-en-2-carboxylat (Produkt A aus Beispiel K.2.) werden unter Stickstoff-Atmosphäre in 50 ml Tetrahydrofuran gelöst und nachfolgend 130 ml einer 1.5molaren Di(isobutyl)aluminiumhydrid-Lösung (195 mmol) zugetropft. Die Lösung wird 16 h unter Rückfluß erwärmt. Nach vollständiger Umsetzung werden nacheinander 60 ml Methanol, 30 ml tert.-Butylmethylether und 10 ml Wasser zugetropft und unter Zusatz von Tonsil abgesaugt. Der Nutschrückstand wird zweimal mit einer Mischung aus Ethanol/konz. Ammoniak/Wasser (10:1:1) verrührt und erneut abgesaugt. Die vgereinigten Filtrate werden eingeengt und das Rohprodukt chromatographisch gereinigt (Laufmittel: Dichlormethan/Methanol/konz. Ammoniak 2:4:1).
Ausbeute: 2,7 g (71 % der Theorie)
¹H-NMR (DMSO-d₆): 5.69 (m, 1H, 4-H); 5.60 (m, 1H, 5-H); 3.39 (dd, 1H, 10a-H); 3.26 (dd, 1H, 10b-H); 2.97 (m, 2H, 7a-H, 9a-H), 2.63 (m, 1H, 9b-H); 2.38 (bs, 1H, 6-H)M; 2.32 (dd, 1H, 7b-H); 2.06 (m, 1H, 3a-H); 1.95 (m, 1H, 1-H); 1.77 (m, 1H, 3b-H); 1.44 ppm (m, 1H, 2-H).

### K.4. (1RS,2RS,6RS)-8-tert.-Butyloxycarbonyl-2-hydroxymethyl-8-azabicyclo[4.3.0]non-4-en

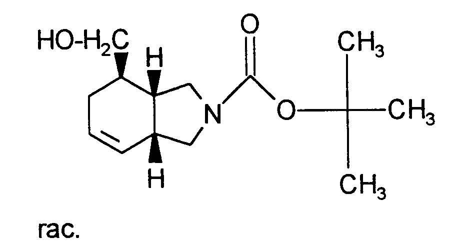

Das Produkt aus Beispiel K.3. (8,87 g; 58 mmol) wird umgesetzt wie im Beispiel H.1. beschrieben.
Ausbeute: 11,0 g (75 % der Theorie)
RF = 0.25 (Cyclohexan/Aceton 2:1)

### K.5. (1RS,2RS,6RS)-8-tert.-Butyloxycarbonyl-2-(4-toluolsulfonyloxymethyl)-8-azabicyclo[4.3.0]non-4-en

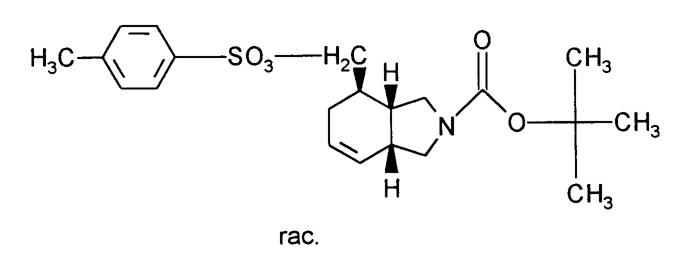

Die Titelverbindung wird aus dem Produkt von Beispiel K.4. in Analogie zum Beispiel F.1. erhalten.
Ausbeute: 97 % der Theorie
RF = 0.40 (Cyclohexan/Aceton 2:1)

### K.6. (1RS,2RS,6RS)-8-tert.-Butyloxycarbonyl-2-azidomethyl-8-azabicyclo[4.3.0]non-4-en

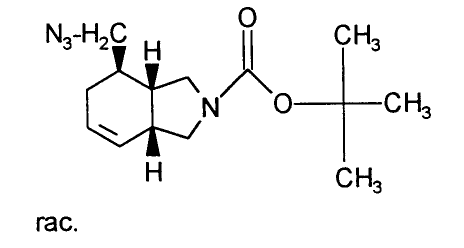

Eine Lösung von 33 g (0,08 mol) (1RS,2RS,6RS)-8-tert.-Butyloxycarbonyl-2-(4-toluolsulfonyloxymethyl)-8-azabicyclo[4.3.0]non-4-en (Titelverbindung aus Beispiel K.5.) und 15,8 g (0,24 mol) Natriumazid in 200 ml N,N-Dimethylformamid wird 40 h bei 70°C gerührt. Die abgekühlte Lösung wird mit Wasser (500 ml) verdünnt und dreimal mit je 250 ml Petrolether extrahiert. Die vereinigte organische Phase wird mit 5 %iger Natriumhydrogencarbonat-Lösung gewaschen, getrocknet (Natriumsulfat) und eingeengt.
Ausbeute: 21,6 g (97 %)
¹H-NMR (CDCl₃): 5.71 (m, 1H, C=CH); 5.58 (m, 1H, C=CH); 3.61-3.22 (m, 2H); 3.10 (m, 1H); 2.70 (bs, 1H); 2.24 (m, 2H); 1.91 (m, 2H), 1.47 ppm (s, 9H, tert.-Butyl).

### K.7.(1RS,2RS,6RS)-8-tert.-Butyloxycarbonyl-2-aminomethyl-8-azabicyclo[4.3.0]non-4-en

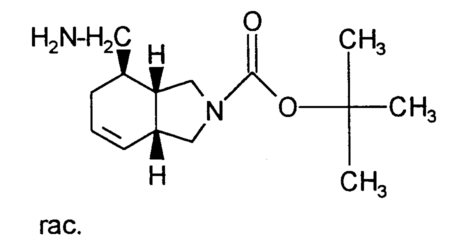

Eine Lösung der Azidoverbindung aus Beispiel K.6. (21,6 g; 78mmol) in 150 ml Pyridin/Wasser (5:1) wird unter Eiskühlung mit Schwefelwasserstoff gesättigt und anschließend 20 h bei Raumtemperatur belassen. Nach vollständigem Umsatz wird in vacuo eingeengt, mehrfach mit Toluol nachdestilliert und der Rückstand chromatographiert (Laufmittel: Cyclohexan/Aceton 1:1).
Ausbeute: 11,0 g (66 % der Theorie)
RF = 0.12 (Cyclohexan/Aceton 1:1)

### K.8. (1RS,2RS,6RS)-8-tert.-Butyloxycarbonyl-2-(ethyloxycarbonylaminomethyl)-8-azabicyclo[4.3.0]non-4-en

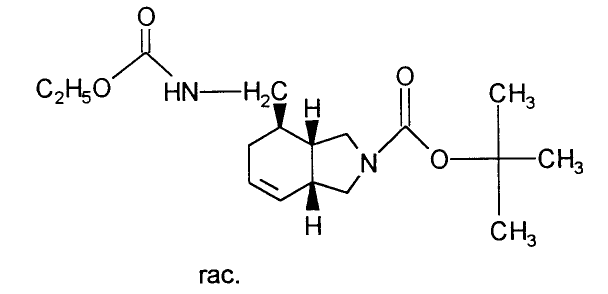

3,7 g (15 mmol) (1RS,2RS,6RS)-8-tert.-Butyloxycarbonyl-2-aminomethyl-8-azabicyclo[4.3.0]non-4-en werden in 40 ml Dioxan und 15 ml Wasser vorgelegt, 2,3 g (16 mmol) Kaliumcarbonat zugegeben und 1,75 g (16 mmol) Chlorameisensäureethylester bei Raumtemperatur zugetropft. Nach zweistündigem Rühren wird in vacuo eingeengt, der Rückstand in Dichlormethan (70 ml) aufgenommen, zweimal mit je 25 ml Wasser ausgeschüttelt, getrocknet (Natriumsulfat) und eingeengt. Das Rohprodukt wird durch Chromatographie (Cyclohexan/Aceton 2:1) gereinigt.
Ausbeute: 2,8 g (59 % der Theorie)
RF = 0.53 (Cyclohexan/Aceton 1:1)

### K.9. (1RS,2RS,6RS)-2-(Ethyloxycarbonylaminomethyl)-8-azabicyclo[4.3.0]non-4-en

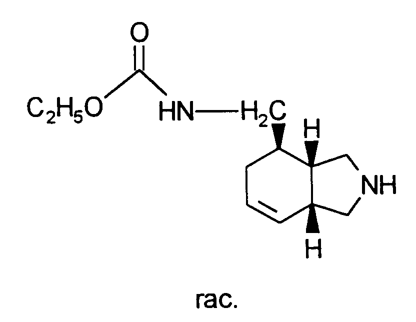

7,6 g (23 mmol) des Produktes aus Beispiel K.8. werden in 100 ml Methanol/Wasser (1:1) vorgelegt und bei Raumtemperatur 30 ml halbkonzentrierte Salzsäure zulaufen gelassen. Nachdem die Gasentwicklung beendet ist, wird 30 Minuten nachgerührt, mit Eiswasser (ca. 100 ml) verdünnt und mit konz. Natronlauge pH 12 eingestellt. Die wäßrige Phase wird viermal mit je 100 ml Dichlormethan extrahiert. Die Extrakte werden vereinigt, über Natriumsulfat getrocknet und in vacuo eingeengt.
Ausbeute: 3,9 g (76 % der Theorie)
RF = 0.45 (Dichlormethan/Methanol/konz. Ammoniak (2:4:0.1)

### Beispiel L:

### (1RS,2RS,6RS)-2-Aminomethyl-8-azabicyclo[4.3.0]non-4-en-bis-trifluormethansulfonat

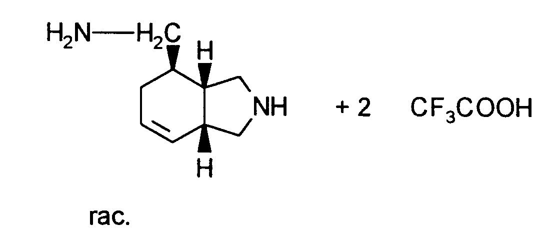

Eine Lösung von 2,0 g (8 mmol) (1RS,2RS,6RS)-8-tert.-Butyloxycarbonyl-2-aminomethyl-8-azabicyclo[4.3.0]non-4-en (Produkt aus Beispiel K.7.) in 30 ml Dichlormethan wird mit 30 ml Trifluoressigsäure versetzt und 30 Minuten bei Raumtemperatur belassen. Das Lösungsmittel und die Säure werden in Gegenwart von Toluol abdestilliert, mehrfach mit Toluol nachdestilliert. Das Produkt wird im Vakuum-Exsikkator über Kaliumhydroxid/Phosphorpentoxid (1:1) getrocknet.
Ausbeute: 1,5 g braunes Öl
¹H-NMR (DMSO-d₆): 5.78 (m, 1H, C=CH); 5.60 (m, 1H, C=CH); 3.34 (M, 2H); 3.03 (m, 1H), 2.87 (m, 2H), 2.73 (m, 1H); 2.45 (m, 1H); 2.34 (m, 1H); 2.22 (M, 1H); 1.94 ppm (m, 2H).
FAB-MS: M+1 = 153.

### Beispiel M:

### (1RS,2RS,6RS)-2-Ethyloxycarbonylaminomethyl-8-azabicyclo[4.3.0]non-4-en

(Das Produkt ist identisch mit der Titelverbindung aus Beispiel F.)

### M.1. (1RS,2RS,6SR)-2-Hydroxymethyl-8-azabicyclo[4.3.0]non-4-en

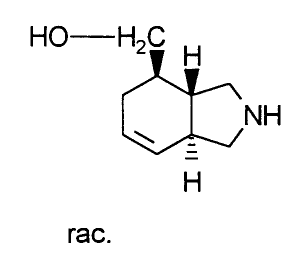

Ethyl-(1RS,2RS,6RS)-9-oxo-8-azabicyclo[4.3.0]non-4-en-2-carboxylat (Produkt B aus Beispiel K.2.) wird analog zu Beispiel K.3. umgesetzt.
Ausbeute: 75 % der Theorie
RF = 0.22 (Dichlormethan/Methanol/konz. Ammoniak (15:4:0.5)

### M.2. (1RS,2RS,6SR)-8-tert.-Butyloxycarbonyl-2-hydroxymethyl-8-azabicyclo[4.3.0]non-4-en

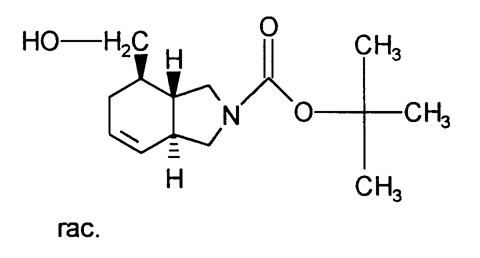

Das Produkt aus Beispiel M.1. wird analog zu Beispiel K.4. umgesetzt.
Ausbeute: 64 % der Theorie
RF = 0.23 (Cyclohexan/Aceton 2:1)

### M.3. (1RS,2RS,6SR)-8-tert.-Butyloxycarbonyl-2-(4-toluolsulfonyloxymethyl)-8-azabicyclo[4.3.0]non-4-en

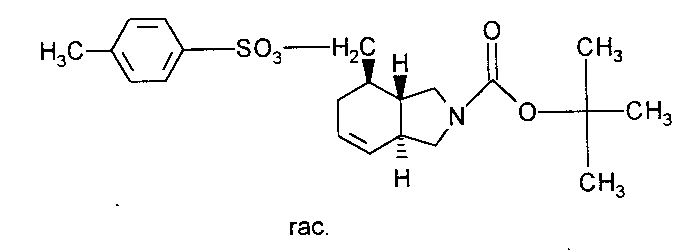

Die Titelverbindung wird aus dem Produkt von Beispiel M.2. in Analogie zum Beispiel F.1. erhalten.
Ausbeute: 91-98 % der Theorie
RF = 0.59 (Cyclohexan/Aceton 2:1)

### M.4. (1RS,2RS,6SR)-8-tert.-Butyloxycarbonyl-2-azidomethyl-8-azabicyclo[4.3.0]non-4-en

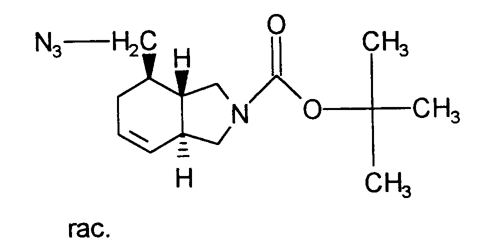

Eine Lösung von 13,0 g (32 mmol) des Produktes aus Beispiel M.3. in 80 ml N,N-Dimethylformamid wird mit 4,15 g (64 mmol) Natriumazid versetzt und 4 h bei 70°C gerührt. Sodann wird nochmals die gleiche Menge Natriumazid nachgesetzt und weitere 6 h bei 100°C gerührt. Anschließend wird aufgearbeitet, wie im Beispiel K.6. beschrieben.
Ausbeute: 7,0 g (79 % der Theorie)
RF = 0.55 (Cyclohexan/Aceton 2:1)

### M.5. (1RS,2RS,6SR)-8-tert.-Butyloxycarbonyl-2-aminomethyl-8-azabicyclo[4.3.0]non-4-en

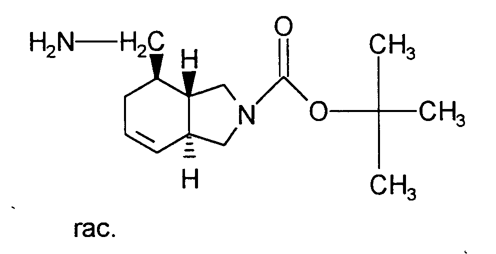

Die Azidoverbindung aus Beispiel M.4. wird umgesetzt wie im Beispiel K.7. beschrieben.

Die Chromatographie erfolgt mit Methanol/Dichlormethan/konz. Ammoniak (15:2:0.1).
Ausbeute: 75 % der Theorie
RF = 0.12 (Methanol/Dichlormethan/konz. Ammoniak 15:2:0.1)

### M.6. (1RS,2RS,6SR)-8-tert.-Butyloxycarbonyl-2-(ethyloxycarbonylmethyl)-8-azabicyclo[4.3.0]non-4-en

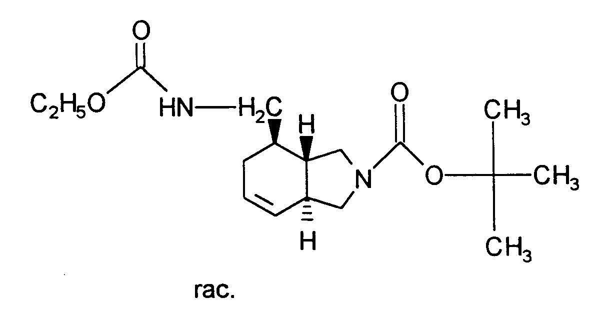

4.3 g (17 mmol) der Aminoverbindung aus Beispiel M.5. und 1,9 g (19 mmol) Triethylamin werden in 50 ml Dichlormethan vorgelegt, bei 0°C 2,2 g (20 mmol) Chlorameisensäureethylester, gelöst in 10 ml Dichlormethan, zugetropft und 24 h bei Raumtemperatur nachgerührt. Die Lösung wird mit Wasser (50 ml) versetzt und die Phasen getrennt. Die wäßrige Phase wird noch dreimal mit je 40 ml Dichlormethan extrahiert. Die organischen Phasen werden vereinigt, getrocknet (Natriumsulfat) und eingeengt.
Ausbeute: 5,3 g (96 % der Theorie)
¹H-NMR (CDCl₂-CDCl₂, 80°C): 5.79 (ddd, 1H, C=CH); 5.58 (m, 1H, C=CH); 4.61 (bs, 1H, Carbamat-NH); 4.23 (m, 1H); 4.12 (q, 2H, Ethyl-CH₂); 3.99 (m, 1H); 3.20-3.08 (m, 2H); 2.82 (m, 2H); 2.25 (m, 2H); 2.09 (m, 1H); 1.84 (m, 2H); 1.42 (s, 9H, tert.-Butyl); 1.37 ppm (t, 3H, Ethyl-CH₃).

### M.7. (1RS,2RS,6SR)-2-(Ethyloxycarbonylaminomethyl)-8-azabicyclo[4.3.0]non-4-en

(1RS,2RS,6SR)-8-tert.-Butyloxycarbonyl-2-(ethyloxycarbonylaminomethyl)-8-azabicyclo[4.3.0]non-4-en wird umgesetzt, wie im Beispiel K.9. beschrieben.
Ausbeute: quantitativ
RF = 0.55 (Methanol/Dichlormethan/konz. Ammoniak 15:4:0.5)

### Beispiel N:

### (1SR,2RS,6RS)-2-Methylamino-8-azabicyclo[4.3.0]non-en

### N.1. (1RS,2RS,6RS)-9-Oxo-azabicyclo[4.3.0]non-4-en-2-carbonsäure

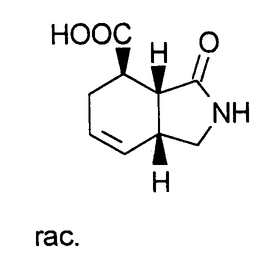

8,36 g (40 mmol) Ethyl-(1RS,2RS,6RS)-9-oxo-8-azabicyclo[4.3.0]non-4-en-2-carboxylat (Produkt A aus Beispiel K.2.) werden mit 30 ml Wasser und 5 ml konz. Schwefelsäure 40 h bei 60°C gerührt. Beim Abkühlen Fallt das Produkt aus. Der Niederschlag wird mit wenig kaltem Wasser gewaschen und im Vakuum-Trockenschrank bei 50°C getrocknet.
Ausbeute: 4,80 g (66 % der Theorie)
¹H-NMR (DMSO-d₆): 12.35 (s, 1H, COOH); 7.60 (s, 1H, Lactam-NH); 5.74 (m, 1H, C=CH); 5.59 (m, 1H, C=CH); 3.45 (dd, 1H, 7a-H); 2.95-2.85 (m, 4H, 1-H, 2-H, 6-H, 7b-H); 2.29 (m, 1H, 3a-H); 2.00 ppm (m, 1H, 3b-H).

### N.2. (1SR,2RS,6RS)-2-Ethoxycarbonylamino-9-oxo-8-azabicyclo[4.3.0]non-4-en

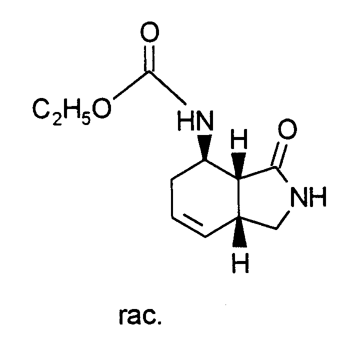

(1RS,2RS,6RS)-9-Oxo-8-azabicyclo[4.3.0]non-4-en-2-carbonsäure (Titelverbindung aus Beispiel N.1.) wird analog Beispiel C.2. umgesetzt.
Ausbeute: 68 % der Theorie
RF = 0.06 (Cyclohexan/Aceton 1:1)

### N.3. (1SR,2RS,6RS)-2-Methylamino-8-azabicyclo[4.3.0]non-4-en

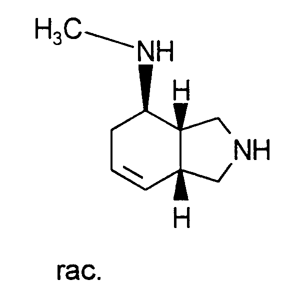

Die Titelverbindung wird erhalten, indem das Produkt aus Beispiel N.2. mit 10 Equivalenten Di(isobutyl)aluminiumhydrid analog Beispiel K.3. umgesetzt und aufgearbeitet wird.
Ausbeute: 51 % der Theorie
¹H-NMR (CDCl₃): 5.72 (m, 1H, C=CH); 5.68 (m, 1H, C=CH); 3.19-3.10 (m, 2H); 2.88 (dd, 1H); 2.60 (dd, 1H); 2.50 (m, 1H); 2.44 (s, 3H, N-CH₃); 2.33-2.28 (m, 2H); 2.19 (m, 1H); 1.89 ppm (m, 1H).

### Beispiel O:

### (1SR,2SR,6RS)-2-Methylamino-8-azabicyclo[4.3.0]non-4-en

### 0.1. (1RS,2SR,6RS)-9-Oxo-8-azabicyclo[4.3.0]non-4-en-2-carbonsäure

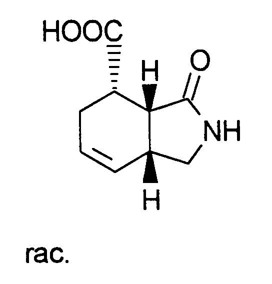

0,2 g konz. Schwefelsäure, 25 ml Wasser und 25 ml Essigsäure werden bei 60°C vorgelegt. 9,8 g (50 mmol) des Produktes aus Beispiel G.3. werden in kleinen Portionen zugegeben. Man rührt 5 h bei 60°C nach. Zur Aufarbeitung wird eine Lösung von 0,8 g Natriumhydrogencarbonat in 10 ml Wasser zugegeben und in vacuo eingeengt. Der Rückstand wird in 40 ml Wasser suspendiert und unter Eiskühlung durch Zugabe konz. Natronlauge in Lösung gebracht. Nachdem von unlöslichen Anteilen abgesaugt worden ist, wird mit halbkonzentrierter Salzsäure sauer gestellt und wieder auf 0°C abgekühlt. Das ausfallende Produkt wird mit wenig kaltem Wasser gewaschen und nachfolgend im Vakuum-Trockenschrank bei 50°C getrocknet.
Ausbeute: 4,8 g (53 % der Theorie)
Schmelzpunkt: 192-193°C

### O.2. (1SR,2SR,6RS)-2-Ethoxycarbonylamino-9-oxo-8-azabicyclo[4.3.0]non-4-en

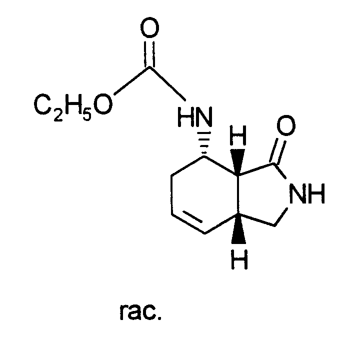

(1RS,2SR,6RS)-9-Oxo-8-azabicyclo[4.3.0]non-4-en-2-carbonsäure (Titelverbindung aus Beispiel O.1.) wird umgesetzt wie im Beispiel C.2. beschrieben.
Ausbeute: 68 % der Theorie
Schmelzpunkt: 160-164°C

### O.3. (1SR,2SR,6RS)-2-Methylamino-8-azabicyclo[4.3.0]non-4-en

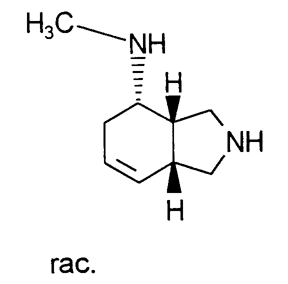

Die Titelverbindung wird erhalten, indem das Produkt aus Beispiel O.2. mit 10 Equivalenten Di(isobutyl)aluminiumhydrid analog Beispiel K.3. umgesetzt und aufgearbeitet wird.
Ausbeute: 81 % der Theorie
¹H-NMR (CDCl₃): 5.72 (m, 1H, C=CH); 5.50 (m, 1H, C=CH); 3.04-2.77 (m, 6H); 2.60 (m; 1H); 2.49 (s, 3H, N-CH₃); 2.31 (bs, 2H, 2xNH); 2.25 (m, 1H); 1.89 ppm (m, 1H).

## Patentansprüche

1. Verbindungen der allgemeinen Formel (I) 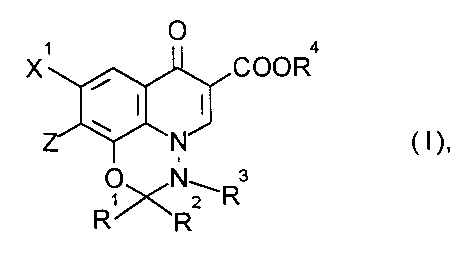 in welcher
R¹ für Wasserstoff oder gegebenenfalls durch Hydroxy oder Halogen substituiertes C₁-C₄-Alkyl steht,
R² unabhängig von R¹ für Wasserstoff oder Methyl steht,
R³ für Wasserstoff oder C₁-C₄-Alkyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
X¹ für Wasserstoff oder Halogen steht,
Z für Reste der Strukturen 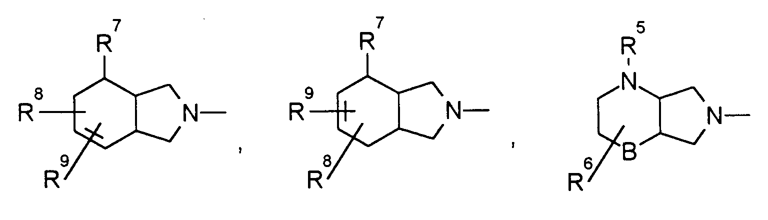 steht,
worin
R⁷ für Wasserstoff, Hydroxy, -NR¹⁰R¹¹, Hydroxymethyl oder -CH₂-NR¹⁰R¹¹, Carboxyl, Methoxycarbonyl oder Ethoxycarbonyl steht,
wobei
R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoff oder Methyl steht,
R⁶ für Wasserstoff oder Methyl steht,
R⁵ für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
R^{5'} für Methyl oder Ethyl steht,
B für -CH₂-, O oder eine direkte Bindung steht.
Die Verbindungen der Formel (I) können in Form von Racematen oder als enantiomerenreine Verbindungen sowie Form ihrer pharmazeutisch verwendbaren Hydrate und Säureadditionssalze sowie in Form ihrer Alkali-, Erdalkali-, Silber- und Guanidiniumsalze vorliegen.

2. Verfahren zur Herstellung der Verbindungen der Formel (I) gemäß Anspruch 1 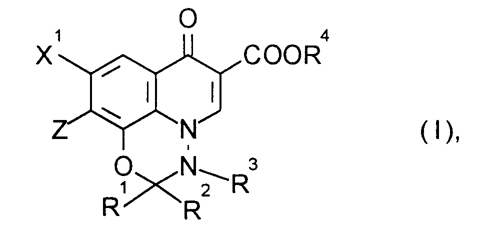 dadurch gekennzeichnet, daß man Verbindungen der Formel (II) 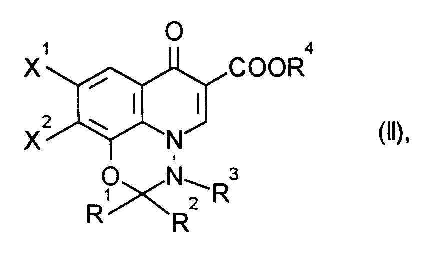 in welcher
R¹, R², R³, R⁴ und X¹ die oben angegebene Bedeutung haben und
X² für Halogen, insbesondere Fluor oder Chlor, steht,
mit Verbindungen der Formel (III)
Z-H (III),
in welcher
Z die oben angegebene Bedeutung hat,
gegebenenfalls in Gegenwart von Säurefängern umsetzt.

3. Verbindungen der Formel (I) gemäß Anspruch 1, in welcher
R¹ für Wasserstoff oder gegebenenfalls durch Hydroxy substituiertes C₁-C₃-Alkyl steht,
R² unabhängig von R¹ für Wasserstoff oder Methyl steht,
R³ für Wasserstoff, Methyl oder Ethyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)methyl steht,
X¹ für Wasserstoff, Fluor oder Chlor steht,
Z für Reste der Strukturen 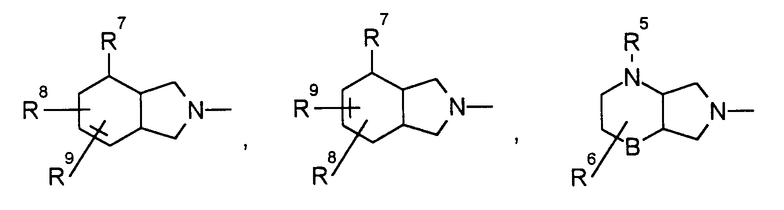 steht,
worin
R⁷ für Wasserstoff, Hydroxy, -NR¹⁰R¹¹, Hydroxymethyl oder -CH₂-NR¹⁰R¹¹ steht,
wobei
R¹⁰ für Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoff oder Methyl steht,
R⁵ für Wasserstoff oder Methyl steht,
R⁶ für Wasserstoff steht,
B für -CH₂-, O oder eine direkte Bindung steht.

4. Verbindungen der Formel (I) gemaß Anspruch 1, in welcher
R¹ für Wasserstoff oder Methyl steht,
R² für Wasserstoff steht,
R³ für Methyl oder Ethyl steht,
R⁴ für Wasserstoff, Methyl oder Ethyl steht,
X¹ für Fluor steht,
Z für Reste der Strukturen 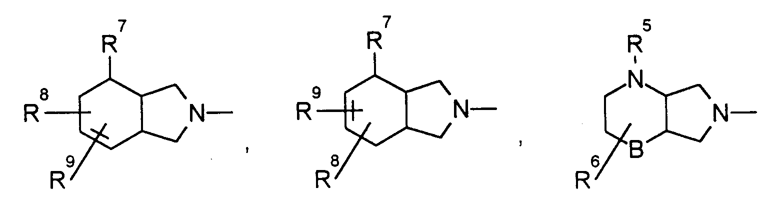 steht,
worin
R⁷ für Wasserstoff, Hydroxy, -NR¹⁰R¹¹, Hydroxymethyl oder -CH₂-NR¹⁰R¹¹ steht,
wobei
R¹⁰ für Wasserstoff, Methyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁶ für Wasserstoff stehen,
R⁹ für Wasserstoff oder Methyl steht,
R⁵ für Wasserstoff oder Methyl steht,
B für -CH₂-, O oder eine direkte Bindung steht

5. Arzneimittel enthaltend Verbindungen der Formel (I) gemäß Anspruch 1.

6. Verwendung von Verbindungen der Formel (I) gemäß Anspruch 1 zur Herstellung von Arzneimitteln.

7. Verwendung von Verbindungen der Formel (I) gemaß Anspruch 1 in antibakteriellen Mitteln.

## Claims

1. Compounds of the general formula (I) 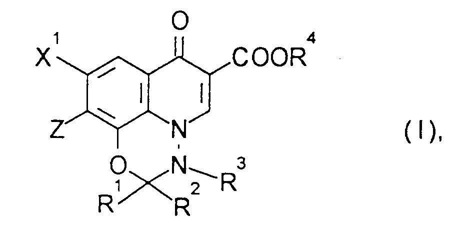 in which
R¹ is hydrogen or C₁-C₄-alkyl optionally substituted by hydroxyl or halogen,
R² independently of R¹ is hydrogen or methyl,
R³ is hydrogen or C₁-C₄-alkyl,
R⁴ is hydrogen, C₁-C₄-alkyl optionally substituted by hydroxyl, methoxy, amino, methylamino or dimethylamino, or (5-methyl-2-oxo-1,3-dioxol-4-yl)-methyl,
X¹ is hydrogen or halogen and
Z is a radical of the structure 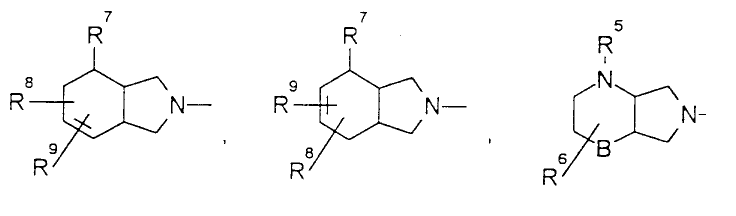
in which
R⁷ is hydrogen, hydroxyl, -NR¹⁰R¹¹, hydroxymethyl, -CH₂-NR¹⁰R¹¹, carboxyl, methoxycarbonyl or ethoxycarbonyl,
R¹⁰ being hydrogen, C₁-C₃-alkyl optionally substituted by hydroxyl, alkoxycarbonyl having 1 to 4 C atoms in the alkoxy moiety, or C₁-C₃-acyl, and
R¹¹ being hydrogen or methyl,
R⁸ is hydrogen, linear or branched C₁-C₃-alkyl or cyclopropyl,
R⁹ is hydrogen or methyl,
R⁶ is hydrogen or methyl,
R⁵ is hydrogen, methyl or a radical of the structure -CH=CH-CO₂R^{5'}, -CH₂-CH₂-CO₂R^{5'}, -CH₂-CO-CH₃ or -CH₂-CH₂-CN,
R^{5'} being methyl or ethyl, and
B is -CH₂-, O or a direct bond,
it being possible for the compounds of the formula (I) to be present in the form of racemates or as enantiomerically pure compounds, in the form of their pharmaceutically acceptable hydrates and acid addition salts or in the form of their alkali metal, alkaline earth metal, silver and guanidinium salts.

2. Process for the preparation of the compounds of the formula (I) according to Claim 1 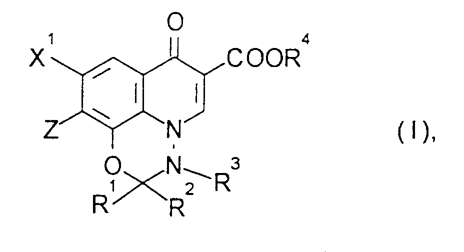 characterized in that compounds of the formula (II) 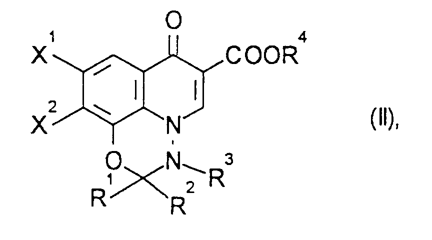 in which
R¹, R², R³, R⁴ and X¹ are as defined above and
X² is halogen, especially fluorine or chlorine,
are reacted with compounds of the formula (III)
Z-H (III),
in which
Z is as defined above,
optionally in the presence of acid acceptors.

3. Compounds of the formula (I) according to Claim 1 in which
R¹ is hydrogen or C₁-C₃-alkyl optionally substituted by hydroxyl,
R² independently of R¹ is hydrogen or methyl,
R³ is hydrogen, methyl or ethyl,
R⁴ is hydrogen, C₁-C₄-alkyl optionally substituted by hydroxyl, methoxy, amino, methylamino or dimethylamino, or (5-methyl-2-oxo-1,3-dioxol-4-yl)methyl,
X¹ is hydrogen, fluorine or chlorine and
Z is a radical of the structure 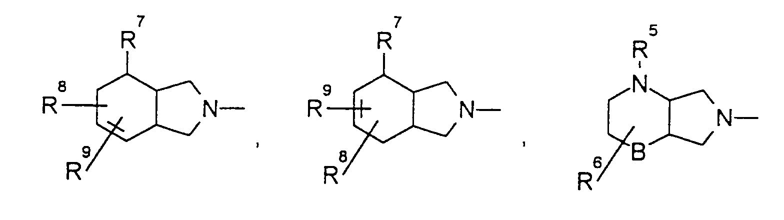
in which
R⁷ is hydrogen, hydroxyl, -NR¹⁰R¹¹, hydroxymethyl or -CH₂-NR¹⁰R¹¹,
R¹⁰ being hydrogen, C₁-C₂-alkyl optionally substituted by hydroxyl, alkoxycarbonyl having 1 to 4 C atoms in the alkoxy moiety, or C₁-C₃-acyl, and
R¹¹ being hydrogen or methyl,
R⁸ is hydrogen, linear or branched C₁-C₃-alkyl or cyclopropyl,
R⁹ is hydrogen or methyl,
R⁵ is hydrogen or methyl,
R⁶ is hydrogen and
B is -CH₂-, O or a direct bond.

4. Compounds of the formula (I) according to Claim 1 in which
R¹ is hydrogen or methyl,
R² is hydrogen,
R³ is methyl or ethyl,
R⁴ is hydrogen, methyl or ethyl,
X¹ is fluorine and
Z is a radical of the structure 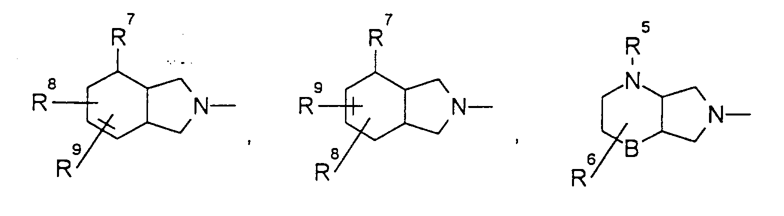
in which
R⁷ is hydrogen, hydroxyl, -NR¹⁰R¹¹, hydroxymethyl or -CH₂-NR¹⁰R¹¹,
R¹⁰ being hydrogen, methyl, alkoxycarbonyl having 1 to 4 C atoms in the alkoxy moiety, or C₁-C₃-acyl, and
R¹¹ being hydrogen or methyl,
R⁸ is hydrogen, linear or branched C₁-C₃-alkyl or cyclopropyl,
R⁶ is hydrogen,
R⁹ is hydrogen or methyl,
R⁵ is hydrogen or methyl and
B is -CH₂-, O or a direct bond.

5. Drugs containing compounds of the formula (I) according to Claim 1.

6. Use of compounds of the formula (I) according to Claim 1 for the preparation of drugs.

7. Use of compounds of the formula (I) according to Claim 1 in antibacterial compositions.

## Revendications

1. Composés de formule générale (I) 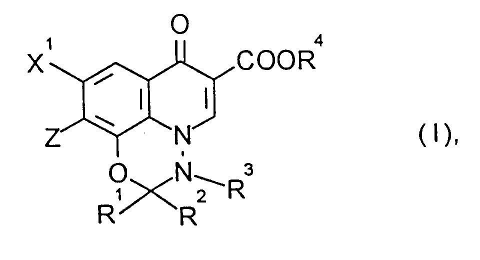 dans laquelle
R¹ représente de l'hydrogène ou un groupe alkyle en C₁ à C₄ éventuellement substitué par un radical hydroxy ou un halogène,
R² représente, indépendamment de R¹, de l'hydrogène ou un groupe méthyle,
R³ est de l'hydrogène ou un groupe alkyle en C₁ à C₄,
R⁴ est de l'hydrogène, un groupe alkyle ayant 1 à 4 atomes de carbone éventuellement substitué par un radical hydroxy, méthoxy, amino, méthylamino ou diméthylamino ou un groupe (5-méthyl-2-oxo-1,3-dioxol-4-yl)-méthyle,
X¹ est de l'hydrogène ou un halogène,
Z représente des restes de structures 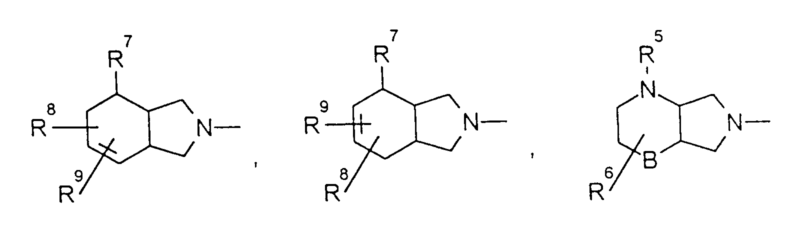
où
R⁷ est de l'hydrogène, un groupe hydroxy, un groupe -NR¹⁰R¹¹, hydroxyméthyle ou -CH₂-NR¹⁰R¹¹, carboxyle, méthoxycarbonyle ou éthoxycarbonyle, où
R¹⁰ est de l'hydrogène, un groupe alkyle en C₁ à C₃ éventuellement substitué par un radical hydroxy, un groupe alkoxy-carbonyle ayant 1 à 4 atomes de carbone dans la partie alkoxy ou un groupe acyle en C₁ à C₃,
R¹¹ est de l'hydrogène ou un groupe méthyle,
R⁸ est de l'hydrogène, un groupe alkyle en C₁ à C₃ linéaire ou ramifié ou un groupe cyclopropyle,
R⁹ est de l'hydrogène ou un groupe méthyle,
R⁶ est de l'hydrogène ou un groupe méthyle,
R⁵ représente de l'hydrogène, un groupe méthyle ou des restes de structures -CH=CH-CO₂R^{5'}, -CH₂-CH₂-CO₂R^{5'}, -CH₂-CO-CH₃, -CH₂-CH₂-CN,
R^{5'} est un groupe méthyle ou éthyle,
B représente un groupe -CH₂-, O ou une liaison simple.
Les composés de formule (I) peuvent exister sous la forme de racémates ou comme composés de pureté énantiomérique ainsi que sous forme de leurs hydrates et sels d'addition d'acides utilisables en pharmacie ainsi que sous forme de leurs sels de métaux alcalins, de métaux alcalino-terreux, d'argent et de guanidinium.

2. Procédé de production des composés de formule (I) suivant la revendication 1 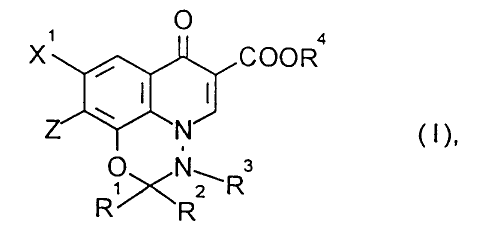 caractérisé en ce qu'on fait réagir des composés de formule (II) 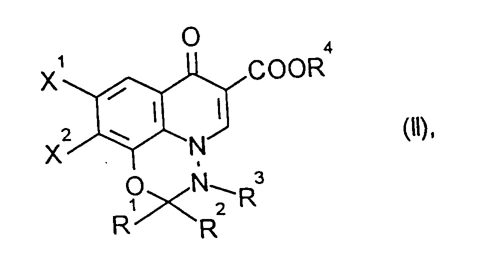 dans laquelle
R¹, R², R³, R⁴ et X¹ ont la définition indiquée ci-dessus et
X² représente un halogène, en particulier le fluor ou le chlore,
avec des composés de formule (III)
Z-H (III),
dans laquelle
Z a la définition indiquée ci-dessus,
le cas échéant en présence d'accepteurs d'acides.

3. Composés de formule (I) suivant la revendication 1, dans laquelle
R¹ représente de l'hydrogène ou un groupe alkyle en C₁ à C₃ éventuellement substitué par un radical hydroxy,
R² représente, indépendamment de R¹, de l'hydrogène ou un groupe méthyle,
R³ est de l'hydrogène, un groupe méthyle ou éthyle,
R⁴ est de l'hydrogène, un groupe alkyle ayant 1 à 4 atomes de carbone éventuellement substitué par un radical hydroxy, méthoxy, amino, méthylamino ou diméthylamino ou un groupe (5-méthyl-2-oxo-1,3-dioxol-4-yl)-méthyle,
X¹ est de l'hydrogène, du fluor ou du chlore,
Z représente des restes de structures 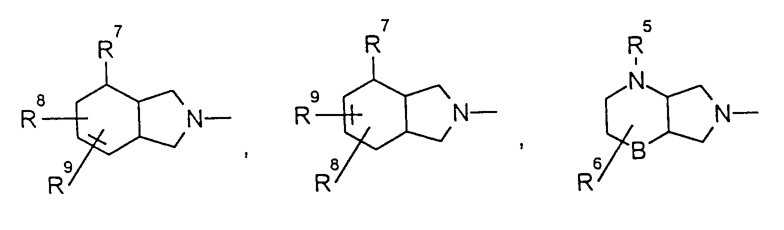
où
R⁷ est de l'hydrogène, un groupe hydroxy, un groupe -NR¹⁰R¹¹, hydroxyméthyle ou -CH₂-NR¹⁰R¹¹, où
R¹⁰ est de l'hydrogène, un groupe alkyle en C₁ ou C₂ éventuellement substitué par un radical hydroxy, un groupe alkoxy-carbonyle ayant 1 à 4 atomes de carbone dans la partie alkoxy ou un groupe acyle en C₁ à C₃,
R¹¹ est de l'hydrogène ou un groupe méthyle,
R⁸ est de l'hydrogène, un groupe alkyle en C₁ à C₃ linéaire ou ramifié ou un groupe cyclopropyle,
R⁹ est de l'hydrogène ou un groupe méthyle,
R⁵ est de l'hydrogène ou un groupe méthyle,
R⁶ est de l'hydrogène,
B est un groupe -CH₂-, O ou une liaison simple,

4. Composés de formule (I) suivant la revendication 1, dans laquelle
R¹ est de l'hydrogène ou un groupe méthyle,
R² est de l'hydrogène,
R³ est un groupe méthyle ou éthyle,
R⁴ est de l'hydrogène, un groupe méthyle ou éthyle,
X¹ est du fluor,
Z représente des restes des structures 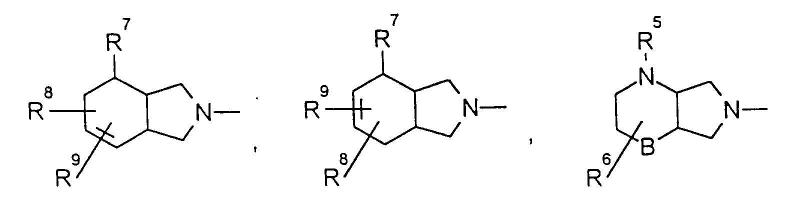
dans lesquelles
R⁷ est de l'hydrogène, un groupe hydroxy, -NR¹⁰R¹¹, hydroxyméthyle ou -CH₂-NR¹⁰R¹¹, où
R¹⁰ est de l'hydrogène, un groupe méthyle, alkoxycarbonyle ayant 1 à 4 atomes de carbone dans la partie alkoxy ou acyle en C₁ à C₃,
R¹¹ est de l'hydrogène ou un groupe méthyle,
R⁸ représente de l'hydrogène, un groupe alkyle en C₁ à C₃ linéaire ou ramifié ou un groupe cyclopropyle,
R⁶ est de l'hydrogène,
R⁹ est de l'hydrogène ou un groupe méthyle,
R⁵ est de l'hydrogène ou un groupe méthyle,
B est un groupe -CH₂-, O ou une liaison simple.

5. Médicament contenant des composés de formule (I) suivant la revendication 1.

6. Utilisation de composés de formule (I) suivant la revendication 1 pour la préparation de médicaments.

7. Utilisation de composés de formule (I) suivant la revendication 1 dans des compositions antibactériennes.
